# EUROPEAN PATENT APPLICATION

(11) **EP 4 711 455 A1**
(43) Date of publication of application: **18.03.2026**
(21) Application number: 24199856.6
(22) Date of filing: 11.09.2024
(51) Int. Cl.: C12N 15/11, C12N 15/113

(54) **SMALL ARTIFICIAL RNA (SMARTRNA) OLIGONUCLEOTIDE FOR MODULATING PROTEIN EXPRESSION**

(71) Applicant: Aarhus Universitet, 8000 Aarhus C (DK)
(72) Inventor: LYKKE-ANDERSEN, Søren, 8000 Aarhus (DK); HØG, Anja Mølhart, 2200 Copenhagen N (DK); SMIDT, Jakob Melgaard, 2200 Copenhagen N (DK); WEILE, Christian Roar Andersen, 2200 Copenhagen N (DK)
(74) Representative: Aera A/S

(57) **Abstract**

The present invention relates to the use of small artificial RNA (hereafter referred to as "smartRNA") containing sequences creating secondary structure motifs similar to C/D box small nucleolar RNAs (snoRNAs), and antisense guide sequences, as a tool for targeted upregulation of protein expression. The smartRNAs assembles into a ribonucleoprotein (RNP) complex resulting in a smartRNP, increasing the 2'-O-methylation level of one or more adenosines of the polyA-tail of the target mRNA, thereby delaying deadenylation of the targeted mRNA, resulting in increased protein production.

## Description

### TECHNICAL FIELD

The present invention relates to the use of small artificial RNA (hereafter referred to as "smartRNA") containing sequences creating secondary structure motifs similar to C/D box small nucleolar RNAs (snoRNAs), and antisense guide sequences, as a tool for targeted upregulation of protein expression. The smartRNAs assembles into a ribonucleoprotein (RNP) complex resulting in a smartRNP, increasing the 2'-O-methylation level of one or more adenosines of the polyA-tail of the target mRNA, thereby delaying deadenylation of the targeted mRNA, resulting in increased protein production.

### BACKGROUND

In several hundred genes, the loss of one of the two alleles results in a disease-causing reduction of gene expression. These haploinsufficiency disorders include a wide range of human diseases and constitute a significant unmet medical need because targeted medical interventions are lacking.

Medical interventions that increase functional protein levels from the affected gene pair are generally expected to be therapeutically relevant, and such interventions can conceptually be designed either by (1) mending the inactivated disease-causing allele, or (2) boosting the healthy allele.

In addition to haploinsufficiency disorders, patients suffering from protein insufficiency disorders caused by mutations in both alleles, may similarly benefit from increased protein expression from a target mRNA. Lastly, in some disorders, boosting the endogenous expression of a protein from a target mRNA may ameliorate the disease.

CRISPR-based genome-editing strategies may be used for mending the disease-causing allele. However, this strategy is non-reversible and is dependent on non-human auxiliary protein factors, thus reducing the likelihood of being approved for use in patients.

Currently, several contemporary oligonucleotide-based therapeutic approaches aim at significantly altering expression of a protein from a target mRNA. Procedures for upregulating gene expression of a targeted gene generally rely on exploiting features that are unique to the individual disease-relevant mRNA or gene. Approaches that lead to increased mRNA stability, and thereby elevated protein levels, have also been suggested. However, these methods depend on the presence of either an upstream open reading frame, alternative polyadenylation sites, or miRNA binding sites in the targeted mRNA, which are not generally present in all mRNAs.

### SUMMARY

The present invention relates to the use of small artificial RNA (hereafter referred to as "smartRNA") containing sequences creating secondary structure motifs similar to C/D box small nucleolar RNAs (snoRNAs), and antisense guide sequences, as a tool for targeted upregulation of protein expression. The smartRNAs assembles into a ribonucleoprotein (RNP) complex resulting in a smartRNP, increasing the 2'-O-methylation level of one or more adenosines of the polyA-tail of the target mRNA, thereby delaying deadenylation of the targeted mRNA, resulting in increased protein production.

In a first aspect, the present disclosure is directed to a compound comprising a small artificial RNA (smartRNA) oligonucleotide comprising or consisting of a partially double stranded ribonucleic acid (dsRNA), the partially dsRNA comprising or consisting of a sense strand and an antisense strand,
wherein the sense strand comprises or consists of a first contiguous nucleotide sequence comprising or consisting of, from 5' to 3':
   - a "stem I" nucleotide sequence comprising a "stem I" region of complementarity;
   - a "C box" nucleotide sequence comprising a "C box" region of complementarity;
   - a "D' guide" nucleotide sequence comprising a "D' guide" region of complementarity;
   - a "D' box" nucleotide sequence comprising a "D' box" region of complementarity;
   - a "stem II" nucleotide sequence comprising a "stem II" region of complementarity;
wherein the antisense strand comprises or consists of a second contiguous nucleotide sequence comprising or consisting of, from 5' to 3':
   - a "stem II" nucleotide sequence comprising a "stem II" region of complementarity;
   - a "C' box" nucleotide sequence comprising a "C' box" region of complementarity;
   - a "D guide" nucleotide sequence comprising a "D guide" region of complementarity;
   - a "D box" nucleotide sequence comprising a "D box" region of complementarity;
   - a "stem I" nucleotide sequence comprising a "stem I" region of complementarity;
wherein the sense strand "stem I" and the antisense strand "stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the sense strand "stem II" and the antisense strand "stem II" regions of complementarity are at least partially complementary to each other and thereby can anneal at least partially to each other to form an at least partially double-stranded "stem II" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

In a second aspect, the present disclosure is directed to a compound comprising a small artificial RNA (smartRNA) oligonucleotide comprising or consisting of a partially double stranded ribonucleic acid (dsRNA), the partially dsRNA comprising or consisting of a sense strand and an antisense strand,
wherein the sense strand comprises or consists of a first contiguous nucleotide sequence comprising or consisting of, from 5' to 3':
   - a "stem I" nucleotide sequence comprising a "stem I" region of complementarity;
   - a "C box" nucleotide sequence comprising a "C box" region of complementarity;
   - a "D' guide" nucleotide sequence comprising a "D' guide" region of complementarity;
   - a "D' box" nucleotide sequence comprising a "D' box" region of complementarity;
wherein the antisense strand comprises or consists of a second contiguous nucleotide sequence comprising or consisting of, from 5' to 3':
   - a "C' box" nucleotide sequence comprising a "C' box" region of complementarity
   - a "D guide" nucleotide sequence comprising a "D guide" region of complementarity;
   - a "D box" nucleotide sequence comprising a "D box" region of complementarity;
   - a "stem I" nucleotide sequence comprising a "stem I" region of complementarity;
wherein the sense strand "stem I" and the antisense strand "stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

In a third aspect, the present disclosure is directed to a compound comprising a small artificial RNA (smartRNA) oligonucleotide comprising, from 5' to 3':
- a "5' stem I" nucleotide sequence comprising a "5' stem I" region of complementarity;
- a "C box" nucleotide sequence comprising a "C box" region of complementarity;
- a "D' guide" nucleotide sequence comprising a "D' guide" region of complementarity;
- a "D' box" nucleotide sequence comprising a "D' box" region of complementarity;
- a "5' stem II" nucleotide sequence comprising a "5' stem II" region of complementarity;
- optionally a "loop" nucleotide sequence;
- a "3' stem II" nucleotide sequence comprising a "3' stem II" region of complementarity;
- a "C' box" nucleotide sequence comprising a "C' box" region of complementarity;
- a "D guide" nucleotide sequence comprising a "D guide" region of complementarity;
- a "D box" nucleotide sequence comprising a "D box" region of complementarity;
- a "3' stem I" nucleotide sequence comprising a "3' stem I" region of complementarity;

wherein the "5' stem I" and the "3' stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;wherein the "D' box" and the "C' box" regions of complementarity are at least partially complementary to each other and thereby can anneal at least partially to each other to form an at least partially double-stranded "C'/D' box" secondary structure;
wherein the "5' stem II" and the 3' stem II" regions of complementarity are at least partially complementary to each other and thereby can anneal at least partially to each other to form an at least partially double-stranded "stem II" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

In a fourth aspect, the present disclosure is directed to a compound comprising a small artificial RNA (smartRNA) oligonucleotide comprising or consisting of, from 5' to 3':
- a "5' stem I" nucleotide sequence comprising a "5' stem I" region of complementarity;
- a "C box" nucleotide sequence comprising a "C box" region of complementarity;
- a "D' guide" nucleotide sequence comprising a "D' guide" region of complementarity;
- a "D' box" nucleotide sequence comprising a "D' box" region of complementarity
- optionally a "loop" nucleotide sequence;
- a "C' box" nucleotide sequence comprising a "C' box" region of complementarity
- a "D guide" nucleotide sequence comprising a "D guide" region of complementarity;
- a "D box" nucleotide sequence comprising a "D box" region of complementarity;
- a "3' stem I" nucleotide sequence comprising a "3' stem I" region of complementarity;

wherein the "5' stem I" and the "3' stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

In another aspect, the smartRNA comprises:
i. a sense strand "stem I" or "5' stem I" nucleotide sequence comprising or consisting of the nucleotide sequence GAUCAC or GGAUCAC;
ii. a "C-box" nucleotide sequence comprising or consisting of the nucleotide sequence GUGAUGA;
iii. a "D' guide" nucleotide sequence;
iv. a "D'-box" nucleotide sequence comprising or consisting of the nucleotide sequence CUGA;
v. a sense strand "stem II" or "5' stem II" nucleotide sequence comprising or consisting of the nucleotide sequence CCUCAG;
vi. optionally, a loop nucleotide sequence comprising or consisting of the nucleotide sequence GCCU;
vii. an antisense strand "stem I" or "3' stem II" nucleotide sequence comprising or consisting of the nucleotide sequence CUGAGGG;
viii. a "C' box" nucleotide sequence comprising or consisting of the nucleotide sequence GUGAUGA;
ix. a "D guide" nucleotide sequence;
x. a "D-box" nucleotide sequence comprising or consisting of the nucleotide sequence CUGA; and
xi. an antisense strand "stem I" or "3' stem I" nucleotide sequence comprising or consisting of the nucleotide sequence GUGAUC or GUGAUCC;

wherein the "D guide" nucleotide sequence comprises or consist of a first contiguous nucleotide sequence which is complementary to a same-length portion of a first target nucleic acid sequence of a target mRNA;
wherein the "D' guide" nucleotide sequence comprises a second contiguous nucleotide sequence which is complementary to a same-length portion of a second target nucleic acid sequence of the same target mRNA; and
wherein the first or second target nucleic acid sequence comprises the polyA-tail of said target mRNA

In another aspect, the smartRNA comprises:
i. a sense strand "stem I" or "5' stem I" nucleotide sequence comprising or consisting of the nucleotide sequence GGUUGGGUCA (SEQ ID NO: 34);
ii. a "C-box" nucleotide sequence comprising or consisting of the nucleotide sequence AUGAUGA;
iii. a "D' guide" nucleotide sequence;
iv. a "D'-box" nucleotide sequence comprising or consisting of the nucleotide sequence CUGA;
v. optionally, a loop nucleotide sequence comprising or consisting of the nucleotide sequence AGAGAG;
vi. a "C' box" nucleotide sequence comprising or consisting of the nucleotide sequence GUGAUGA;
vii. a "D guide" nucleotide sequence;
viii. a "D-box" nucleotide sequence comprising or consisting of the nucleotide sequence CUGA; and
ix. an antisense strand "stem I" or "3' stem I" nucleotide sequence comprising or consisting of the nucleotide sequence GUCCCAGCCU (SEQ ID NO: 35);

wherein the "D guide" nucleotide sequence comprises or consist of a first contiguous nucleotide sequence which is complementary to a same-length portion of a first target nucleic acid sequence of a target mRNA;
wherein the "D' guide" nucleotide sequence comprises a second contiguous nucleotide sequence which is complementary to a same-length portion of a second target nucleic acid sequence of the same target mRNA; and
wherein the first or second target nucleic acid sequence comprises the polyA-tail of said target mRNA.

In another aspect, the present disclosure is directed to a compound comprising a small artificial RNA (smartRNA) oligonucleotide comprising, or consists of, a sequence selected from the group consisting of SEQ ID NO: 7 to 10.

In another aspect, the present disclosure is directed to a compound comprising a small artificial RNA (smartRNA) oligonucleotide comprising, or consists of, the nucleotide sequence of a sequence selected from the group consisting of SEQ ID NO: 7 to 10.

In another aspect, the present disclosure is directed to a compound comprising a small artificial RNA (smartRNA) oligonucleotide comprising, or consists of, a sequence selected from the group of SEQ ID NO: 85 to 116.

In another aspect, the present disclosure is directed to a compound comprising a small artificial RNA (smartRNA) oligonucleotide comprising, or consists of, the nucleotide sequence of a sequence selected from the group of SEQ ID NO: 85 to 116.

In another aspect, the present disclosure is directed to a compound comprising a small artificial RNA (smartRNA) oligonucleotide comprising, or consists of, a sequence selected from any one of the sequences of Table 4a.

In another aspect, the present disclosure is directed to a compound comprising a small artificial RNA (smartRNA) oligonucleotide comprising, or consists of, the nucleotide sequence of a sequence selected from any one of the sequences of Table 4a.

In another aspect, the present disclosure is directed to a compound comprising a small artificial RNA (smartRNA) oligonucleotide comprising, or consists of, a sequence selected from the group of SEQ ID NO: 117 to 132.

In another aspect, the present disclosure is directed to a compound comprising a small artificial RNA (smartRNA) oligonucleotide comprising, or consists of, the nucleotide sequence of a sequence selected from the group of SEQ ID NO: 117 to 132.

In another aspect, the present disclosure is directed to a compound comprising a small artificial RNA (smartRNA) oligonucleotide comprising, or consists of, a sequence selected from any one of the sequences of Table 4b.

In another aspect, the present disclosure is directed to a compound comprising a small artificial RNA (smartRNA) oligonucleotide comprising, or consists of, the nucleotide sequence of a sequence selected from any one of the sequences of Table 4b.

In another aspect, the present disclosure is directed to a compound comprising a small artificial RNA (smartRNA) oligonucleotide comprising, or consists of, a sequence selected from the group of SEQ ID NO: 133 to 169.

In another aspect, the present disclosure is directed to a compound comprising a small artificial RNA (smartRNA) oligonucleotide comprising, or consists of, the nucleotide sequence of a sequence selected from the group of SEQ ID NO: 133 to 169.

In another aspect, the present disclosure is directed to a compound comprising a small artificial RNA (smartRNA) oligonucleotide comprising, or consists of, a sequence selected from any one of the sequences of Table 4c.

In another aspect, the present disclosure is directed to a compound comprising a small artificial RNA (smartRNA) oligonucleotide comprising, or consists of, the nucleotide sequence of a sequence selected from any one of the sequences of Table 4c.

In another aspect, the present disclosure is directed to a compound comprising a small artificial RNA (smartRNA) oligonucleotide comprising, or consists of, a sequence selected from the group of SEQ ID NO: 170 to 171.

In another aspect, the present disclosure is directed to a compound comprising a small artificial RNA (smartRNA) oligonucleotide comprising, or consists of, the nucleotide sequence of a sequence selected from the group of SEQ ID NO: 170 to 171.

In another aspect, the present disclosure is directed to a compound comprising a small artificial RNA (smartRNA) oligonucleotide comprising, or consists of, a sequence selected from any one of the sequences of Table 5a.

In another aspect, the present disclosure is directed to a compound comprising a small artificial RNA (smartRNA) oligonucleotide comprising, or consists of, the nucleotide sequence of a sequence selected from any one of the sequences of Table 5a.

In another aspect, the present disclosure is directed to a compound comprising a small artificial RNA (smartRNA) oligonucleotide comprising, or consists of, a sequence of SEQ ID NO: 172.

In another aspect, the present disclosure is directed to a compound comprising a small artificial RNA (smartRNA) oligonucleotide comprising, or consists of, the nucleotide sequence of SEQ ID NO: 172.

In another aspect, the present disclosure is directed to a compound comprising a small artificial RNA (smartRNA) oligonucleotide comprising, or consists of, a sequence selected from any one of the sequences of Table 5b.

In another aspect, the present disclosure is directed to a compound comprising a small artificial RNA (smartRNA) oligonucleotide comprising, or consists of, the nucleotide sequence of a sequence selected from any one of the sequences of Table 5b.

In another aspect, the present disclosure is directed to a compound comprising a small artificial RNA (smartRNA) oligonucleotide comprising, or consists of, a sequence selected from the group of SEQ ID NO: 173 to 192.

In another aspect, the present disclosure is directed to a compound comprising a small artificial RNA (smartRNA) oligonucleotide comprising, or consists of, the nucleotide sequence of a sequence selected from the group of SEQ ID NO: 173 to 192.

In another aspect, the present disclosure is directed to a compound comprising a small artificial RNA (smartRNA) oligonucleotide comprising, or consists of, a sequence selected from any one of the sequences of Table 5c.

In another aspect, the present disclosure is directed to a compound comprising a small artificial RNA (smartRNA) oligonucleotide comprising, or consists of, the nucleotide sequence of a sequence selected from any one of the sequences of Table 5c.

In another aspect, the present disclosure is directed to a vector comprising a nucleotide sequence encoding the smartRNA as described herein.

In another aspect, the present disclosure is directed to a host cell expressing the smartRNA as described herein.

In another aspect, the present disclosure is directed to a composition comprising the smartRNA as described herein, the vector as described herein, or the host cell as described herein.

In another aspect, the present disclosure is directed to a smartRNA as described herein, a vector as described herein, or a composition as described herein, for use as a medicament.

In another aspect, the present disclosure is directed to a smartRNA as described herein, a vector as described herein, or a composition as described herein for use in preventing, treating and/or ameliorating a medical condition affecting a subject.

In another aspect, the present disclosure is directed to the use of a compound as described herein, a vector as described herein, a host cell as described herein, or a composition as described herein, for the preparation of a medicament for preventing, treating and/or ameliorating a medical condition affecting a subject.

In another aspect, the present disclosure is directed to a method for preventing, treating and/or ameliorating a medical condition affecting a subject comprising administering a therapeutically or prophylactically effective amount of a compound as described herein, a vector as described herein, a host cell as described herein, or a composition as described herein, to the subject.

In another aspect, the present disclosure is directed to a compound as described herein, a vector as described herein, a host cell as described herein, a composition as described, a use as described herein, or a method as described herein, for use in preventing, treating and/or ameliorating a medical condition affecting a subject, wherein the medical condition is characterized by deficient protein expression of the protein encoded by the target mRNA.

In another aspect, the present disclosure is directed to a method of modulating the stability of a target mRNA, such as increasing the stability of a target mRNA, the method comprising the step of contacting the mRNA with a compound as described herein, a vector as described herein, a host cell as described herein, or a composition as described herein.

In another aspect, the present disclosure is directed to a method of increasing or upregulating the expression of a target mRNA, the method comprising the step of contacting the mRNA with a compound as described herein, a vector as described herein, a host cell as described herein, or a composition as described herein.

In another aspect, the present disclosure is directed to a kit comprising a compound as described herein, a vector as described herein, a host cell as described herein, or a composition as described herein.

In another aspect, the present disclosure is directed to a kit comprising a compound as described herein, a vector as described herein, a host cell as described herein, or a composition as described herein, and instructions for use.

### DESCRIPTION OF FIGURES

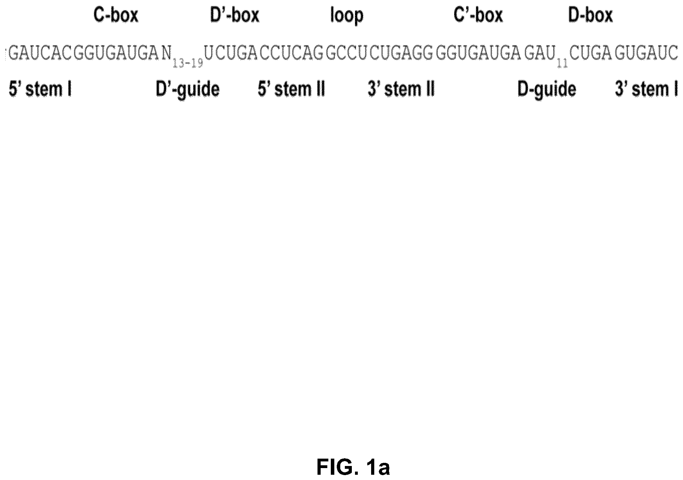

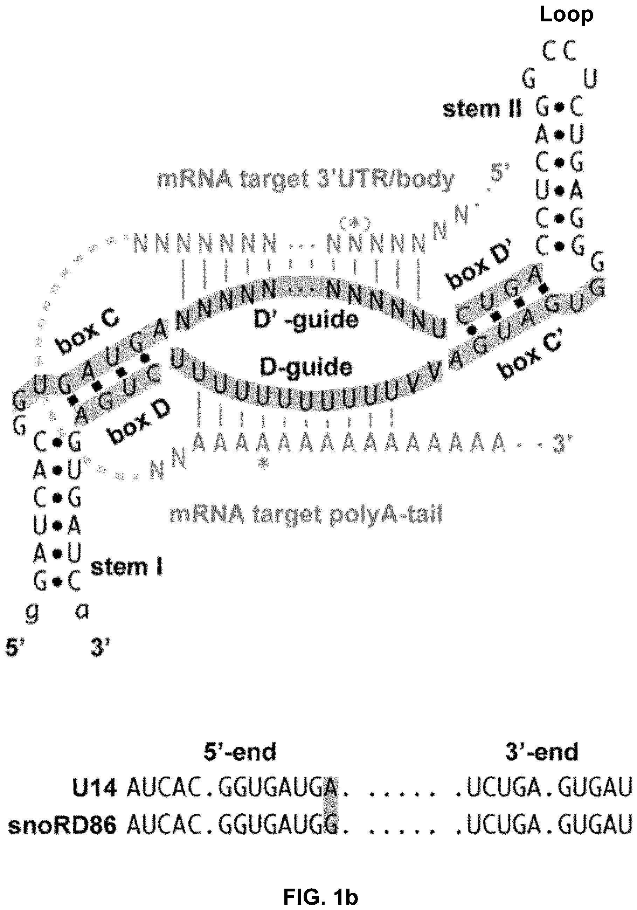

**Figure 1****:** Figure 1a represents an example of the sequence of a compound according to the invention. The sequence comprises, from 5' to 3': a 5' stem I sequence (here GAUCACG), a C-box sequence (here GUGAUGA), a D' guide sequence (here a sequence from 13 to 19 nucleotides in length), a D'-box sequence (here CUGA), a 5' stem II sequence (here CCUCAG), a loop sequence (here GCCU), a 3' stem II sequence (here CUGAGGG), a C'-box sequence (here GUGAUGA), a D guide sequence (here a sequence of 11 nucleotides in length), a D-box sequence (here CUGA), and a 3' stem I sequence (here GUGAUC).
Figure 1b represents the sequence of Figure 1a, with the schematic secondary structure of a pseudo-symmetric smartRNA oligonucleotide, which is designed based on contemporary knowledge about the ability of the RNA to pre-fold into the correct box C/D smartRNP-forming structure. The 'first' and 'second' antisense (AS) sequences correspond to the D'-guide and D-guide, e.g., the first AS corresponds to D'-guide, and the second AS corresponds to the D-guide, or *vice versa.* The interaction between the smartRNP and a target mRNA is indicated.

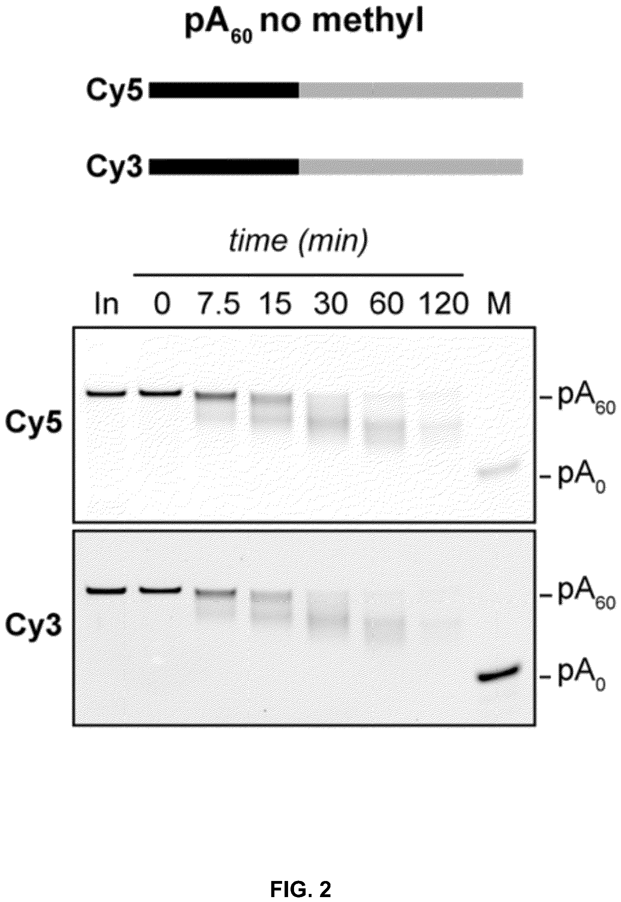

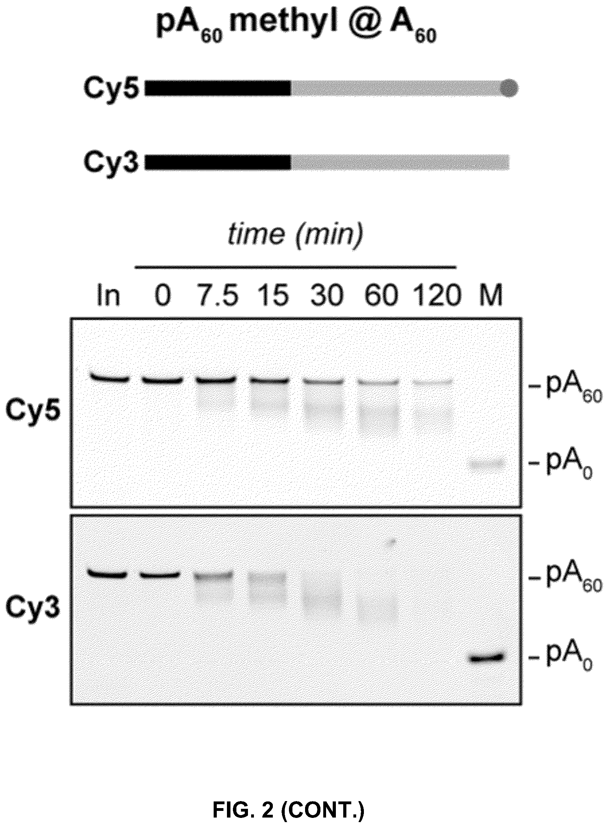

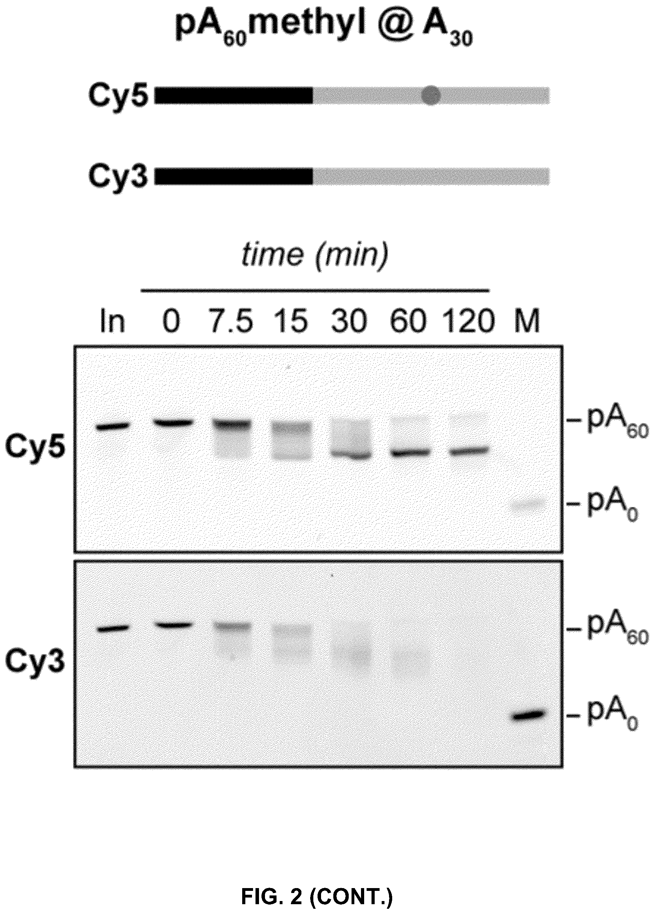

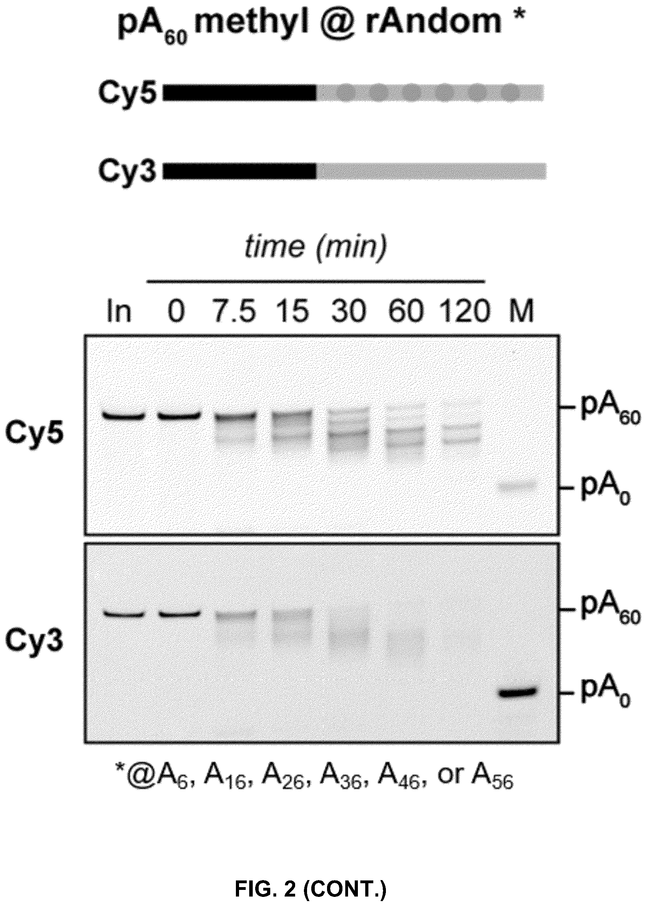

**Figure 2****:** Singular 2'-O-methylations in polyA-tail halts deadenylation in S100 extracts from HeLa cells. Cy5-labelled RNAs with unmodified (SEQ ID NO: 23) or mono-modified polyA-tail at position 30 (SEQ ID NO: 25), 60 (SEQ ID NO: 24) or random (positions 6, 16, 26, 36, 46, or 56) (SEQ ID NO: 26-31) were assayed together with Cy3-labelled RNA with unmodified tail (SEQ ID NO: 22).

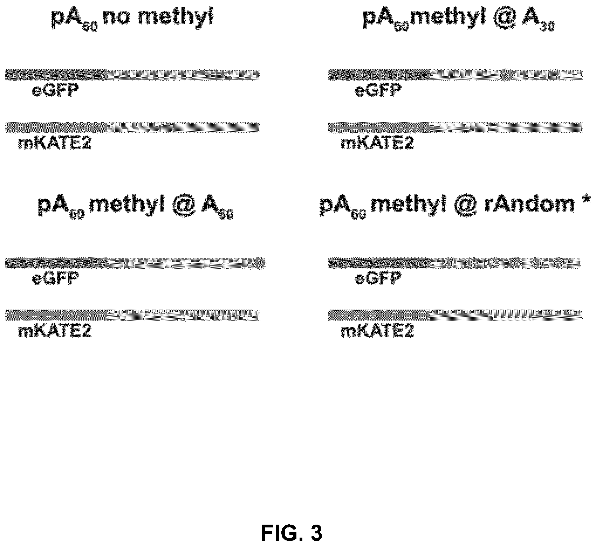

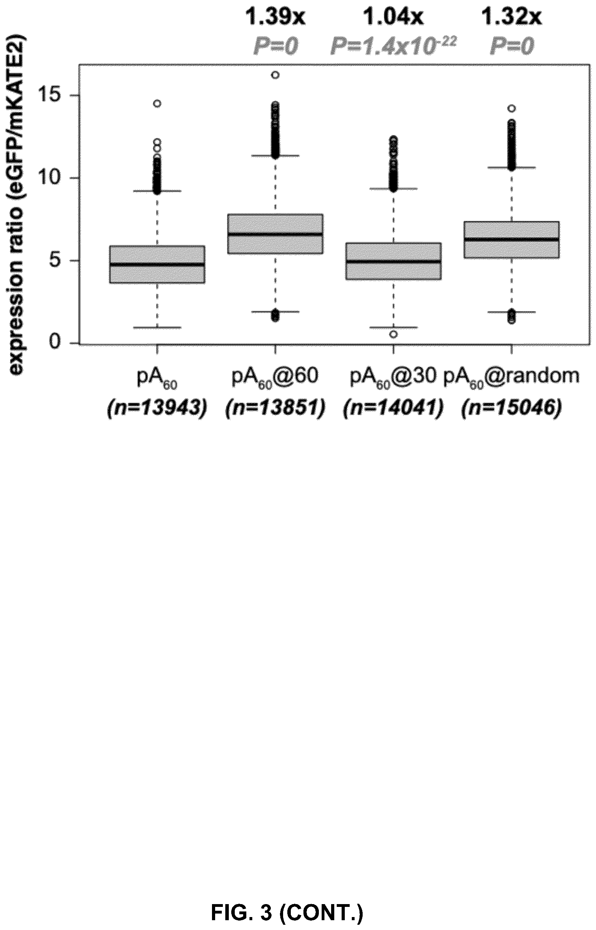

**Figure 3****:** Singular 2'-O-methylations in polyA-tail increases protein output if positioned after the first 30 nucleotides of the polyA-tail. Flow analysis of HeLa cells co-transfected with the indicated eGFP (without or with modifications) and mKATE2 (without modifications) mRNAs. The boxplot displays the distributions of ratios of recorded eGFP vs mKATE2 flow-signal per cell for the four different pA60-tails (numbers (n) of cells for each distribution are indicated below the plot). Box limits represent the first and third quartiles, and the band inside the box is the median. The ends of the whiskers extend the box by 1.5 times the interquartile range. Fold-increase relative to control and absolute P-values based on Wilcoxon tests are displayed above the plot.

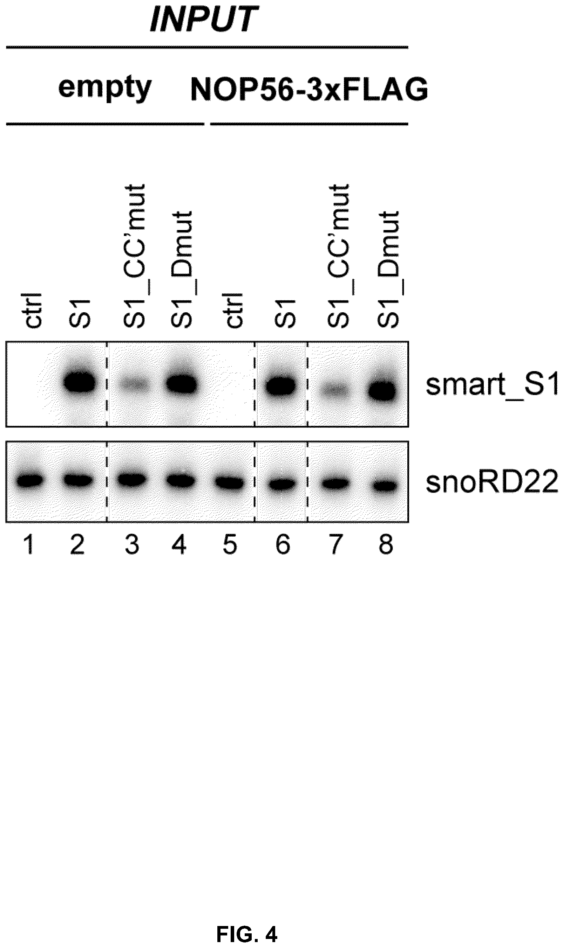

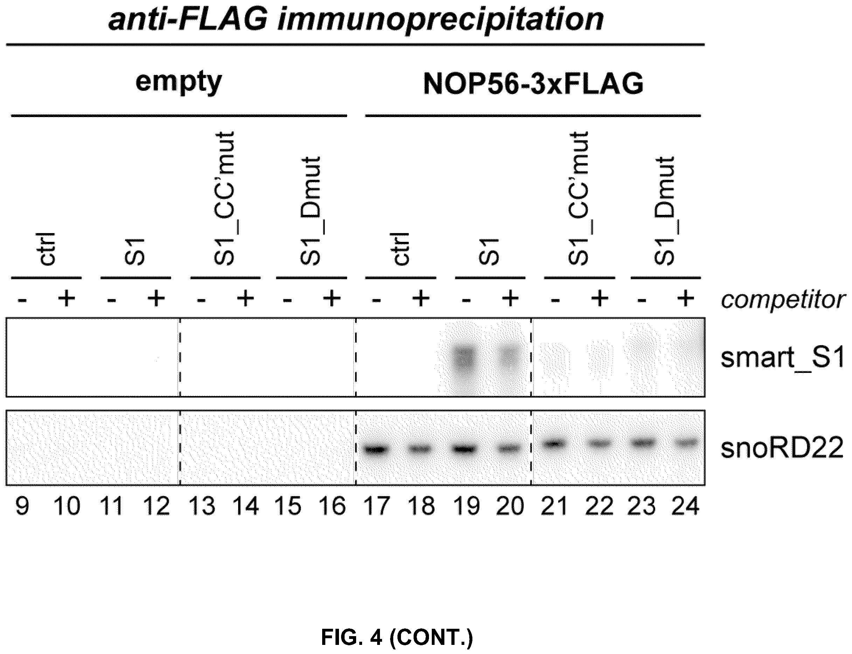

**Figure 4****:** Northern blot demonstrating that a transfected unmodified smartRNA (smart_S1) assembles with endogenous protein factors. RNA co- immunoprecipitation (IP) from 'empty' or NOP56-3xFLAG expressing HEK293 cells using M2 anti-FLAG antibody. co-IPs were conducted in the absence or presence of an excess of unspecific RNA competitor, which had only a minor effect. snoRD22 co-IP was included as a positive control. RNAs from INPUT and IP were analysed in parallel on separate northern blots. However, all samples within the INPUT and IP groups were analysed on the same northern blots with some irrelevant regions excised (cuts indicated by vertical dashed lines). S1 = smart_S1 (smartRNA) (SEQ ID NO: 6), S1_CC'mut = smart_S1 with C- and C'-box mutated (SEQ ID NO: 15), S1_Dmut = smart_S1 with D-box mutated (SEQ ID NO: 16), snoRD22 = endogenous box C/D snoRNA snoRD22 (SEQ ID NO: 5).

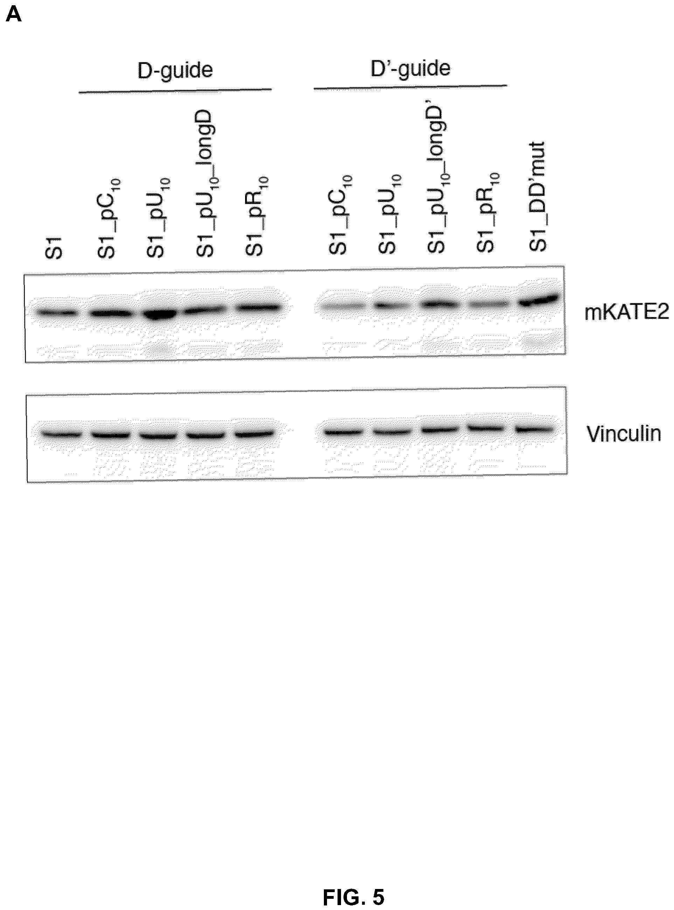

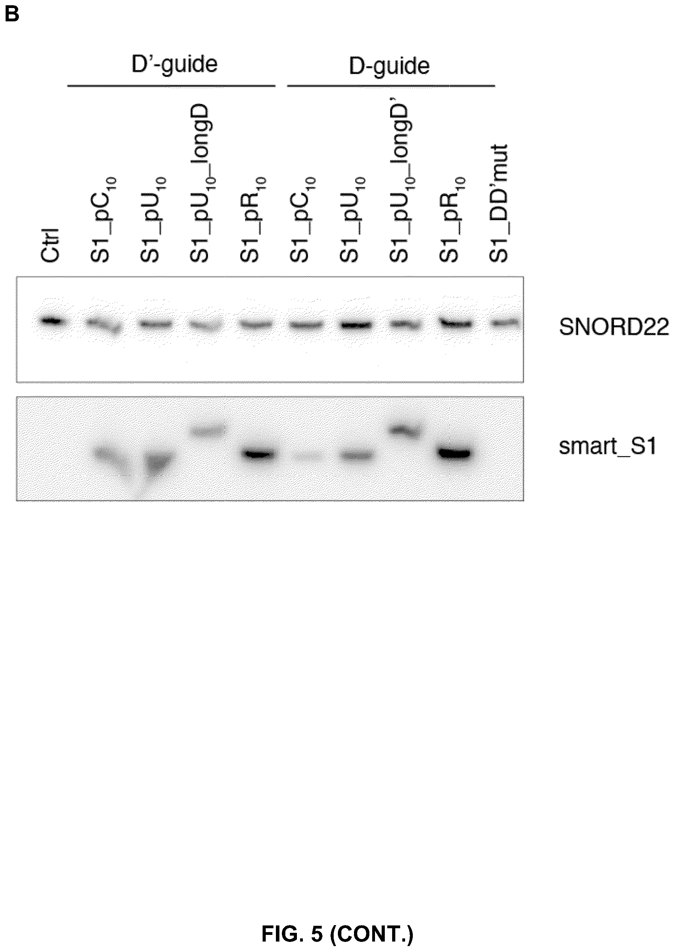

**Figure 5****:** smartRNAs with homo-polymeric U-stretches and long opposing AS elements are stable when expressed from plasmids transfected into HeLa cells. (A) Western blot illustrating the expression of mKATE2 (Vinculin served as an endogenous loading control). (B) Northern blot illustrating the expression of the smartRNA variants. snoRD22 included as an endogenous loading control. S1 = smart_S1 (SEQ ID NO: 6), pC = smart_S1 with polyC-stretch as the D'-guide antisense element (SEQ ID NO: 11) or as the D-guide antisense element (SEQ ID NO: 12), pU = smart_S1 with polyU-stretch as the D'-guide antisense element (SEQ ID NO: 7) or as the D-guide antisense element (SEQ ID NO: 8), pU-long = smart_S1 with polyU-stretch as the D'-guide antisense element (SEQ ID NO: 9) or as the D-guide antisense element (SEQ ID NO: 10) and the other antisense element is comprised in a nucleotide sequence 23 long (23 nts) compared to the generic smart_S1 which has guides comprised in nucleotides sequences 13 nts long, pR = smart_S1 with a randomized nucleotide stretch that does not match any human mRNA targets as the D'-guide antisense element (SEQ ID NO: 13) or as the D-guide antisense element (SEQ ID NO: 14), DDmut = smart_S1 with D- and D'-box mutated (SEQ ID NO: 17).

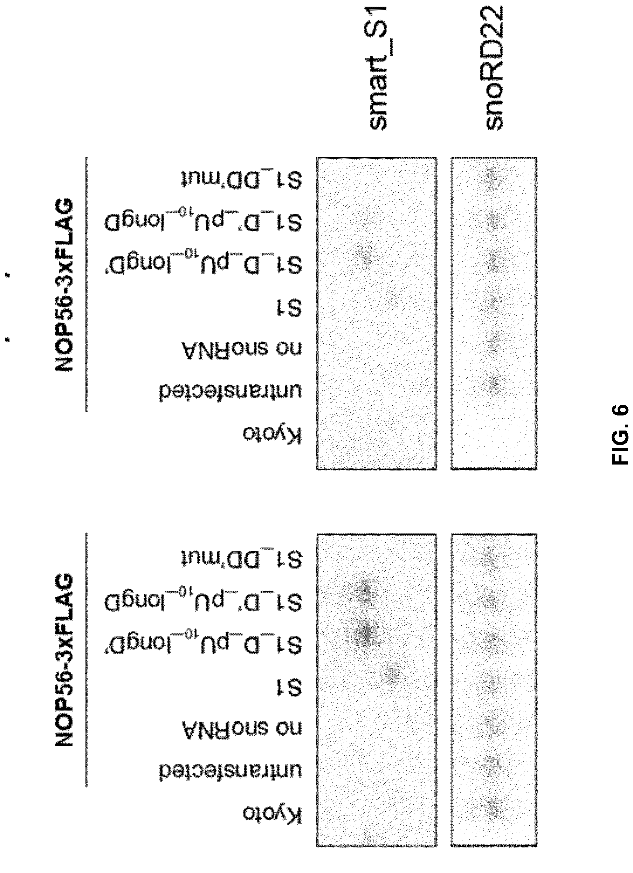

**Figure 6****:** Northern blot demonstrating that a smartRNA with homo-polymeric U-stretches and long opposing AS elements assembles with endogenous protein factors when expressed from plasmids transfected into HeLa cells. RNA co-IP from 'empty' or NOP56-3xFLAG HeLa Kyoto cells using the same smartRNA expression system as in Figure 5. The interaction is dependent on the presence of intact box D/D' elements. snoRD22 co-IP was included as a positive control. No smartRNA = empty expression vector (no smartRNA expressed), S1 = smart_S1 (SEQ ID NO: 6), S1_D_pU long = smart_S1 with polyU-stretch as the D- antisense element and the D'- antisense element is comprised in a nucleotide sequence 23 nt long (SEQ ID NO: 10), S1_D'_pU long = smart_S1 with polyU-stretch as the D'- antisense element and the D- antisense element is comprised in a nucleotide sequence 23 nts long (SEQ ID NO: 9), S1_DD'mut = smart_S1 with D- and D'-box mutated (SEQ ID NO: 17).

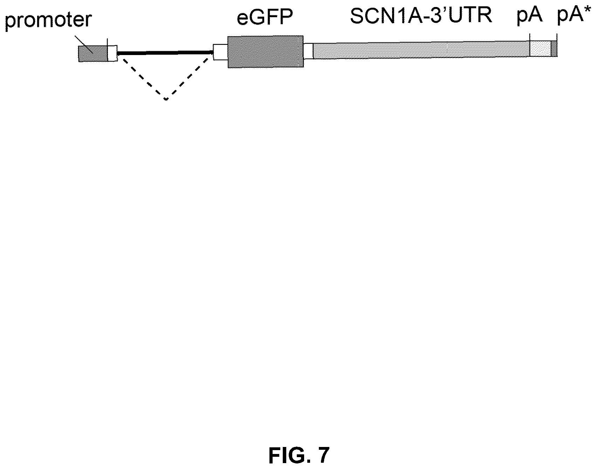

**Figure 7** represents the reporter gene. The chicken β-actin promoter drives the expression of an mRNA with an open reading frame encoding enhanced Green Fluorescent Protein (eGFP) with a codon content that makes the mRNA relative unstable compared to the corresponding codon-optimized open reading frame. The upstream part consists of a vector-encoded exon-intron-exon sequences. The downstream part consists of a major SCN1A 3'UTR (2083 nts) including its natural polyA signal (pA) followed by a vector-encoded β-globin backup polyA signal (pA*).

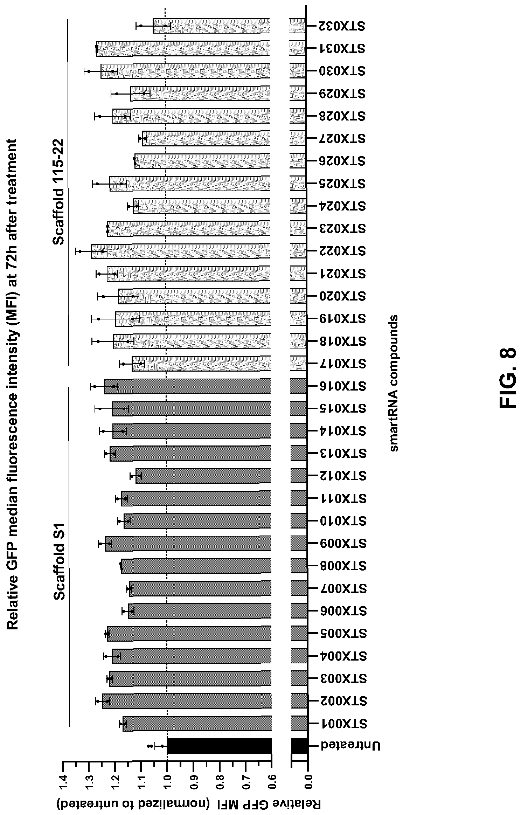

**Figure 8****:** HEK293 cells expressing eGFP mRNA with SCN1A 3''UTR and polyA sequence shows increase in eGFP median fluorescence intensity (MFI) when transfected using RNAiMAX with 10nM of SCN1A-targeting smartRNAs for 72 hours, relative to untreated cells. STX001-STX016 (SEQ ID: 85-100) are based on the original S1 sequence, STX017-STX032 (SEQ ID: 117-132) are based on the human SNORD115-22 sequence. The different compounds are targeting different sites on the SCN1A 3''UTR. The figure shows eGFP MFI from at least 20.000 events relative to that of untreated cells.

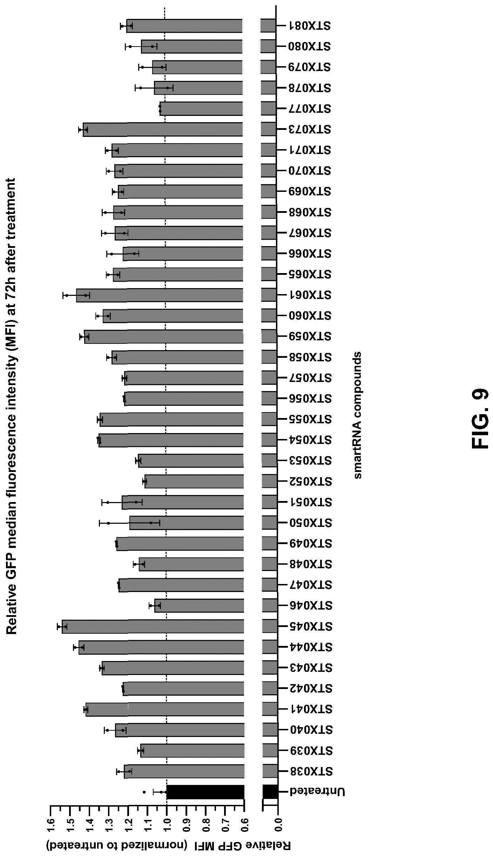

**Figure 9****:** HEK293 cells expressing eGFP mRNA with SCN1A 3''UTR and polyA sequence shows increase in eGFP median fluorescence intensity (MFI) when transfected using RNAiMAX with 10nM of SCN1A-targeting smartRNAs for 72 hours, relative to untreated cells. STX038-STX081 (SEQ ID: 133-169) are based on different snoRNA scaffold sequences, all containing the same D' and D guide sequences. Figure shows eGFP MFI from at least 20.000 events relative to that of untreated cells.

### DEFINITIONS

Several definitions are provided below, which should be used to interpret the disclosure of the invention.

Unless specific definitions are provided, the nomenclature utilized in connection with analytical chemistry, synthetic organic chemistry, and medicinal and pharmaceutical chemistry are those well-known and commonly used in the art. Standard techniques may be used for chemical synthesis, and chemical analysis. Unless otherwise indicated, the following terms have the following meanings:

### Approximately and about

As used herein, the terms "approximately" and "about," as applied to one or more values of interest, refer to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) than the stated reference value unless otherwise stated or otherwise evident from the context (except where such number would exceed 100% of a possible value and it is apparent that this was not intended).

### Independently selected

The term "independently selected" means that each time a selection is made it is made independently of any other selection. For example, if there are twenty nucleotides within a nucleic acid molecule and each is independently selected from adenine (A), cytosine (C), guanine (G) and uracil (U), the selection of any of the four nucleotides at any position is permitted.

### Compound

A "compound" is a physical entity which may have any features as defined further herein. The term "compound" merely denotes a physical entity and does not in itself imply any additional features.

Herein the terms "compound of the invention", "compound", "antisense compound of the invention", "antisense compound", "nucleic acid molecule of the invention", "nucleic acid molecule", "ribonucleic acid of the invention" and "ribonucleic acid" are used interchangeably.

### Antisense compound

An "antisense compound" is a compound that is capable of undergoing hybridization to a target nucleic acid through hydrogen bonding. Examples of antisense compounds include single-stranded and double stranded compounds, such as, antisense oligonucleotides, siRNAs, shRNAs, ssRNAs, smartRNAs, and occupancy-based compounds.

### Nucleic acid molecule

The term "nucleic acid molecule" or "therapeutic nucleic acid molecule" as used herein is defined as it is generally understood by the skilled person as a molecule comprising two or more covalently linked nucleosides (i.e. a nucleotide sequence).

A "nucleic acid molecule" may be a deoxyribose nucleic acid or a ribonucleic acid.

Nucleic acid molecules, such as for siRNAs, shRNAs, smartRNAs, and antisense oligonucleotides, are typically for inhibiting the expression of a target nucleic acid(s).

As used herein, the terms "polynucleotide", "nucleic acid", "nucleic acid molecule" and "nucleic acid sequence" are intended to be synonymous with each other.

Nucleic acid molecules are commonly made in the laboratory by solid-phase chemical synthesis or *in-vitro* transcription, followed by purification and isolation. When referring to a sequence of the nucleic acid molecule, reference is made to the sequence or order of nucleobase moieties, or modifications thereof, of the covalently linked nucleotides or nucleosides. Nucleic acid molecules referred to herein are man-made, are chemically synthesized, and are typically purified or isolated. A nucleic acid molecule may comprise one or more modified nucleosides or nucleotides as described further herein.

A library of nucleic acid molecules is to be understood as a collection of variant nucleic acid molecules. The purpose of the library of nucleic acid molecules can vary. In some embodiments, the library of nucleic acid molecules is composed of oligonucleotides with overlapping nucleobase sequences targeting a region in common, with the purpose of identifying the most potent sequence within the library of nucleic acid molecules. In some embodiments, the library of nucleic acid molecules is a library of nucleic acid molecule design variants (child nucleic acid molecules) of a parent or ancestral nucleic acid molecule, wherein the nucleic acid molecule design variants retain the core nucleobase sequence of the parent nucleic acid molecule.

### Target nucleic acid

The target nucleic acid is the nucleic acid sequence of which modulation is desired. Within the present invention the target nucleic acid may be selected from the group consisting of *AAGAB; ABCB4; ABCD1; ACVRL1; ADNP; AFF2; AHDC1; ALX4; ANK2; ANKRD11; ANKRD17; ANOS1; AP1S2; APC; APOB; AR; ARCN1; ARHGEF9; ARID1A; ARID1B; ARID2; ARSL; ARX; ASH1L; ASXL1; ASXL3; ATM; ATP1A2; ATP7A; ATRX; AUTS2; AVPR2; AXIN2; BAG3; BAP1; BARD1; BCAP31; BCL11A; BCOR; BMPR1A; BMPR2; BPTF; BRCA1; BRCA2; BRIP1; BRPF1; BRWD3; BTK; CACNA1A; CAMTA1; CASK; CD40LG;* CDC73; *CDH1; CDKL5; CoKN1B; CDKN1C; CDKN2A; CHD2; CHD7; CHD8; CHEK2; CHM; CHRDL1; CIC; CLCN5; CLTC; CNKSR2; CNOT1; COL1A1; COL2A1; COL3A1; COL4A5; COL5A1; CREBBP; CTCF; CTNNA1; CTNNB1; CUL3; CUL4B; CYBB; CYLD; DCX; DDX3X; DDX41; DICER1; DLG3; DLG4; DLL1; DMD; DSP; DYRK1A; EBF3; EBP; EDA; EFNB1; EFTUD2; EHMT1; ELN; ENG; EP300; ERF; ETV6; EXT1; EXT2; EYA1; EYA4; F8; F9; FANCB; FBN1; FGD1; FGF10; FGFR1; FH; FLCN; FLG; FLNA; FLNC; FMR1; FOXC1; FOXC2; FOXF1; FOXG1; FOXL2; FOXP1; FRMD7; FZD4; GATA2; GATA3; GATA4; GATA6; GATAD2B; GCH1; GDF5; GK; GLA; GLI2; GLI3; GLMN; GNAS; GPC3; GRIN2A; GRIN2B; GRN; HCCS; HDAC8; HIVEP2; HMBS; HNF1A; HNF1B; HNRNPK; HOXD13; HPRT1; IDS; IGF1R; IKBKG; IL1RAPL1; IQSEC2; IRF6; JAG1; KANSL1; KAT6A; KAT6B; KCNH2; KCNQ1; KCNQ2; KDM5C; KDM6A; KIF11; KIT; KMT2A; KMT2B; KMT2C; KMT2D; KMT2E; KMT5B; L1CAM; LAMP2; LDLR; LEMD3; LMNA; LMX1B; MAGT1; MAX; MBD5; MECP2; MED13L; MEF2C; MEIS2; MEN1; MID1; MITF; MLH1; MNX1; MSH2; MSH6; MSL3; MSX1; MTM1; MYBPC3; MYCN; MYT1L; NAA15; NBEA; NCKAP1; NDP; NEXMIF; NF1; NF2; NFIA; NFIX; NHS; NIPBL; NKX2-5; NOG; NPRL3; NR0B1; NR2F1; NR3C2; NR4A2; NR5A1; NRXN1; NSD1; NSD2; NSDHL; NUS1; NYX; OCRL; OFD1; OPHN1; OTC; OTX2; PAFAH1B1; PAX2; PAX3; PAX6; PAX9; PBX1; PCDH19; PDHA1; PHEX; PHF21A; PHF6; PHF8; PHIP; PITX2; PKD1; PKD2; PKP2; PLP1; PMP22; PMS2; POGZ; POLR1D; PORCN; POU4F3; PQBP1; PRPS1; PRR12; PRRT2; PTCH1; PTCHD1; PTEN; PTHLH; PTPN11; PUF60; PURA; QRICH1; RAB39B; RAD21; RAD51C; RAD51D; RAI1; RASA1; RB1; RET; RP2; RPS17; RPS19; RPS24; RPS26; RPS6KA3; RS1; RUNX1; SALL4; SATB2; SCN1A; SCN2A; SCNSA; SDHAF2; SDHB;* SDHC; *SDHD; SETBP1; SETD1A; SETD2; SETD5; SF3B4; SGCE; SH2D1A; SHANK2; SHANK3; SHH; SHOX; SIN3A; SIX3; SLC16A2; SLC2A1; SLC35A2; SLC6A1; SLC6A8; SLC9A6; SMAD3; SMAD4; SMARCB1; SMC1A; SMS; SON; SOX10; SOX11; SOX2; SOX5; SOX9; SPAST; SPINK1; SPRED1; SRRM2; SRY; STAG2; STK11; STS; STXBP1; SYN1; SYNCRIP; SYNGAP1; TAOK1; TBL 1 XR 1; TBR1; TBX22; TBX3; TBX4; TBX5; TCF12; TCF20; TCF4; TCOF1; TFAP2B; TGFBR1; TGIF1; TIMM8A; TMEM127; TNRC6B; TP53; TRAPPC2; TRIO; TRIP12; TRPS1; TSC1; TSC2; TTN; TWIST1; UBE2A; UBE3A; UPF3B; USP7; USP9X; VHL; WAC; WDFY3; WDR26; WDR45; WT1; XIAP; XIST; ZBTB18; ZC4H2; ZDHHC9; ZEB2; ZIC2; ZIC3; ZMYND11; and*/*or ZNF462.*

### Target sequence

Herein, a "target sequence" refers to a contiguous portion of the sequence of nucleotides present in the target nucleic acid which is complementary to a first or second contiguous nucleotide sequence, within the nucleotide sequence of the compound of the invention.

The compound of the invention comprises a first contiguous nucleotide sequence, such as part of the "D guide" region of complementarity of the compound of the invention, which is complementary to or hybridizes to a first region on the target nucleic acid, such as a target sequence described herein.

The compound of the invention comprises a second contiguous nucleotide sequence, such as part of the "D' guide" region of complementarity of the compound of the invention, which is complementary to or hybridizes to a second region on the target nucleic acid, such as a target sequence described herein.

The target nucleic sequence to which the first contiguous nucleotide sequence is complementary to, or hybridizes to, generally comprises a stretch of contiguous nucleobases of at least 7 nucleotides in length, preferably between 7 and 30 nucleotides in length, such as between 7 and 8, 9, 10, 11, 12, 13, 14, 15, 16, 17, 18, 19, 20, 21, 22, 23, 24, 25, 26, 27, 28, 29, 30 nucleotides in length, such as 7, 8, 9, 10, 11, 12, 13, 14, 15, 16, 17, 18, 19, 20, 21, 22, 23, 24, 25, 26, 27, 28, 29, or 30 nucleotides in length.

The target nucleic sequence to which the second contiguous nucleotide sequence is complementary to, or hybridizes to, generally comprises a stretch of contiguous nucleobases of at least 7 nucleotides in length, preferably between 7 and 30 nucleotides in length, such as between 7 and 8, 9, 10, 11, 12, 13, 14, 15, 16, 17, 18, 19, 20, 21, 22, 23, 24, 25, 26, 27, 28, 29, 30 nucleotides in length, such as 7, 8, 9, 10, 11, 12, 13, 14, 15, 16, 17, 18, 19, 20, 21, 22, 23, 24, 25, 26, 27, 28, 29, or 30 nucleotides in length.

The first and/or second contiguous nucleotide sequence may be between 7 and 15 nucleotides, such as between 7 and 15, 7 and 14, 7 and 13, 7 and 12, 7 and 11, 7 and 10, 7 and 9, 7 and 8, 8 and 15, 8 and 14, 8 and 13, 8 and 12, 8 and 11, 8 and 10, 8 and 9, 9 and 15, 9 and 14, 9 and 13, 9 and 12, 9 and 11, 9 and 10, 10 and 15, 10 and 14, 10 and 13, 10 and 12, 10 and 11, 11 and 15, 11 and 14, 11 and 13, 11 and 12, 12 and 15, 12 and 14, 12 and 13, 13 and 15, 13 and 14, or between 14 and 15 nucleotides in length.

The first and/or second contiguous nucleotide sequence may be between 12 and 30 nucleotides in length, such as between 12 and 30, 13 and 30, 14 and 30, 15 and 30, 16 and 30, 17 and 30, 18 and 30, 19 and 30, 20 and 30, 21 and 30, 22 and 30, 23 and 30, 24 and 30, 25 and 30, 26 and 30, 27 and 30, 28 and 30, 29 and 30, 12 and 29, 13 and 29, 14 and 29, 15 and 29, 16 and 29, 17 and 29, 18 and 29, 19 and 29, 20 and 29, 21 and 29, 22 and 29, 23 and 29, 24 and 29, 25 and 29, 26 and 29, 27 and 29, 28 and 29, 12 and 28, 13 and 28, 14 and 28, 15 and 28, 16 and 28, 17 and 28, 18 and 28, 19 and 28, 20 and 28, 21 and 28, 22 and 28, 23 and 28, 24 and 28, 25 and 28, 26 and 28, 27 and 28, 12 and 27, 13 and 27, 14 and 27, 15 and 27, 16 and 27, 17 and 27, 18 and 27, 19 and 27, 20 and 27, 21 and 27, 22 and 27, 23 and 27, 24 and 27, 25 and 27, 26 and 27, 12 and 26, 13 and 26, 14 and 26, 15 and 26, 16 and 26, 17 and 26, 18 and 26, 19 and 26, 20 and 26, 21 and 26, 22 and 26, 23 and 26, 24 and 26, 25 and 26, 12 and 25, 13 and 25, 14 and 25, 15 and 25, 16 and 25, 17 and 25, 18 and 25, 19 and 25, 20 and 25, 21 and 25, 22 and 25, 23 and 25, 24 and 25, 12 and 24, 13 and 24, 14 and 24, 15 and 24, 16 and 24, 17 and 24, 18 and 24, 19 and 24, 20 and 24, 21 and 24, 22 and 24, 23 and 24, 12 and 23, 13 and 23, 14 and 23, 15 and 23, 16 and 23, 17 and 23, 18 and 23, 19 and 23, 20 and 23, 21 and 23, 22 and 23, 12 and 22, 13 and 22, 14 and 22, 15 and 22, 16 and 22, 17 and 22, 18 and 22, 19 and 22, 20 and 22, 21 and 22, 12 and 21, 13 and 21, 14 and 21, 15 and 21, 16 and 21, 17 and 21, 18 and 21, 19 and 21, 20 and 21, 12 and 20, 13 and 20, 14 and 20, 15 and 20, 16 and 20, 17 and 20, 18 and 20, 19 and 20, 12 and 19, 13 and 19, 14 and 19, 15 and 19, 16 and 19, 17 and 19, 18 and 19, 12 and 18, 13 and 18, 14 and 18, 15 and 18, 16 and 18, 17 and 18, 12 and 17, 13 and 17, 14 and 17, 15 and 17, 16 and 17, 12 and 16, 13 and 16, 14 and 16, 15 and 16, 12 and 15, 13 and 15, 14 and 15, 12 and 14, 13 and 14, or between 12 and 13 nucleotides in length.

The length of the first contiguous nucleotide sequence and of the second contiguous nucleotide sequence may be selected independently from each other; e.g. when one of the first or second contiguous nucleotide sequence is e.g. between 7 and 15 nucleotides in length, the other one is between 12 and 30 nucleotides in length.

### Oligonucleotide

The term "oligonucleotide" as used herein is defined, as is generally understood by the skilled person, as a molecule comprising two or more covalently linked nucleosides. Such covalently bound nucleosides may also be referred to as nucleic acid molecules or oligomers.

Oligonucleotides are commonly made in the laboratory by solid-phase chemical synthesis followed by purification. When referring to a sequence of the oligonucleotide, reference is made to the sequence or order of nucleobase moieties, or modifications thereof, of the covalently linked nucleotides or nucleosides. Oligonucleotides as described herein are obtained by *in vitro* transcription (IVT) but they also can be man-made, chemically synthesized, and typically purified or isolated. Oligonucleotides may comprise one or more modified nucleosides or nucleotides.

### Nucleotide sequence

The term "nucleotide sequence of SEQ ID NO..." refers to the sequence of nucleotides of a given SEQ ID NO, independently of any modified/unmodified nucleobase and/or modified/unmodified internucleoside linkage that would be defined by said given SEQ ID NO in accordance with WIPO Standard 26 (ST.26).

For example, when the application defines a compound *"wherein the D'guide nucleotide sequence comprises, or consists of a nucleotide sequence selected from the group consisting of the nucleotide sequences* of *SEQ ID NO:* 76 *to* 84", it is to be understood that said compound comprises, or consists of, only the nucleotide sequence of SEQ ID NO: 76 to 84, not limited to any corresponding modified/unmodified nucleobase and/or modified/unmodified internucleoside linkage that would be defined in SEQ ID NO: 76 to 84 in accordance with WIPO Standard 26 (ST.26)..

Similarly, when the application defines a compound *"comprising, or consists of a nucleotide sequence selected from any one of the nucleotide sequences of Table 4a",* it is to be understood that said compound comprises, or consists of, only the nucleotide sequence of a given SEQ ID NO comprised in Table 4a, not limited to any modified/unmodified nucleobase and/or modified/unmodified internucleoside linkage that would be defined in said given SEQ ID NO in accordance with WIPO Standard 26 (ST.26).

Conversely, when the application defines a compound *"wherein the D' guide nucleotide sequence comprises, or consists of a sequence selected from the group consisting* of *SEQ ID NO:* 76 *to* 84", it is to be understood that said compound comprises or consists of the nucleotide sequence defined by SEQ ID NO: 76 to 84, i.e. limited to the modified/unmodified nucleobase and/or modified/unmodified internucleoside linkage defined in SEQ ID NO: 76 to 84, in accordance with WIPO Standard 26 (ST.26).

The same way, when the application defines a compound "comprising, or consists of a sequence selected from any one of the sequences of Table 4a", it is to be understood that said compound comprises, or consists of, a given SEQ ID NO comprised in Table 4a, i.e. limited to the modified/unmodified nucleobase and/or modified/unmodified internucleoside linkage defined in said given SEQ ID NO, in accordance with WIPO Standard 26 (ST.26).

### Example:

SEQ ID NO: X = AtGCATgC
Lower case a, c, g or u indicates 2'-O-methyl modified nucleotide.
Upper case A, C, G or U indicates DNA or RNA nucleotide.
All internucleoside linkages are phosphodiester internucleoside linkages.

Therefore, "a *compound comprising, or consists of, a nucleotide sequence selected from the group consisting of the nucleotide sequences* of *SEQ ID NO: X'* means a compound comprising, or consisting of, the nucleotide sequence of AtGCATgC , i.e. a sequence of ATGCATGC (i.e. not limited to any modified/unmodified nucleobase and/or modified/unmodified internucleoside linkage as defined in SEQ ID NO: X in accordance with WIPO Standard 26 (ST.26)).

Conversely, "*a* c*ompound comprising, or consisting of, a sequence selected from the group consisting* of *SEQ ID NO: X"* means a compound comprising, or consisting of, a sequence of AtGCATgC (i.e. limited to the modified/unmodified nucleobase and/or modified/unmodified internucleoside linkage defined in SEQ ID NO: X in accordance with WIPO Standard 26 (ST.26), i.e. lower case a, c, g or u indicating 2'-O-methyl modified nucleotide, upper case A, C, G or U indicating DNA or RNA nucleotide, and all internucleoside linkages being phosphodiester internucleoside linkages).

In case of discrepancy between a STX number and a SEQ ID NO, the STX number should prevail.

### Naturally occurring variant

The term "naturally occurring variant" refers to variants of a gene or transcript (e.g. mRNA) which originate from the same genetic loci as the target nucleic acid, but may differ for example, by virtue of degeneracy of the genetic code causing a multiplicity of codons encoding the same amino acid, or due to alternative splicing of pre-mRNA, or the presence of polymorphisms, such as single nucleotide polymorphisms, and allelic variants. The nucleic acid of the invention may target the target nucleic acid and naturally occurring variants thereof.

### Ribonucleic acid

A "ribonucleic acid" as described herein is a type of nucleic acid molecule comprising predominantly ribonucleotides, i.e. nucleotides comprising a ribose sugar.

A ribonucleic acid comprises at least about 50% ribonucleotides, such as at least about 60%, at least about 70%, at least about 80%, at least about 90%, at least about 95%, at least about 99% or about 100% ribonucleotides. The ribonucleic acid may also comprise one or more deoxyribose containing nucleotides (i.e. DNA nucleotides), such as one, two, three, four, five, six, seven, eight, nine, ten or more deoxyribose containing nucleotides.

### Portion

A *"portion"* means a defined number of contiguous (i.e., linked) nucleobases of a nucleic acid. In certain embodiments, a portion is a defined number of contiguous nucleobases of a target nucleic acid.

### Nucleotides and nucleosides

Nucleotides and nucleosides are the building blocks of nucleic acid molecules, oligonucleotides and polynucleotides, and for the purposes of the present invention include both naturally occurring and non-naturally occurring nucleotides and nucleosides.

Nucleotides, such as DNA and RNA nucleotides, comprise a deoxyribose or ribose sugar moiety, a nucleobase moiety and one or more phosphate groups.

Nucleosides comprise a deoxyribose or ribose sugar moiety and a nucleobase moiety.

Nucleosides and nucleotides may also interchangeably be referred to as "units", "monomers", "bases" or "nucleobases".

### Single stranded

The term "single stranded" is generally understood by the skilled person in the art as a nucleic acid having only one strand. Especially it is understood that a single stranded nucleic acid can form hairpins or intermolecular duplex structures (duplex between two sequences of the same oligonucleotide), notably through the complementarity of "regions of complementarity" sequences within the nucleic acid.

### Double stranded

The term "double stranded" is generally understood by the skilled person in the art as a nucleic acid having two strands, e.g. a sense strand and an antisense strand. Especially it is understood that a double stranded nucleic acid can form intermolecular duplex structures, notably through the complementarity of a "region of complementarity" of one strand and a "regions of complementarity" of another strand.

The two strands may hybridize along at least about 50% of the length of the shortest strand, such as at least about 60%, at least about 70%, at least about 80%, at least about 90%, at least about 95%, at least about 99% or about 100% of the length of the shortest strand.

There is no requirement for the two strands to be the same length or to hybridize along the entire length of either strand.

### Antisense and sense strand

The antisense strand is one strand of a double-stranded nucleic acid and is substantially complementary to the sense strand.

The sense strand is the other strand of a double-stranded nucleic acid and is substantially complementary to the antisense strand.

### Regions of complementarity

Within a single-stranded compound of the invention, there are regions of complementarity. A "region of complementarity" is a region of the single-stranded oligonucleotide (e.g. "5' stem I" region of complementarity) containing multiple nucleotides that are complementary with multiple nucleotides of another "region of complementarity" (e.g. "3' stem I" region of complementarity) of the same single-stranded oligonucleotide.

Within the double-stranded compound of the invention, there are regions of complementarity. A "region of complementarity" is a region of one strand of the double-stranded oligonucleotide (e.g. "5' stem I" region of complementarity of the sense strand) containing multiple nucleotides that are complementary with multiple nucleotides of another "region of complementarity" of the other strand of the same double-stranded oligonucleotide (e.g. "3' stem I" region of complementarity of the antisense strand).

Regions of complementarity may be between 0 and 30 nucleotides in length, such as between 0 and 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length.

When a given "region of complementarity" is 0 nucleotides in length, it is to be understood that said given "region of complementarity" is absent from the compound of the invention.

The same applies when a given "nucleotide sequence" (e.g. "5' stem I" and/or "3' stem I" nucleotide sequences) is defined as being 0 nucleotides in length, i.e. said nucleotide sequence (e.g. "5' stem I" and/or "3' stem I" nucleotide sequences) is absent from the compound of the invention.

The nucleotide sequences within two "regions of complementarity" complementary to each other are at least about 50% complementary across the length of the region of complementarity, such as at least about 60%, at least about 70%, at least about 80%, at least about 90%, at least about 95%, at least about 99% or about 100% complementary.

Within two "regions of complementarity" complementary to each other, there may be one or more mismatches, such as one, two, three, four, five, six, seven, eight, nine, ten or more mismatches.

### Secondary structure

The term stem secondary structure as used herein refers to a duplex formed by hybridization of fully or partially "regions of complementarity" complementary to each other within the compound of the invention.

### C/D box

The term C/D box secondary structure as used herein refers to a structure formed by hybridization of C box and D box regions of complementarity to each other, to form a stem-bulge-stem structure including a kink-turn secondary structure. The same applies to the term "C'/D' box", with reference to the hybridization of C' box and D' box regions of complementarity to each other.

### 5'-untranslated region (5'UTR)

The term "5'-untranslated region" or "5'UTR" as used herein is typically understood to be a particular section of messenger RNA (mRNA). It is located 5' of the open reading frame of the mRNA. Typically, the 5'UTR starts with the transcriptional start site and ends one nucleotide before the start codon of the open reading frame. The 5'UTR may comprise elements for controlling gene expression, also called regulatory elements. Such regulatory elements may be, for example, ribosomal binding sites or a 5'-Terminal Oligopyrimidine Tract. The 5'UTR may be post-transcriptionally modified, for example by addition of a 5'-CAP.

### Open reading frame (ORF)

The term "open reading frame (ORF)" as used herein may typically be a sequence of several nucleotide triplets, which may be translated into a peptide or protein. An open reading frame preferably contains a start codon, i.e. a combination of three subsequent nucleotides coding usually for the amino acid methionine (ATG), at its 5'-end and a subsequent region, which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present disclosure is preferably a nucleotide sequence, consisting of a number of nucleotides that may be divided by three, which starts with a start codon (e.g., ATG) and which preferably terminates with a stop codon (e.g., TAA, TGA, or TAG). The open reading frame may be isolated, or it may be incorporated in a longer nucleic acid sequence, for example in a vector or an mRNA. An open reading frame may also be termed "protein coding region". In the context of mRNA, it is understood that the nucleotide T of the above codons is to be replaced by U.

### 3'-untranslated region (3'UTR)

The term "3'-untranslated region" or "3'UTR" as used herein is typically the part of an mRNA which is located between the protein coding region (i.e., the open reading frame) and the polyA-tail of the mRNA. A 3'UTR of the mRNA is not translated into an amino acid sequence. The 3'UTR sequence is generally encoded by the gene transcribed into the respective mRNA during the gene expression process.

### PolyA-tail

The term "polyA-tail" as used herein refers to a monotonous sequence consisting predominantly of adenosine nucleotides enzymatically added to the 3'end of an mRNA. It is located 3'of the 3'UTR and polyadenylation signal. Typically, the poly(A) tail of a mammalian mRNA contains up to about 250 adenine nucleotides., such as between 30 and 250, such as around 60.

### Contiguous nucleotide sequence

The term "contiguous nucleotide sequence" refers to a region of a strand of the ribonucleic acid which is complementary to another region of the same or another strand of the ribonucleic acid, and *vice versa.*

The term is used interchangeably herein with the term "contiguous nucleobase sequence".

Within a single-stranded compound of the invention, the term "contiguous nucleotide sequence" refers to a region of the ribonucleic acid which is complementary to another region of the same ribonucleic acid, and *vice versa* (e.g. "5' stem I" and 3' stem I" sequences that belong to the same single-stranded ribonucleic acid).

Within a double-stranded compound of the invention, the term "contiguous nucleotide sequence" refers to a region of the antisense strand of the ribonucleic acid which is complementary to a region of the sense strand of the ribonucleic acid, and *vice versa* (e.g. "5' stem I" and 3' stem I" sequences of the antisense strand of the ribonucleic acid and of the sense strand of the ribonucleic acid, respectively).

The term "contiguous nucleotide sequence" also refers to a region of a compound of the invention which is complementary to a region of the target nucleic acid (e.g. first contiguous nucleotide sequence of the "D guide" complementary to a same-length portion of a first target nucleic acid sequence of a target mRNA).

A "contiguous nucleotide sequence" can be represented by a symbol "N_{x-y}", where "x-y" represents the length range of said contiguous nucleotide sequence.

For example, the term "N₇₋₃₀" represents an undefined contiguous nucleotide sequence (i.e. not limited to a particular nucleotide sequence, modified nucleotide, or modified internucleoside linkage) of 7 to 30 nucleotides in length.

### Loop

Stem-loop intramolecular base pairing is a pattern that can occur in single-stranded RNA. The structure is also known as a hairpin or hairpin loop. It occurs when two regions of the same strand, usually complementary in nucleotide sequence when read in opposite directions, base-pair to form a double helix that ends in an unpaired loop. The resulting structure is a key building block of many RNA secondary structures.

In the present disclosure, a stem-loop structure occurs with the formation of the stem II structure (by complementarity of the 5' stem II and the 3' stem II nucleotide sequences), thereby defining the stem II structure and the loop structure such as illustrated in Figure 1b.

### Nick

The term "nick" refers to a discontinuity in a double stranded DNA molecule where there is no phosphodiester bond between adjacent nucleotides of one strand.

A compound of the invention may comprise one or more nicks.

A single-stranded compound of the invention may comprise one or more nicks.

A double-stranded compound of the invention may comprise one or more nicks, such as the antisense strand may comprise one or more nicks, and/or the sense strand may comprise one or more nicks.

The one or more nicks may be independently located between:
- the "5' stem I" nucleotide sequence comprising a "5' stem I" region of complementarity and the "C box" nucleotide sequence comprising a "C box" region of complementarity;
- the sense strand "stem I" nucleotide sequence comprising a sense strand "stem I" region of complementarity and the "C box" nucleotide sequence comprising a "C box" region of complementarity;
- the "C box" nucleotide sequence comprising a "C box" region of complementarity and the "D' guide" nucleotide sequence comprising a "D' guide" region of complementarity;
- the "D' guide" nucleotide sequence comprising a "D' guide" region of complementarity and the "D' box" nucleotide sequence comprising a "D' box" region of complementarity
- the "D' box" nucleotide sequence comprising a "D' box" region of complementarity and the "5' stem II" nucleotide sequence comprising a "5' stem II" region of complementarity;
- the "D' box" nucleotide sequence comprising a "D' box" region of complementarity and the sense strand "stem II" nucleotide sequence comprising a sense strand "stem II" region of complementarity;
- optionally (when a loop sequence is present) the "5' stem II" nucleotide sequence comprising a "5' stem II" region of complementarity and the "loop" nucleotide sequence;
- optionally (when a loop sequence is present) the "loop" nucleotide sequence and the "3' stem II" nucleotide sequence comprising a "3' stem II" region of complementarity;
- the "3' stem II" nucleotide sequence comprising a "3' stem II" region of complementarity and the "C' box" nucleotide sequence comprising a "C' box" region of complementarity;
- the antisense strand "stem II" nucleotide sequence comprising an antisense strand "stem II" region of complementarity and the "C' box" nucleotide sequence comprising a "C' box" region of complementarity;
- the "C' box" nucleotide sequence comprising a "C' box" region of complementarity and the "D guide" nucleotide sequence comprising a "D guide" region of complementarity;
- the "D guide" nucleotide sequence comprising a "D guide" region of complementarity and the "D box" nucleotide sequence comprising a "D box" region of complementarity;
- the "D box" nucleotide sequence comprising a "D box" region of complementarity and the "3' stem I" nucleotide sequence comprising a "3' stem I" region of complementarity; or
- the "D box" nucleotide sequence comprising a "D box" region of complementarity and the antisense strand "stem I" nucleotide sequence comprising an antisense strand "stem I" region of complementarity.

The one or more nicks may also be independently located between:
- the "5' stem I" region of complementarity and the "C box" region of complementarity;
- the sense strand "stem I" region of complementarity and the "C box" region of complementarity
- the "C box" region of complementarity and the "D' guide" region of complementarity;
- the "D' guide" region of complementarity and the "D' box" region of complementarity
- the "D' box" region of complementarity and the "5' stem II" region of complementarity;
- the "D' box" region of complementarity and the sense strand "stem II" region of complementarity;
- optionally (when a loop sequence is present) the "5' stem II" region of complementarity and the "loop" nucleotide sequence;
- optionally (when a loop sequence is present) the "loop" nucleotide sequence and the "3' stem II" region of complementarity;
- the "3' stem II" region of complementarity and the "C' box" region of complementarity;
- the antisense strand "stem II" region of complementarity and the "C' box" region of complementarity;
- the "C' box" region of complementarity and the "D guide" region of complementarity;
- the "D guide" region of complementarity and the "D box" region of complementarity;
- the "D box" region of complementarity and the "3' stem I" region of complementarity; or
- the "D box" region of complementarity and the antisense strand "stem I" region of complementarity.

The one or more nicks may also be independently located within:
- the "5' stem I" nucleotide sequence;
- the sense strand "stem I" nucleotide sequence;
- the "C box" nucleotide sequence;
- the "D' guide" nucleotide sequence;
- the "D' box" nucleotide sequence;
- the "5' stem II" nucleotide sequence (when present);
- the sense strand "stem II" nucleotide sequence (when present)
- the "loop" nucleotide sequence (when present);
- the "3' stem II" nucleotide sequence (when present);
- the antisense strand "stem II" nucleotide sequence (when present)
- the "C' box" nucleotide sequence;
- the "D guide" nucleotide sequence;
- the "D box" nucleotide sequence;
- the "3' stem I" nucleotide sequence; or
- the antisense strand "stem I" nucleotide sequence.

The one or more nicks may also be independently located within:
- the "5' stem I" region of complementarity;
- the sense strand "stem I" region of complementarity;
- the "C box" region of complementarity;
- the "D' guide" region of complementarity;
- the "D' box" region of complementarity;
- the sense strand "stem II" region of complementarity (when present);
- the "5' stem II" region of complementarity (when present);
- the "3' stem II" region of complementarity (when present);
- the antisense strand "stem II" region of complementarity (when present);
- the "C' box" region of complementarity;
- the "D guide" region of complementarity;
- the "D box" region of complementarity;
- the "3' stem I" region of complementarity; or
- the antisense strand "stem I" region of complementarity.

### Conjugate moiety

The term "conjugate" as used herein refers to a nucleic acid molecule, such as a ribonucleic acid, which is linked, such as covalently linked, to a non-nucleotide moiety (conjugate moiety or region C or third region). The conjugate moiety may be linked, such as covalently linked, to the nucleic acid molecule directly, or the conjugate moiety may be linked to the nucleic acid via a linker group, such as a linker group comprising one, two or three nucleotides.

Conjugation of the compound of the invention to one or more non-nucleotide moieties may improve the pharmacology of the compound, e.g. by affecting the activity, cellular distribution, cellular uptake or stability of the compound. In some embodiments the conjugate moiety may modify or enhance the pharmacokinetic properties of the compound by improving cellular distribution, bioavailability, metabolism, excretion, permeability, and/or cellular uptake. In particular the conjugate may target the compound to a specific organ, tissue or cell type and thereby enhance the effectiveness of the compound in that organ, tissue or cell type. At the same time the conjugate may serve to reduce activity of the compound in non-target cell types, tissues or organs, e.g. off target activity or activity in non-target cell types, tissues or organs.

Nucleic acid conjugates and their synthesis has also been reported in comprehensive reviews by Manoharan in Antisense Drug Technology, Principles, Strategies, and Applications, S.T. Crooke, ed., Ch. 16, Marcel Dekker, Inc., 2001 and Manoharan, Antisense and Nucleic Acid Drug Development, 2002, 12, 103, both of which are incorporated by reference in their entirety.

In some embodiments, the conjugate moiety is selected from the group consisting of carbohydrates, cell surface receptor ligands, drug substances, hormones, lipophilic substances, polymers, proteins, peptides, toxins (e.g. bacterial toxins), vitamins, viral proteins (e.g. capsids) and combinations thereof.

The terms "attached", "positioned", "linked" and "conjugated" are interchangeable in reference to the nucleic acid molecule and the conjugate moiety.

### Linkers

A linkage, linker or spacer is a connection between two atoms that links one chemical group or segment of interest to another chemical group or segment of interest via one or more covalent bonds. Conjugate moieties can be attached to the double-stranded RNA molecule directly or through a linking moiety (e.g. linker or spacer). The linkers serve to covalently connect a conjugate moiety to a double-stranded RNA molecule or contiguous nucleotide sequence thereof.

Used herein, the terms "linker" and "spacer" are interchangeable.

In some embodiments of the present disclosure, the double-stranded RNA molecule of the invention may comprise a linker (also termed "linker region") which is positioned between the double-stranded RNA molecule and the conjugate moiety. The linker may be attached to the contiguous nucleotide sequence of a strand of the double-stranded RNA molecule complementary to the target nucleic acid and the conjugate moiety.

In some embodiments, the linker is C6.

In some embodiments, the linker is TEG.

In some embodiments, the linker is a dinucleotide. In some embodiments, the linker is a CA dinucleotide.

In some embodiments, the linker is a biocleavable linker. Biocleavable linkers comprise or consist of a physiologically labile bond that is cleavable under conditions normally encountered or analogous to those encountered within a mammalian body. Conditions under which physiologically labile linkers undergo chemical transformation (e.g., cleavage) include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic enzymes or hydrolytic enzymes or nucleases. In some embodiments, the biocleavable linker is susceptible to S1 nuclease cleavage. In some embodiments the nuclease susceptible linker comprises between 1 and 5 nucleosides, such as DNA nucleoside(s) comprising at least two consecutive phosphodiester linkages. Phosphodiester containing biocleavable linkers are described in more detail in WO 2014/076195.

In some embodiments, the linker is not a biocleavable linker. Linkers that are not necessarily biocleavable but primarily serve to covalently connect a conjugate moiety to an oligonucleotide are known. These linkers may comprise a chain structure or an oligomer of repeating units such as ethylene glycol, amino acid units or amino alkyl groups, or a combination thereof. In some embodiments, the linker is an amino alkyl, such as a C2 - C36 amino alkyl group, including, for example C6 to C12 amino alkyl groups.

### Additional 5' and/or 3' nucleosides

The addition of the further 5' and/or 3' nucleosides may be used for the purpose of joining the compound of the invention to a conjugate moiety or another functional group. When used for joining the compound with a conjugate moiety it can serve as a biocleavable linker.

Alternatively, it may be used to provide exonucleoase protection or for ease of synthesis or manufacture.

The further 5' and/or 3' nucleosides may serve as a nuclease susceptible bio-cleavable linker. In some embodiments the additional 5' and/or 3' end nucleotides are linked with phosphodiester linkages, and are DNA or RNA. Nucleotide based bio-cleavable linkers suitable for such use are disclosed in WO2014/076195, which include by way of example a phosphodiester linked DNA dinucleotide. The use of bio-cleavable linkers in poly-oligonucleotide constructs is disclosed in WO2015/113922, where they are used to link multiple antisense constructs within a single oligonucleotide. WO2014/076195 and WO2015/113922 are incorporated by reference in their entirety.

### Modified nucleic acid molecule

The term "modified nucleic acid molecule" encompasses a nucleic acid molecule comprising one or more sugar-modified nucleosides and/or modified internucleoside linkages. The term "chimeric" is a term that has been used in the literature to describe nucleic acid molecules with modified nucleosides, in particular gapmer oligonucleotides.

The nucleic acid molecule or contiguous nucleotide sequence thereof may be unmodified (e.g. RNA or DNA nucleobases) or may include modified nucleobases which function as the shown nucleobase in base pairing, for example 5-methyl cytosine may be used in place of methyl cytosine. Inosine may be used as a universal base.

It is understood that the contiguous nucleobase sequences (motif sequence) can be modified to, for example, increase nuclease resistance and/or binding affinity to the target nucleic acid.

The pattern in which the modified nucleosides (such as high affinity modified nucleosides) are incorporated into the oligonucleotide sequence is generally termed "oligonucleotide design".

### 5-methylcytosine

"5-methylcytosine" or "5-me-C" means a methylated form of the DNA base cytosine (C) in which a methyl group is attached to the fifth carbon of the 6 atoms ring. 5-methylcytosine is a modified nucleobase.

### Modified internucleoside linkage

The term "modified internucleoside linkage" is defined as generally understood by the skilled person as linkages, other than phosphodiester (PO) linkages, which covalently couple two nucleosides together. Nucleotides with modified internucleoside linkage are also termed "modified nucleotides".

The compound of the present disclosure may comprise one or more modified internucleoside linkages such as one or more phosphorothioate, chiral phosphorothioates, diphosphorothioates, phosphorodithioates, phosphoramidates, boranophosphates, methyl phosphonates, chiral methyl phosphonates, sulfonylphosphoramidates, methoxypropylphosphonates, 5'-vinylphosphonates, 5'-methylphosphonates, 5'-phosphorothioates, phosphodiesters, phosphotriesters. 5'-hydroxyphosphonates, Inverted nucleotide linkages (3'-3', and 5'-5'), or 3'-O-methylphosphonatesinternucleoside linkage.

For naturally occurring oligonucleotides, the internucleoside linkage includes phosphate groups creating a phosphodiester bond between adjacent nucleosides. The modified internucleoside linkage may increase the nuclease resistance of the nucleic acid molecules of the present disclosure compared to a phosphodiester linkage. Modified internucleoside linkages are particularly useful in stabilizing nucleic acids for *in vivo* use, and may serve to protect against nuclease cleavage at regions of DNA or RNA nucleosides in the nucleic acid of the present disclosure.

Modified internucleoside linkages may be independently selected from the group comprising phosphorothioate, diphosphorothioate,boranophosphate, chiral phosphorothioates, phosphorodithioates, phosphoramidates, methyl phosphonates, chiral methyl phosphonates, sulfonylphosphoramidates, methoxypropylphosphonates, 5'-vinylphosphonates, 5'-methylphosphonates, 5'-phosphorothioates, phosphodiesters, phosphotriesters. 5'-hydroxyphosphonates, Inverted nucleotide linkages (3'-3', and 5'-5'), and 3'-O-methylphosphonates internucleoside linkage. A phosphorothioate internucleoside linkage is particularly useful due to nuclease resistance, beneficial pharmakokinetics and ease of manufacture.

Each of the design regions may however comprise internucleoside linkages other than phosphorothioate, such as phosphodiester linkages, in particularly in regions where modified nucleosides, such as LNA, protect the linkage against nuclease degradation. Inclusion of phosphodiester linkages, such as one or two linkages, particularly between or adjacent to modified nucleoside units (typically in the non-nuclease recruiting regions) can modify the bioavailability and/or bio-distribution of an oligonucleotide - see WO2008/113832 which is incorporated herein by reference in its entirety.

### Modified nucleoside

The term "modified nucleoside" or "nucleoside modification" as used herein refers to nucleosides modified as compared to the equivalent DNA or RNA nucleoside by the introduction of one or more modifications of the sugar moiety or the (nucleo)base moiety.

The term modified nucleoside may also be used herein interchangeably with the term "nucleoside analogue" or modified "units" or modified "monomers". Nucleosides with an unmodified DNA or RNA sugar moiety are termed DNA or RNA nucleosides herein.

Nucleosides with modifications in the base region of the DNA or RNA nucleoside are still generally termed DNA or RNA if they allow Watson Crick base pairing. Exemplary modified nucleosides include LNA, 2'-O-MOE, 2'OMe and morpholino nucleoside analogues. These are discussed further below.

### Nucleobase

The term nucleobase includes the purine (e.g. adenine and guanine) and pyrimidine (e.g. uracil, thymine and cytosine) moiety present in nucleosides and nucleotides which form hydrogen bonds in nucleic acid hybridization.

In the context of the present disclosure the term nucleobase also encompasses modified nucleobases which may differ from naturally occurring nucleobases, but which are functional during nucleic acid hybridization. In this context "nucleobase" refers to both naturally occurring nucleobases such as adenine, guanine, cytosine, thymidine, uracil, xanthine and hypoxanthine, as well as non-naturally occurring variants. Such variants are for example described in Hirao et al., 2012, Accounts of Chemical Research, 45, 2055-2065 and Bergstrom, 2009, Curr. Protoc. Nucleic Acid Chem., 37, 1.4.1-1.4.32, which are incorporated by reference in their entirety.

In some embodiments the nucleobase moiety is modified by changing the purine or pyrimidine into a modified purine or pyrimidine, such as substituted purine or substituted pyrimidine.

A nucleoside with a modified base may be independently selected from isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine, 2-chloro-6-aminopurine, pseudouridine (Ψ), N1-methylspeudouridine (m1Ψ), 5-methylcytidine (m5C), N6-methyladenosine (m6A), 7-methylguanosine (m7G), 5-methoxyuridine, inosine, purine, xanthine, hypoxanthine, 2,2,7-trimethylguanosine (m3G/TMG cap), pyridin-4-one, pyridin-2-one, phenyl, 2,4,6-trimethoxy benzene, 3-methyl uracil, 2-thiouridine,s dihydrouridine, naphthyl, aminophenyl, 5-alkylcytidines, 5-fluoro-2'-deoxyuridine, 2',2'-difluoro-2'-deoxy nucleosides, 5-alkyluridines (e.g., ribothymidine), 5-halouridine (e.g., 5-bromouridine), 2',4'-difluorotoluyl nucleosides, 6-azapyrimidines, 6-alkylpyrimidines (e.g. 6-methyluridine), 5-nitroindole, and 3-nitropyrrole, preferably pseudouridine (Ψ), N1-methylspeudouridine (m1Ψ), and 5-methylcytidine (m5C).

The nucleobase moieties may be indicated by the letter code for each corresponding nucleobase, e.g. A, T, G, C or U, wherein each letter may optionally include modified nucleobases of equivalent function. For example, in the exemplified nucleic acids, the nucleobase moieties are selected from A, U, G, C, and 5-methyl cytosine. Optionally, for LNA gapmers, 5-methyl cytosine LNA nucleosides may be used. 5-methyl cytosine may be denoted as "E".

Unless otherwise indicated or contradicted by context, in the present disclosure, thymine (T) nucleobases within RNA sequences disclosed herein (e.g. smartRNA, smartRNA structure sequences, or mRNA target sequences) are to be interpreted as uracil (U) nucleobases.

### High affinity modified nucleosides

A high affinity modified nucleoside is a modified nucleoside which, when incorporated into a nucleic acid, enhances the affinity of the nucleic acid for its complementary target, for example as measured by the melting temperature (T^{m}). A high affinity modified nucleoside of the present invention preferably results in an increase in melting temperature between +0.5 to +12°C, more preferably between +1.5 to +10°C and most preferably between +3 to +8°C per modified nucleoside. Numerous high affinity modified nucleosides are known in the art and include for example, many 2' substituted nucleosides as well as locked nucleic acids (LNA) (see e.g. Freier & Altmann, Nucl. Acid Res., 1997, 25, 4429-4443 and Uhlmann; Curr. Opinion in Drug Development, 2000, 3(2), 203-213, which are incorporated herein by reference in their entirety).

### Sugar modifications

The nucleic acid molecules of the invention may comprise one or more nucleosides which have a modified sugar moiety, i.e. a modification of the sugar moiety when compared to the ribose sugar moiety found in DNA and RNA.

Numerous nucleosides with modification of the ribose sugar moiety have been made, primarily with the aim of improving certain properties of nucleic acid, such as affinity and/or nuclease resistance.

The modified sugar modification may be a bicyclic sugar moiety or a non-bicyclic sugar moiety.

Sugar modifications include those where the ribose ring structure is modified, e.g. by replacement with a hexose ring (HNA), or a bicyclic ring.

In a bicyclic sugar moiety there may be a biradical bridge between two carbon atoms of the sugar ring, such as the C2 and C4 carbons on the ribose ring. e.g. a locked nucleic acid (LNA) or a constrained ethyl nucleic acid (cEt).

The bicyclic sugar modification may be independently selected from (LNAs), bridged nucleic acids (BNAs) and analogues thereof, constrained ethyl nucleic acid (cEt), constrained ethyl BNAs (cET-BNAs), Tricyclo-DNA, and constrained methoxyethyl BNAs.

Alternatively, the sugar modification may be a non-bicyclic sugar moiety, which may include an unlinked ribose ring which typically lacks a bond between the C2 and C3 carbons (e.g. UNA).

The non-bicyclic sugar modification may be independently selected from 2'-O-alkyl-RNA, 2'-O-methyl-RNA (2'OMe modified sugar), 2'-alkoxy-RNA, 2'-O-methoxyethyl-RNA, 2'-amino-DNA/RNA, 2'-fluoro-DNA/RNA (2'F modified sugar), arabino nucleic acid (ANA), 2'-fluoro-ANA, Glycol nucleic acid (GNA), inverted RNA (3'-3' linked, or 5'-5' linked), 2'-azido-DNA, 2'-O-propargyl-RNA, L-ribose based nucleotides (mirror image nucleotides), and unlocked nucleic acid (UNA); preferably 2'F modified sugar and/or 2'OMe modified sugar..

Other sugar modified nucleosides include, for example, bicyclohexose nucleic acids (WO 2011/017521, which is incorporated by reference in its entirety) or tricyclic nucleic acids (WO 2013/154798, which is incorporated by reference in its entirety).

Modified nucleosides also include nucleosides where the sugar moiety is replaced with a non-sugar moiety, for example in the case of peptide nucleic acids (PNA), or morpholino nucleic acids.

Sugar modifications also include modifications made via altering the substituent groups on the ribose ring to groups other than hydrogen, or the 2'-OH group naturally found in DNA and RNA nucleosides. Substituents may, for example be introduced at the 2', 3', 4' or 5' positions.

### 2' sugar modified nucleosides

A 2' sugar modified nucleoside is a nucleoside which has a substituent other than H or -OH at the 2' position (2' substituted nucleoside) or comprises a 2' linked biradical capable of forming a bridge between the 2' carbon and a second carbon in the ribose ring, such as LNA (2'- 4' biradical bridged) nucleosides.

Indeed, much focus has been spent on developing 2' sugar substituted nucleosides, and numerous 2' substituted nucleosides have been found to have beneficial properties when incorporated into nucleic acids. For example, the 2' modified sugar may provide enhanced binding affinity and/or increased nuclease resistance to the nucleic acid. Examples of 2' substituted modified nucleosides are 2'-O-alkyl-RNA, 2'-O-methyl-RNA (2'oMe). 2'-alkoxy-RNA, 2'-O-methoxyethyl-RNA (MOE), 2'-amino-DNA, 2'-Fluoro-RNA, and 2'-F-ANA nucleoside. For further examples, please see e.g. Freier & Altmann; Nucl. Acid Res., 1997, 25, 4429-4443 and Uhlmann; Curr. Opinion in Drug Development 2000, 3(2), 203-213, and Deleavey and Damha, Chemistry and Biology 2012, 19, 937, which are incorporated by reference in their entirety.

Below are illustrations of some 2' substituted modified nucleosides.

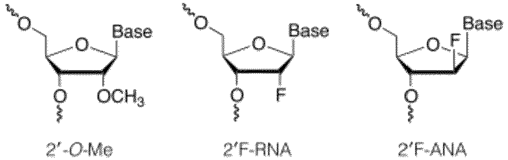

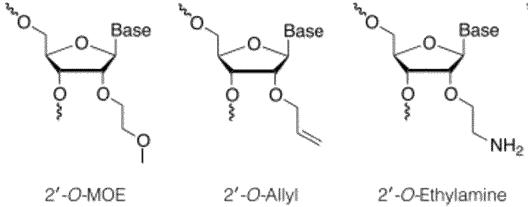

"2'-O-methoxyethyl" (also 2'-MOE and 2'-OCH₂CH₂-OCH₃ and MOE) refers to an O-methoxyethyl modification of the 2' position of a furanose ring. A 2'-O-methoxyethyl modified sugar is a modified sugar.

"2'-MOE nucleoside" (also 2'-0-methoxyethyl nucleoside) means a nucleoside comprising a 2'MOE modified sugar moiety.

### Locked Nucleic Acid Nucleosides (LNA nucleoside)

A "LNA nucleoside" is a 2'- modified nucleoside which comprises a biradical linking the C2' and C4' of the ribose sugar ring of said nucleoside (also referred to as a "2' - 4' bridge"), which restricts or locks the conformation of the ribose ring.

These nucleosides are also termed bridged nucleic acid or bicyclic nucleic acid (BNA) in the literature. The locking of the conformation of the ribose is associated with an enhanced affinity of hybridization (duplex stabilization) when the LNA is incorporated into a nucleic acid or a complementary RNA or DNA molecule. This can be routinely determined by measuring the melting temperature of the nucleic acid.

Non limiting, exemplary LNA nucleosides are disclosed in WO 99/014226, WO 00/66604, WO 98/039352, WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett., 12, 73-76, Seth et al., J. Org. Chem., 2010, Vol 75(5) pp. 1569-81, Mitsuoka et al., Nucleic Acids Research, 2009, 37(4), 1225-1238, and Wan and Seth, J. Medical Chemistry, 2016, 59, 9645-9667, which are incorporated by reference in their entirety.

Further non limiting, exemplary LNA nucleosides are disclosed in Scheme 1.

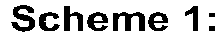

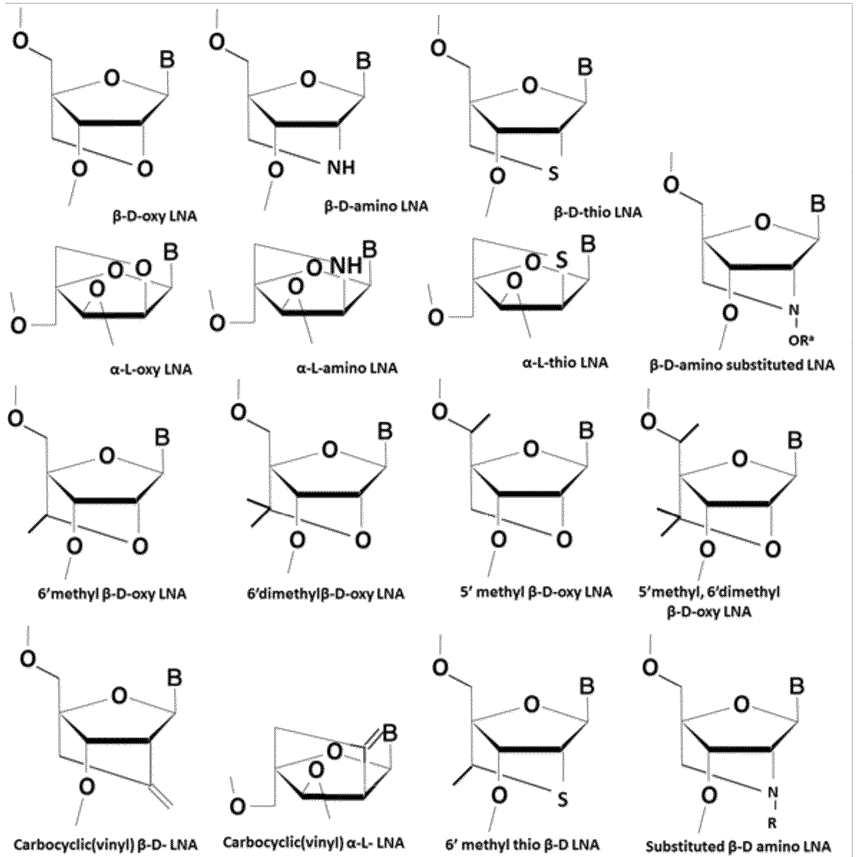

Particular LNA nucleosides are beta-D-oxy-LNA, 6'-methyl-beta-D-oxy LNA such as (S)-6'-methyl-beta-D-oxy-LNA (ScET) and ENA.

A particularly advantageous LNA is beta-D-oxy-LNA.

### Morpholino oligonucleotides

In some embodiments, the double stranded RNA molecule of the invention comprises or consists of morpholino nucleosides (i.e. is a morpholino oligomer or a phosphorodiamidate morpholino oligomer (PMO)). Splice modulating morpholino oligonucleotides have been approved for clinical use - see for example eteplirsen, a 30 nucleotide morpholino oligonucleotide targeting a frame shift mutation in DMD, used to treat Duchenne muscular dystrophy. Morpholino oligonucleotides have nucleobases attached to six membered morpholino rings rather than ribose, such as methylenemorpholine rings linked through phosphorodiamidate groups, for example as illustrated by the following illustration of 4 consecutive morpholino nucleotides:

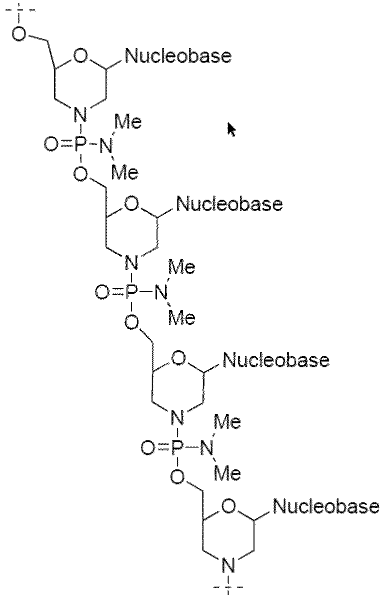

### Totalmer and mixmer

A "totalmer" is a nucleic acid which does not comprise DNA or RNA nucleosides, and may for example comprise only 2'-O-MOE nucleosides, such as a fully MOE phosphorothioate, e.g. MMMMMMMMMMMMMMMMMMMM, where M = 2'-O-MOE, or may for example comprise only 2'oMe nucleosides, which are reported to be effective for therapeutic use.

A "mixmer" is a nucleic acid which does not comprise DNA or RNA nucleosides, and may for example comprise a mixture of modified nucleosides, such as MLMLMLMLMLMLMLMLMLML, wherein L = LNA and M = 2'-O-MOE nucleosides. Advantageously, the internucleoside nucleosides in mixmers and totalmers may be phosphorothioate, or a majority of nucleoside linkages in mixmers may be phosphorothioate.

Mixmers and totalmers may comprise other internucleoside linkages, such as phosphodiester or phosphorodithioate, by way of example.

In some embodiments, the compound of the invention is or comprises an oligonucleotide mixmer or totalmer. In some embodiments, the contiguous nucleotide sequence is a mixmer or a totalmer.

### Complementarity

The term "complementarity" describes the capacity for base-pairing of nucleosides/nucleotides, such as Watson-Crick base pairing, non-Watson-Crick base pairing, or base pairing formed from non-natural and/or modified nucleotides.

Watson-Crick base pairs are guanine (G) - cytosine (C) and adenine (A) - thymine (T)/uracil (U). For example, in DNA, adenine (A) is complementary to thymine (T) and guanine (G) is complementary to cytosine (C). For example, in RNA, adenine (A) is complementary to uracil (U) and guanine (G) is complementary to cytosine (C). In some embodiments, complementary nucleotides can base pair in the Watson-Crick manner or in any other manner that allows for the formation of stable duplexes.

Non-Watson-Crick base pairs, such as non-canonical base pairs, are planar hydrogen bonded pairs of nucleobases, having hydrogen bonding patterns which differ from the patterns observed in Watson-Crick base pairs, as in the classic double helical DNA. Examples of non-canonical base pairs are e.g. trans A:G Hoogsteen/sugar edge, A:U Hoogsteen/WC, or G:U Wobble pairs.

Hoogsteen base pairs occur between adenine (A) and thymine (T); and guanine (G) and cytosine(C); similarly to Watson-Crick base pairs. However, the purine (A and G) takes on an alternative conformation with respect to the pyrimidine. In the A-U Hoogsteen base pair, the adenine is rotated 180° about the glycosidic bond, resulting in an alternative hydrogen bonding scheme which has one hydrogen bond in common with the Watson-Crick base pair (adenine N6 and thymine N4), while the other hydrogen bond, instead of occurring between adenine N1 and thymine N3 as in the Watson-Crick base pair, occurs between adenine N7 and thymine N3. In the G-C Hoogsteen base pair, like the A-T Hoogsteen base pair, the purine (guanine) is rotated 180° about the glycosidic bond while the pyrimidine (cytosine) remains in place. One hydrogen bond from the Watson-Crick base pair is maintained (guanine 06 and cytosine N4) and the other occurs between guanine N7 and a protonated cytosine N3 (the Hoogsteen G-C base pair has two hydrogen bonds, while the Watson-Crick G-C base pair has three).

Wobble base pairing occur between two nucleotides that are not Watson-Crick base pairs, such as guanine-uracil (G-U), hypoxanthine-uracil (I-U), hypoxanthine-adenine (I-A), and hypoxanthine-cytosine (I-C).

It will be understood that oligonucleotides may comprise nucleosides with modified nucleobases, for example 5-methyl cytosine is often used in place of cytosine, and as such the term complementarity encompasses Watson Crick, non-Watson Crick, or any other type of base-paring between non-modified and modified nucleobases (see for example Hirao et al., 2012, Accounts of Chemical Research, 45, 2055 and Bergstrom, 2009, Curr. Protoc. Nucleic Acid Chem., 37, 1.4.1, which are incorporated by reference in their entirety).

The term "complementary" (such as in the phrase "the second contiguous nucleotide sequence is complementary to a target nucleic acid sequence") does not require 100% complementarity. Rather, within the present invention, the term "complementary" requires a given sequence to be at least about 70% complementary to another sequence, such as at least about 75% complementary, at least about 80% complementary, at least about 85% complementary, at least about 90% complementary, at least about 95% complementary, or at least about 99% complementary.

The term "% complementary" as used herein, refers to the proportion of nucleotides (in percent) within a double-stranded region of complementarity which across the length of the double-stranded region of complementarity, are complementary.

Herein the first contiguous nucleotide sequence and the second contiguous nucleotide sequence may be at least about 70% complementary, at least about 80% complementary, at least about 90% complementary, at least about 95% complementary, or at least about 99% complementary. Therefore, the double-stranded region of complementarity may comprise one, two or three mismatches.

The first contiguous nucleotide sequence and the second contiguous nucleotide sequence may be fully complementary, i.e. 100% complementary.

The term "% complementary" is also used herein to refer to the proportion of nucleotides (in percent) within a query sequence (such as a second contiguous nucleotide sequence or an antisense strand as described herein) which are complementary to a reference sequence (such as a first contiguous nucleotide sequence, a sense strand, a target nucleic acid sequence or a target sequence as described herein).

Herein the second contiguous nucleotide sequence may be at least about 70% complementary, at least about 80% complementary, at least about 90% complementary, at least about 95% complementary, or at least about 99% complementary to the target sequence. Therefore, the second contiguous nucleotide sequence and the target sequence may comprise one, two or three mismatches.

The second contiguous nucleotide sequence and the target sequence may be fully complementary, i.e. 100% complementary.

To calculate the percentage of complementarity, the query sequence and reference sequence are first aligned with the query sequence running 5'-3' and the reference sequence 3'-5'. The sequences are aligned to maximise the number of complementary base pairs (e.g. Watson Crick base pairs, non-Watson-Crick base pairs, or any other type of base pairs) between the two sequences. The percentage complementarity is calculated by counting the number of aligned nucleobases that are complementary (e.g. form Watson Crick base pairs, non-Watson-Crick base pairs, or any other type of base pairs) between the two aligned sequences, dividing that number by the total number of nucleotides in the portion of the query sequence aligned with the reference sequence (i.e. any nucleotides at the 5' and 3' ends of the query sequence that are not complementary to the reference sequence, and any nucleotides at the 5' and 3' ends of the reference sequence that are not complementary to the query sequence, do not count towards the length of the sequence) and multiplying by 100. The resulting number is the percentage complementarity of the query sequence to the reference sequence.

In such a comparison a nucleobase/nucleotide which does not align (form a base pair) is termed a mismatch. Insertions and deletions are not allowed in the calculation of % complementarity of a contiguous nucleotide sequence.

It will be understood that in determining complementarity, chemical modifications of the nucleobases are disregarded as long as the functional capacity of the nucleobase to form base pairing (e.g. Watson-Crick base pairing, non-Watson-Crick base pairing, or any other type of base pairing) is retained (e.g. 5'-methyl cytosine is considered identical to a cytosine for the purpose of calculating % complementarity).

Thus, the expression "wherein the first contiguous nucleotide sequence and the second contiguous nucleotide sequence within the double stranded region of complementarity are at least 80% complementary" will be understood to mean that when the first contiguous nucleotide sequence and second contiguous nucleotide sequence are aligned to maximise complementarity, and any non-complementary (i.e. overhanging nucleotides) at the 5' and 3' ends of each contiguous nucleotide sequence are disregarded, the percentage of nucleotides in either sequence which form complementary base pairs with the other sequence is at least 80%.

A corresponding meaning will be understood to apply to all other percentage complementarity values between the first contiguous nucleotide sequence and the second contiguous nucleotide sequence within the double stranded region of complementarity described herein, such as 81%, 82%, 83%, 84%, 85%, 86%, 87%, 88%, 89%, 90%, 91%, 92%, 93%, 94%, 95%, 96%, 97%, 98%, 99% and 100%.

Thus, the expression "wherein the first contiguous nucleotide sequence and the second contiguous nucleotide sequence within the double stranded region of complementarity are at least 90% complementary" will be understood to mean that when the first contiguous nucleotide sequence and second contiguous nucleotide sequence are aligned to maximise complementarity, and any non-complementary (i.e. overhanging nucleotides) at the 5' and 3' ends of each contiguous nucleotide sequence are disregarded, the percentage of nucleotides in either sequence which form complementary base pairs with the other sequence is at least 90%.

The expression "wherein the first contiguous nucleotide sequence and the second contiguous nucleotide sequence within the double stranded region of complementarity are at least 95% complementary" will be understood to mean that when the first contiguous nucleotide sequence and second contiguous nucleotide sequence are aligned to maximise complementarity, and any non-complementary (i.e. overhanging nucleotides) at the 5' and 3' ends of each contiguous nucleotide sequence are disregarded, the percentage of nucleotides in either sequence which form complementary base pairs with the other sequence is at least 95%.

The expression "wherein the first contiguous nucleotide sequence and the second contiguous nucleotide sequence within the double stranded region of complementarity are fully complementary" will be understood to mean that when the first contiguous nucleotide sequence and second contiguous nucleotide sequence are aligned to maximise complementarity, and any non-complementary (i.e. overhanging nucleotides) at the 5' and 3' ends of each contiguous nucleotide sequence are disregarded, all nucleotides in each sequence form complementary base pairs with the other sequence (i.e. 100% complementarity).

Similarly, the expression "the second contiguous nucleotide sequence is fully complementary to a target sequence" will be understood to mean that when the second contiguous nucleotide sequence and target sequence are aligned to maximise complementarity, and any non-complementary (i.e. overhanging nucleotides) at the 5' and 3' ends of each sequence are disregarded, all nucleotides in each sequence form complementary base pairs with the other sequence (i.e. 100% complementarity).

Likewise, the expression "the antisense strand is fully complementary to a target sequence" will be understood to mean that when the antisense strand sequence and target sequence are aligned to maximise complementarity, and any non-complementary (i.e. overhanging nucleotides) at the 5' and 3' ends of each sequence are disregarded, all nucleotides in each sequence form complementary base pairs with the other sequence (i.e. 100% complementarity).

### Identity

The term "identity" as used herein, refers to the proportion of nucleotides (expressed in percent) of a contiguous nucleotide sequence in a nucleic acid molecule which at a given position, are identical to (i.e. in their ability to form Watson Crick / non-Watson Crick base pairs, or any other type of base pairs, with the complementary nucleoside) a contiguous nucleotide sequence, at a given position of a separate nucleic acid molecule.

The percentage identity is thus calculated by counting the number of aligned nucleobases that are identical (a Match) between two sequences, dividing that number by the total number of nucleotides in the oligonucleotide and multiplying by 100. Therefore, percentage identity = (matches x 100)/length of aligned region (e.g. the contiguous nucleotide sequence). Preferably, insertions and deletions are not allowed in the calculation of the percentage identity of a contiguous nucleotide sequence. It will be understood that in determining identity, chemical modifications of the nucleobases are disregarded as long as the functional capacity of the nucleobase to form Watson Crick / non-Watson Crick base pairing, or any other type of base pairing, is retained (e.g. 5-methyl cytosine is considered identical to a cytosine for the purpose of calculating % identity).

### Hybridization

The terms "hybridizing" or "hybridizes" or "anneal" as used herein are to be understood as two nucleic acid strands (e.g. a sense strand and an antisense strand) forming hydrogen bonds between base pairs on opposite strands thereby forming a duplex.

It is also to be understood as two nucleic acid sequences of a same strand (e.g. "C box" and "D box" sequences) forming hydrogen bonds between base pairs on opposite sequences (e.g. "C box" and opposite "D box" of a same nucleic acid strand) thereby forming a double-stranded region, and thereby forming a secondary structure. The affinity of the binding between two nucleic acid strands is the strength of the hybridization. It is often described in terms of the melting temperature (Tₘ) defined as the temperature at which half of the oligonucleotides are duplexed with the target nucleic acid. At physiological conditions Tₘ is not strictly proportional to the affinity (Mergny and Lacroix, 2003, Oligonucleotides 13:515-537, which is hereby incorporated by reference in its entirety).

The standard state Gibbs free energy ΔG° is a more accurate representation of binding affinity and is related to the dissociation constant (K_{d}) of the reaction by ΔG°=-RTIn(K_{d}), where R is the gas constant and T is the absolute temperature. Therefore, a very low ΔG° of the reaction between an oligonucleotide and the target nucleic acid reflects a strong hybridization between the sense strand and the antisense strand. ΔG° is the energy associated with a reaction where aqueous concentrations are 1M, the pH is 7, and the temperature is 37°C. The hybridization of nucleic acid strands is a spontaneous reaction and for spontaneous reactions ΔG° is less than zero. ΔG° can be measured experimentally, for example, by use of the isothermal titration calorimetry (ITC) method as described in Hansen et al., 1965, Chem. Comm. 36-38 and Holdgate et al., 2005, Drug Discov Today*,* which are incorporated by reference in their entirety. The skilled person will know that commercial equipment is available for ΔG° measurements. ΔG° can also be estimated numerically by using the nearest neighbour model as described by SantaLucia, 1998, Proc Natl Acad Sci USA. 95: 1460-1465 (incorporated by reference in its entirety) using appropriately derived thermodynamic parameters described by Sugimoto et al., 1995, Biochemistry 34:11211-11216 and McTigue et al., 2004, Biochemistry 43:5388-5405 (incorporated by reference in their entirety).

In some embodiments the degree or strength of hybridization is measured by the standard state Gibbs free energy ΔG°. The nucleic acid strands may hybridize with estimated ΔG° values below the range of -10 kcal, such as below -15 kcal, such as below -20 kcal and such as below -25 kcal. In some embodiments the nucleic acid strands may hybridize with an estimated ΔG° value of -10 to -60 kcal, such as -12 to -40, such as from -15 to -30 kcal, or-16 to -27 kcal such as -18 to -25 kcal.

### Modulation of expression

The term "modulation of expression" as used herein is to be understood as an overall term for a nucleic acid molecules ability to alter the amount of a target when compared to the amount of the target before administration of the nucleic acid molecule. Alternatively, modulation of expression may be determined by reference to a control experiment. It is generally understood that the control is an individual or target cell treated with a saline composition or an individual or target cell treated with a non-targeting or nucleic acid molecule (mock). It may however also be an individual treated with the standard of care.

### Increasing the expression

"Increasing the expression" of a target nucleic acid is one type of modulating the expression of that target nucleic acid.

The compounds of the invention have the ability to stimulate, up-regulate, increase, recover, promote, rise, or strengthen expression of a target nucleic acid, e.g. by directly or indirectly increasing the stability of the target mRNA and/or reducing deadenylation of the target mRNA.

### Antisense activity

"Antisense activity" means any detectable or measurable activity attributable to the hybridization of a compound of the invention to its target nucleic acid, i.e. by hydridization of an antisense strand comprising a first contiguous nucleotide sequence to a target nucleic acid.

Antisense activity can be a decrease or an increase in the amount or expression of a target nucleic acid or protein encoded by such target nucleic acid.

### Salts

The term "salts" as used herein conforms to its generally known meaning, i.e. an ionic assembly of anions and cations.

For example, the salt may comprise a metal cation, such as a sodium salt or a potassium salt.

### Composition

The compound of the invention may be in the form of a "composition".

A nucleic acid molecule composition has less than 20% impurities, preferably less than 15% or 10% impurities, more preferably less than 9%, 8%, 7% or 6% impurities, most preferably less than 5 % impurities. The impurities are typically nucleic acid molecules which are one or two nucleotides shorter (n-1 or n-2) than the primary nucleic acid molecule component.

### Pharmaceutical compositions

A pharmaceutical composition is a composition according to the invention.

A pharmaceutical composition comprises the nucleic acid molecule of the invention, and a pharmaceutically acceptable diluent, solvent, carrier, salt and/or adjuvant.

The solution may be a phosphate buffered saline solution, such as a sterile phosphate buffered saline solution.

### Pharmaceutically acceptable salt

As used herein the term "pharmaceutically acceptable salt" is any salt of a compound of the invention which is generally considered to be safe and non-toxic to humans and/or animals.

Examples of pharmaceutically acceptable salts include sodium salts and potassium salts.

### Treatment

The terms "treatment", "treating", "treats" and the like are used herein generally mean obtaining a desired pharmacological and/or physiological effect. This effect is therapeutic in terms of partially or completely curing a disease and/or adverse effect attributed to the disease.

The term "treatment" as used herein covers any treatment of a disease in a subject and includes: (a) inhibiting the disease, i.e. arresting its development; (b) ameliorating (i.e. relieving) the disease, i.e. causing regression of the disease; and (c) preventing the disease, i.e. stopping the disease starting or progressing. Thus, a compound that ameliorates and/or inhibits an infection is a compound that treats an infection.

Preferably, the term "treatment" as used herein relates to medical intervention of an already manifested disorder, like the treatment of an already defined and manifested infection.

### Amelioration

"Amelioration" refers to a lessening, slowing, stopping, or reversing of at least one indicator of the severity of a syndrome or condition. The severity of indicators may be determined by subjective or objective measures, which are known to those skilled in the art.

### Prevention

Herein the term "preventing", "prevention" or "prevents" relates to a prophylactic treatment, i.e. to a measure or procedure the purpose of which is to prevent, rather than to cure a disease. Prevention means that a desired pharmacological and/or physiological effect is obtained that is prophylactic in terms of completely or partially preventing a disease or symptom thereof.

### Patient

For the purposes of the present invention the "patient" (or "subject") may be a vertebrate. In context of the present invention, the term "subject" includes both humans and other animals, particularly mammals, and other organisms. Thus, the herein provided means and methods are applicable to both human therapy and veterinary applications. Accordingly, herein the subject may be an animal such as a mouse, rat, hamster, rabbit, guinea pig, ferret, cat, dog, chicken, sheep, bovine species, horse, camel, or primate. Preferably, the subject is a mammal. More preferably the subject is human.

### Administering

"Administering" or "administration" means providing a pharmaceutical agent (e.g. a double stranded RNA or a composition of the invention) to a subject in a manner that is pharmacologically useful (e.g. to treat a condition in a subject) and includes, but is not limited to, administering by a medical professional and self-administering.

Methods of administration may include parenteral, intravenous, intrathecal, intramuscular, subcutaneous, oral, rectal, vaginal or inhaled. It is understood that the skilled person will be able to determine an appropriate method of administration.

The administration can be a "concomitant administration" in which two pharmaceutical agents are administered to a subject at the same time (i.e. "administered concomitantly"). Concomitant administration does not require that both pharmaceutical agents are administered in a single pharmaceutical composition, in the same dosage form, or by the same route of administration. Concomitant administration can be either simultaneous, sequential or separate administration of the two pharmaceutical agents. The effects of both pharmaceutical agents do not need to manifest themselves at the same time or location. Preferably, the effects only need to overlap for a period of time.

### Antibody

The term "antibody" refers to a molecule characterized by reacting specifically with an antigen in some way, where the antibody and the antigen are each defined in terms of the other.

Herein the term "antibody" may refer to a complete antibody molecule or any fragment or region thereof, such as the heavy chain, the light chain, Fab region, and Fc region.

### DETAILED DESCRIPTION OF THE INVENTION

### SmartRNA

The present disclosure is directed to small artificial RNA oligonucleotides (smartRNAs) based on sequence motifs from endogenous small nucleolar RNAs (snoRNAs) including two antisense elements, of which one targets a target sequence located between the 3' end of the 3'UTR and the 5'end of the 5'UTR of a target mRNA, and the other one targets a sequence located within the polyA-tail of the same target mRNA.

SnoRNAs are non-protein coding RNAs that function in guiding methylation and pseudouridylation of a target RNA. To carry out said modifications of the target RNA, each snoRNA associates with at least four protein molecules, thereby forming an RNA/protein complex referred to as a small nucleolar ribonucleoprotein (snoRNP). The proteins associated with each snoRNA depend on the type of snoRNA. The snoRNA molecule contains antisense elements which are complementary to the sequence surrounding the nucleotide targeted for modification in the RNA molecule. This enables the snoRNP to recognise and bind to the target RNA. Once the snoRNP has bound to the target site, the associated proteins are recruited to catalyse a chemical modification of the target base. Two different types of RNA modification (methylation and pseudouridylation) are directed by two different families of snoRNAs. These families of snoRNAs are referred to as antisense C/D box and H/ACA box snoRNAs based on the presence of conserved sequence motifs in the snoRNA. The smartRNA of the present disclosure is based preferably on C/D box snoRNA sequence motifs.

The inventors of the present disclosure have found that it is possible to design a modified snoRNA-like oligonucleotide (called smartRNA) able to assemble an endogenous RNA binding protein (RNP) complex resulting in a smartRNP, wherein a first antisense element of the smartRNA confers specificity to a target mRNA located between the 3' end of the 3'UTR and the 5'end of the 5'UTR of a target mRNA, and a second antisense elements promotes the introduction of a chemical modification in the polyA-tail of said target mRNA via the smartRNP's enzymatic function. Said smartRNA may be single stranded, or double stranded.

Thus, in a first aspect, the present disclosure is directed to a compound comprising a small artificial RNA (smartRNA) oligonucleotide comprising or consisting of a partially double stranded ribonucleic acid (dsRNA), the partially dsRNA comprising or consisting of a sense strand and an antisense strand,
wherein the sense strand comprises or consists of a first contiguous nucleotide sequence comprising or consisting of, from 5' to 3':
   - a "stem I" nucleotide sequence comprising a "stem I" region of complementarity;
   - a "C box" nucleotide sequence comprising a "C box" region of complementarity;
   - a "D' guide" nucleotide sequence comprising a "D' guide" region of complementarity;,
   - a "D' box" nucleotide sequence comprising a "D' box" region of complementarity;
   - a "stem II" nucleotide sequence comprising a "stem II" region of complementarity;
wherein the antisense strand comprises or consists of a second contiguous nucleotide sequence comprising or consisting of, from 5' to 3':
   - a "stem II" nucleotide sequence comprising a "stem II" region of complementarity;
   - a "C' box" nucleotide sequence comprising a "C' box" region of complementarity;
   - a "D guide" nucleotide sequence comprising a "D guide" region of complementarity;
   - a "D box" nucleotide sequence comprising a "D box" region of complementarity;
   - a "stem I" nucleotide sequence comprising a "stem I" region of complementarity;
wherein the sense strand "stem I" and the antisense strand "stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the sense strand "stem II" and the antisense strand "stem II" regions of complementarity are at least partially complementary to each other and thereby can anneal at least partially to each other to form an at least partially double-stranded "stem II" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

In a second aspect, the present disclosure is directed to a compound comprising a small artificial RNA (smartRNA) oligonucleotide comprising or consisting of a partially double stranded ribonucleic acid (dsRNA), the partially dsRNA comprising or consisting of a sense strand and an antisense strand,
wherein the sense strand comprises or consists of a first contiguous nucleotide sequence comprising or consisting of, from 5' to 3':
   - a "stem I" nucleotide sequence comprising a "stem I" region of complementarity;
   - a "C box" nucleotide sequence comprising a "C box" region of complementarity;
   - a "D' guide" nucleotide sequence comprising a "D' guide" region of complementarity;
   - a "D' box" nucleotide sequence comprising a "D' box" region of complementarity;
wherein the antisense strand comprises or consists of a second contiguous nucleotide sequence comprising or consisting of, from 5' to 3':
   - a "C' box" nucleotide sequence comprising a "C' box" region of complementarity
   - a "D guide" nucleotide sequence comprising a "D guide" region of complementarity;
   - a "D box" nucleotide sequence comprising a "D box" region of complementarity;
   - a "stem I" nucleotide sequence comprising a "stem I" region of complementarity;
wherein the sense strand "stem I" and the antisense strand "stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

In a third aspect, the present disclosure is directed to a compound comprising a small artificial RNA (smartRNA) oligonucleotide comprising or consisting of, from 5' to 3':
- a "5' stem I" nucleotide sequence comprising a "5' stem I" region of complementarity;
- a "C box" nucleotide sequence comprising a "C box" region of complementarity;
- a "D' guide" nucleotide sequence comprising a "D' guide" region of complementarity;
- a "D' box" nucleotide sequence comprising a "D' box" region of complementarity;
- a "5' stem II" nucleotide sequence comprising a "5' stem II" region of complementarity;
- optionally a "loop" nucleotide sequence;
- a "3' stem II" nucleotide sequence comprising a "3' stem II" region of complementarity;
- a "C' box" nucleotide sequence comprising a "C' box" region of complementarity;
- a "D guide" nucleotide sequence comprising a "D guide" region of complementarity;
- a "D box" nucleotide sequence comprising a "D box" region of complementarity;
- a "3' stem I" nucleotide sequence comprising a "3' stem I" region of complementarity;

wherein the "5' stem I" and the "3' stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the "5' stem II" and the 3' stem II" regions of complementarity are at least partially complementary to each other and thereby can anneal at least partially to each other to form an at least partially double-stranded "stem II" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

In a fourth aspect, the present disclosure is directed to a compound comprising a small artificial RNA (smartRNA) oligonucleotide comprising or consisting of, from 5' to 3':
- a "5' stem I" nucleotide sequence comprising a "5' stem I" region of complementarity;
- a "C box" nucleotide sequence comprising a "C box" region of complementarity;
- a "D' guide" nucleotide sequence comprising a "D' guide" region of complementarity;
- a "D' box" nucleotide sequence comprising a "D' box" region of complementarity
- optionally a "loop" nucleotide sequence;
- a "C' box" nucleotide sequence comprising a "C' box" region of complementarity
- a "D guide" nucleotide sequence comprising a "D guide" region of complementarity;
- a "D box" nucleotide sequence comprising a "D box" region of complementarity;
- a "3' stem I" nucleotide sequence comprising a "3' stem I" region of complementarity;

wherein the "5' stem I" and the "3' stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA

In some embodiments, the sense strand "stem I", the antisense strand "stem I", the "5' stem I" and/or the "3' stem I" nucleotide sequences and/or regions of complementarity are independently between 1 and 30 nucleotides in length, such as between 1 and 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 2 and 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 3 and 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 4 and 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 5 and 6, 7, 8, 9, 10, 11, 12, 13, 14, 15, 16, 17, 18, 19, 20, 21, 22, 23, 24, 25, 26, 27, 28, 29, or 30 nucleotides in length, such as between 6 and 7, 8, 9, 10, 11, 12, 13, 14, 15, 16, 17, 18, 19, 20, 21, 22, 23, 24, 25, 26, 27, 28, 29, or 30 nucleotides in length, such as between 7 and 8, 9, 10, 11, 12, 13, 14, 15, 16, 17, 18, 19, 20, 21, 22, 23, 24, 25, 26, 27, 28, 29, or 30 nucleotides in length, such as between 8 and 9, 10, 11, 12, 13, 14, 15, 16, 17, 18, 19, 20, 21, 22, 23, 24, 25, 26, 27, 28, 29, or 30 nucleotides in length, such as between 9 and 10, 11, 12, 13, 14, 15, 16, 17, 18, 19, 20, 21, 22, 23, 24, 25, 26, 27, 28, 29, or 30 nucleotides in length, such as between 10 and 11, 12, 13, 14, 15, 16, 17, 18, 19, 20, 21, 22, 23, 24, 25, 26, 27, 28, 29, or 30 nucleotides in length, such as between 11 and 12, 13, 14, 15, 16, 17, 18, 19, 20, 21, 22, 23, 24, 25, 26, 27, 28, 29, or 30 nucleotides in length, such as between 12 and 13, 14, 15, 16, 17, 18, 19, 20, 21, 22, 23, 24, 25, 26, 27, 28, 29, or 30 nucleotides in length, such as between 13 and 14, 15, 16, 17, 18, 19, 20, 21, 22, 23, 24, 25, 26, 27, 28, 29, or 30 nucleotides in length, such as between 14 and 15, 16, 17, 18, 19, 20, 21, 22, 23, 24, 25, 26, 27, 28, 29, or 30 nucleotides in length, such as between 15 and 16, 17, 18, 19, 20, 21, 22, 23, 24, 25, 26, 27, 28, 29, or 30 nucleotides in length, such as between 16 and 17, 18, 19, 20, 21, 22, 23, 24, 25, 26, 27, 28, 29, or 30 nucleotides in length, such as between 17 and 18, 19, 20, 21, 22, 23, 24, 25, 26, 27, 28, 29, or 30 nucleotides in length, such as between 18 and 19, 20, 21, 22, 23, 24, 25, 26, 27, 28, 29, or 30 nucleotides in length, such as between 19 and 20, 21, 22, 23, 24, 25, 26, 27, 28, 29, or 30 nucleotides in length, such as between 20 and 21, 22, 23, 24, 25, 26, 27, 28, 29, or 30 nucleotides in length, such as between 21 and 22, 23, 24, 25, 26, 27, 28, 29, or 30 nucleotides in length, such as between 22 and 23, 24, 25, 26, 27, 28, 29, or 30 nucleotides in length, such as between 23 and 24, 25, 26, 27, 28, 29, or 30 nucleotides in length, such as between 24 and 25, 26, 27, 28, 29, or 30 nucleotides in length, such as between 25 and 26, 27, 28, 29, or 30 nucleotides in length, such as between 26 and 27, 28, 29, or 30 nucleotides in length, such as between 27 and 28, 29, or 30 nucleotides in length, such as between 28 and 29, or 30 nucleotides in length, such as between 29 and 30 nucleotides in length, preferably independently between 1 and 12, such as preferably independently between 6 and 12 nucleotides in length, more preferably between 7 and 10 nucleotides in length.

In some embodiments, the sense strand "stem I", the antisense strand "stem I", the "5' stem I" and/or the "3' stem I" nucleotide sequences and/or regions of complementarity are independently such as 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, preferably independently 6, 7, 8, 9, 10, 11, or 12 nucleotides in length, more preferably between 7, 8, 9, or 10 nucleotides in length.

In some embodiments, the "C box" and "D box" nucleotide sequences and/or regions of complementarity are independently between 1 and 30 nucleotides in length, such as between 1 and 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 2 and 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 3 and 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 4 and 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 5 and 6, 7, 8, 9, 10, 11, 12, 13, 14, 15, 16, 17, 18, 19, 20, 21, 22, 23, 24, 25, 26, 27, 28, 29, or 30 nucleotides in length, such as between 6 and 7, 8, 9, 10, 11, 12, 13, 14, 15, 16, 17, 18, 19, 20, 21, 22, 23, 24, 25, 26, 27, 28, 29, or 30 nucleotides in length, such as between 7 and 8, 9, 10, 11, 12, 13, 14, 15, 16, 17, 18, 19, 20, 21, 22, 23, 24, 25, 26, 27, 28, 29, or 30 nucleotides in length, such as between 8 and 9, 10, 11, 12, 13, 14, 15, 16, 17, 18, 19, 20, 21, 22, 23, 24, 25, 26, 27, 28, 29, or 30 nucleotides in length, such as between 9 and 10, 11, 12, 13, 14, 15, 16, 17, 18, 19, 20, 21, 22, 23, 24, 25, 26, 27, 28, 29, or 30 nucleotides in length, such as between 10 and 11, 12, 13, 14, 15, 16, 17, 18, 19, 20, 21, 22, 23, 24, 25, 26, 27, 28, 29, or 30 nucleotides in length, such as between 11 and 12, 13, 14, 15, 16, 17, 18, 19, 20, 21, 22, 23, 24, 25, 26, 27, 28, 29, or 30 nucleotides in length, such as between 12 and 13, 14, 15, 16, 17, 18, 19, 20, 21, 22, 23, 24, 25, 26, 27, 28, 29, or 30 nucleotides in length, such as between 13 and 14, 15, 16, 17, 18, 19, 20, 21, 22, 23, 24, 25, 26, 27, 28, 29, or 30 nucleotides in length, such as between 14 and 15, 16, 17, 18, 19, 20, 21, 22, 23, 24, 25, 26, 27, 28, 29, or 30 nucleotides in length, such as between 15 and 16, 17, 18, 19, 20, 21, 22, 23, 24, 25, 26, 27, 28, 29, or 30 nucleotides in length, such as between 16 and 17, 18, 19, 20, 21, 22, 23, 24, 25, 26, 27, 28, 29, or 30 nucleotides in length, such as between 17 and 18, 19, 20, 21, 22, 23, 24, 25, 26, 27, 28, 29, or 30 nucleotides in length, such as between 18 and 19, 20, 21, 22, 23, 24, 25, 26, 27, 28, 29, or 30 nucleotides in length, such as between 19 and 20, 21, 22, 23, 24, 25, 26, 27, 28, 29, or 30 nucleotides in length, such as between 20 and 21, 22, 23, 24, 25, 26, 27, 28, 29, or 30 nucleotides in length, such as between 21 and 22, 23, 24, 25, 26, 27, 28, 29, or 30 nucleotides in length, such as between 22 and 23, 24, 25, 26, 27, 28, 29, or 30 nucleotides in length, such as between 23 and 24, 25, 26, 27, 28, 29, or 30 nucleotides in length, such as between 24 and 25, 26, 27, 28, 29, or 30 nucleotides in length, such as between 25 and 26, 27, 28, 29, or 30 nucleotides in length, such as between 26 and 27, 28, 29, or 30 nucleotides in length, such as between 27 and 28, 29, or 30 nucleotides in length, such as between 28 and 29, or 30 nucleotides in length, such as between 29 and 30 nucleotides in length, preferably between 4 and 8 nucleotides in length.

In some embodiments, the "C box" and "D box" nucleotide sequences and/or regions of complementarity are independently such as 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, preferably independently 4, 5, 6, 7, or 8 nucleotides in length.

In some embodiments, the "D' box" and/or "C' box" regions of complementarity are independently between 1 and 30 nucleotides in length, such as between 1 and 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 2 and 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 3 and 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 4 and 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 5 and 6, 7, 8, 9, 10, 11, 12, 13, 14, 15, 16, 17, 18, 19, 20, 21, 22, 23, 24, 25, 26, 27, 28, 29, or 30 nucleotides in length, such as between 6 and 7, 8, 9, 10, 11, 12, 13, 14, 15, 16, 17, 18, 19, 20, 21, 22, 23, 24, 25, 26, 27, 28, 29, or 30 nucleotides in length, such as between 7 and 8, 9, 10, 11, 12, 13, 14, 15, 16, 17, 18, 19, 20, 21, 22, 23, 24, 25, 26, 27, 28, 29, or 30 nucleotides in length, such as between 8 and 9, 10, 11, 12, 13, 14, 15, 16, 17, 18, 19, 20, 21, 22, 23, 24, 25, 26, 27, 28, 29, or 30 nucleotides in length, such as between 9 and 10, 11, 12, 13, 14, 15, 16, 17, 18, 19, 20, 21, 22, 23, 24, 25, 26, 27, 28, 29, or 30 nucleotides in length, such as between 10 and 11, 12, 13, 14, 15, 16, 17, 18, 19, 20, 21, 22, 23, 24, 25, 26, 27, 28, 29, or 30 nucleotides in length, such as between 11 and 12, 13, 14, 15, 16, 17, 18, 19, 20, 21, 22, 23, 24, 25, 26, 27, 28, 29, or 30 nucleotides in length, such as between 12 and 13, 14, 15, 16, 17, 18, 19, 20, 21, 22, 23, 24, 25, 26, 27, 28, 29, or 30 nucleotides in length, such as between 13 and 14, 15, 16, 17, 18, 19, 20, 21, 22, 23, 24, 25, 26, 27, 28, 29, or 30 nucleotides in length, such as between 14 and 15, 16, 17, 18, 19, 20, 21, 22, 23, 24, 25, 26, 27, 28, 29, or 30 nucleotides in length, such as between 15 and 16, 17, 18, 19, 20, 21, 22, 23, 24, 25, 26, 27, 28, 29, or 30 nucleotides in length, such as between 16 and 17, 18, 19, 20, 21, 22, 23, 24, 25, 26, 27, 28, 29, or 30 nucleotides in length, such as between 17 and 18, 19, 20, 21, 22, 23, 24, 25, 26, 27, 28, 29, or 30 nucleotides in length, such as between 18 and 19, 20, 21, 22, 23, 24, 25, 26, 27, 28, 29, or 30 nucleotides in length, such as between 19 and 20, 21, 22, 23, 24, 25, 26, 27, 28, 29, or 30 nucleotides in length, such as between 20 and 21, 22, 23, 24, 25, 26, 27, 28, 29, or 30 nucleotides in length, such as between 21 and 22, 23, 24, 25, 26, 27, 28, 29, or 30 nucleotides in length, such as between 22 and 23, 24, 25, 26, 27, 28, 29, or 30 nucleotides in length, such as between 23 and 24, 25, 26, 27, 28, 29, or 30 nucleotides in length, such as between 24 and 25, 26, 27, 28, 29, or 30 nucleotides in length, such as between 25 and 26, 27, 28, 29, or 30 nucleotides in length, such as between 26 and 27, 28, 29, or 30 nucleotides in length, such as between 27 and 28, 29, or 30 nucleotides in length, such as between 28 and 29, or 30 nucleotides in length, such as between 29 and 30 nucleotides in length, preferably between 4 and 8 nucleotides in length.

In some embodiments, the "D' box" and/or "C' box" regions of complementarity are independently such as 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, preferably independently 4, 5, 6, 7, or 8 nucleotides in length.

In some embodiments, the sense strand "stem II", the antisense strand "stem II", the "5' stem II" and/or the "3' stem II" nucleotide sequences and/or regions of complementarity are independently between 0 and 30 nucleotides in length, such as between 0 and 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 1 and 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 2 and 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 3 and 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 4 and 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 5 and 6, 7, 8, 9, 10, 11, 12, 13, 14, 15, 16, 17, 18, 19, 20, 21, 22, 23, 24, 25, 26, 27, 28, 29, or 30 nucleotides in length, such as between 6 and 7, 8, 9, 10, 11, 12, 13, 14, 15, 16, 17, 18, 19, 20, 21, 22, 23, 24, 25, 26, 27, 28, 29, or 30 nucleotides in length, such as between 7 and 8, 9, 10, 11, 12, 13, 14, 15, 16, 17, 18, 19, 20, 21, 22, 23, 24, 25, 26, 27, 28, 29, or 30 nucleotides in length, such as between 8 and 9, 10, 11, 12, 13, 14, 15, 16, 17, 18, 19, 20, 21, 22, 23, 24, 25, 26, 27, 28, 29, or 30 nucleotides in length, such as between 9 and 10, 11, 12, 13, 14, 15, 16, 17, 18, 19, 20, 21, 22, 23, 24, 25, 26, 27, 28, 29, or 30 nucleotides in length, such as between 10 and 11, 12, 13, 14, 15, 16, 17, 18, 19, 20, 21, 22, 23, 24, 25, 26, 27, 28, 29, or 30 nucleotides in length, such as between 11 and 12, 13, 14, 15, 16, 17, 18, 19, 20, 21, 22, 23, 24, 25, 26, 27, 28, 29, or 30 nucleotides in length, such as between 12 and 13, 14, 15, 16, 17, 18, 19, 20, 21, 22, 23, 24, 25, 26, 27, 28, 29, or 30 nucleotides in length, such as between 13 and 14, 15, 16, 17, 18, 19, 20, 21, 22, 23, 24, 25, 26, 27, 28, 29, or 30 nucleotides in length, such as between 14 and 15, 16, 17, 18, 19, 20, 21, 22, 23, 24, 25, 26, 27, 28, 29, or 30 nucleotides in length, such as between 15 and 16, 17, 18, 19, 20, 21, 22, 23, 24, 25, 26, 27, 28, 29, or 30 nucleotides in length, such as between 16 and 17, 18, 19, 20, 21, 22, 23, 24, 25, 26, 27, 28, 29, or 30 nucleotides in length, such as between 17 and 18, 19, 20, 21, 22, 23, 24, 25, 26, 27, 28, 29, or 30 nucleotides in length, such as between 18 and 19, 20, 21, 22, 23, 24, 25, 26, 27, 28, 29, or 30 nucleotides in length, such as between 19 and 20, 21, 22, 23, 24, 25, 26, 27, 28, 29, or 30 nucleotides in length, such as between 20 and 21, 22, 23, 24, 25, 26, 27, 28, 29, or 30 nucleotides in length, such as between 21 and 22, 23, 24, 25, 26, 27, 28, 29, or 30 nucleotides in length, such as between 22 and 23, 24, 25, 26, 27, 28, 29, or 30 nucleotides in length, such as between 23 and 24, 25, 26, 27, 28, 29, or 30 nucleotides in length, such as between 24 and 25, 26, 27, 28, 29, or 30 nucleotides in length, such as between 25 and 26, 27, 28, 29, or 30 nucleotides in length, such as between 26 and 27, 28, 29, or 30 nucleotides in length, such as between 27 and 28, 29, or 30 nucleotides in length, such as between 28 and 29, or 30 nucleotides in length, such as between 29 and 30 nucleotides in length, preferably between 0 (i.e. no stem II sequence and/or regions of complementarity) and 7 nucleotides in length.

In some embodiments, the sense strand "stem II", the antisense strand "stem II", the "5' stem II" and/or the "3' stem II" nucleotide sequences and/or regions of complementarity are independently such as 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, preferably independently 0 (i.e. no stem II nucleotide sequence and/or regions of complementarity), 1, 2, 3, 4, 5, 6, or 7 nucleotides in length.

In some embodiments, the "loop" nucleotide sequence is between 1 and 30 nucleotides in length, , such as between 1 and 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 2 and 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 3 and 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 4 and 5, 6, 7, 8, 9, 10, 11, 12, 13, 14, 15, 16, 17, 18, 19, 20, 21, 22, 23, 24, 25, 26, 27, 28, 29, or 30 nucleotides in length, such as between 5 and 6, 7, 8, 9, 10, 11, 12, 13, 14, 15, 16, 17, 18, 19, 20, 21, 22, 23, 24, 25, 26, 27, 28, 29, or 30 nucleotides in length, such as between 6 and 7, 8, 9, 10, 11, 12, 13, 14, 15, 16, 17, 18, 19, 20, 21, 22, 23, 24, 25, 26, 27, 28, 29, or 30 nucleotides in length, such as between 7 and 8, 9, 10, 11, 12, 13, 14, 15, 16, 17, 18, 19, 20, 21, 22, 23, 24, 25, 26, 27, 28, 29, or 30 nucleotides in length, such as between 8 and 9, 10, 11, 12, 13, 14, 15, 16, 17, 18, 19, 20, 21, 22, 23, 24, 25, 26, 27, 28, 29, or 30 nucleotides in length, such as between 9 and 10, 11, 12, 13, 14, 15, 16, 17, 18, 19, 20, 21, 22, 23, 24, 25, 26, 27, 28, 29, or 30 nucleotides in length, such as between 10 and 11, 12, 13, 14, 15, 16, 17, 18, 19, 20, 21, 22, 23, 24, 25, 26, 27, 28, 29, or 30 nucleotides in length, such as between 11 and 12, 13, 14, 15, 16, 17, 18, 19, 20, 21, 22, 23, 24, 25, 26, 27, 28, 29, or 30 nucleotides in length, such as between 12 and 13, 14, 15, 16, 17, 18, 19, 20, 21, 22, 23, 24, 25, 26, 27, 28, 29, or 30 nucleotides in length, such as between 13 and 14, 15, 16, 17, 18, 19, 20, 21, 22, 23, 24, 25, 26, 27, 28, 29, or 30 nucleotides in length, such as between 14 and 15, 16, 17, 18, 19, 20, 21, 22, 23, 24, 25, 26, 27, 28, 29, or 30 nucleotides in length, such as between 15 and 16, 17, 18, 19, 20, 21, 22, 23, 24, 25, 26, 27, 28, 29, or 30 nucleotides in length, such as between 16 and 17, 18, 19, 20, 21, 22, 23, 24, 25, 26, 27, 28, 29, or 30 nucleotides in length, such as between 17 and 18, 19, 20, 21, 22, 23, 24, 25, 26, 27, 28, 29, or 30 nucleotides in length, such as between 18 and 19, 20, 21, 22, 23, 24, 25, 26, 27, 28, 29, or 30 nucleotides in length, such as between 19 and 20, 21, 22, 23, 24, 25, 26, 27, 28, 29, or 30 nucleotides in length, such as between 20 and 21, 22, 23, 24, 25, 26, 27, 28, 29, or 30 nucleotides in length, such as between 21 and 22, 23, 24, 25, 26, 27, 28, 29, or 30 nucleotides in length, such as between 22 and 23, 24, 25, 26, 27, 28, 29, or 30 nucleotides in length, such as between 23 and 24, 25, 26, 27, 28, 29, or 30 nucleotides in length, such as between 24 and 25, 26, 27, 28, 29, or 30 nucleotides in length, such as between 25 and 26, 27, 28, 29, or 30 nucleotides in length, such as between 26 and 27, 28, 29, or 30 nucleotides in length, such as between 27 and 28, 29, or 30 nucleotides in length, such as between 28 and 29, or 30 nucleotides in length, such as between 29 and 30 nucleotides in length, preferably between 1 and 7 nucleotides in length.

In some embodiments, the "loop" nucleotide sequence is such as 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, preferably 1, 2, 3, 4, 5, 6, or 7 nucleotides in length.

When the compound of the invention is double-stranded (i.e. comprises or consists of a sense strand and an antisense strand), it is to be understood that it does not comprise a "loop" sequence.

In some embodiments, the sense strand "stem I", the antisense strand "stem I", the "5' stem I" and/or the "3' stem I" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other.

In some embodiments, the "C box" and the "D box" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other.

In some embodiments, the "D' box" and the "C' box" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other.

In some embodiments, the sense strand "stem II", the antisense strand "stem II", the "5' stem II" and/or the "3' stem II" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other.

In some embodiments, the sense strand "stem I", the antisense strand "stem I", the "5' stem I" and/or the "3' stem I" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other.

In some embodiments, the "C box" and the "D box" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other.

In some embodiments, the "D' box" and the "C' box" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other.

In some embodiments, the sense strand "stem II", the antisense strand "stem II", the "5' stem II" and/or the "3' stem II" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other.

In some embodiments, the first contiguous nucleotide sequence of the "D guide" region of complementarity and/or the second contiguous nucleotide sequence of the "D' guide" region of complementarity is/are independently of at least 7 nucleotides in length, preferably between 7 and 30 nucleotides in length (which can be represented in a sequence with the term "N₇₋₃₀"), such as between 7 and 8, 9, 10, 11, 12, 13, 14, 15, 16, 17, 18, 19, 20, 21, 22, 23, 24, 25, 26, 27, 28, 29, or 30 nucleotides in length, such as between 8 and 9, 10, 11, 12, 13, 14, 15, 16, 17, 18, 19, 20, 21, 22, 23, 24, 25, 26, 27, 28, 29, or 30 nucleotides in length, such as between 9 and 10, 11, 12, 13, 14, 15, 16, 17, 18, 19, 20, 21, 22, 23, 24, 25, 26, 27, 28, 29, or 30 nucleotides in length, such as between 10 and 11, 12, 13, 14, 15, 16, 17, 18, 19, 20, 21, 22, 23, 24, 25, 26, 27, 28, 29, or 30 nucleotides in length, such as between 11 and 12, 13, 14, 15, 16, 17, 18, 19, 20, 21, 22, 23, 24, 25, 26, 27, 28, 29, or 30 nucleotides in length, such as between 12 and 13, 14, 15, 16, 17, 18, 19, 20, 21, 22, 23, 24, 25, 26, 27, 28, 29, or 30 nucleotides in length, such as between 13 and 14, 15, 16, 17, 18, 19, 20, 21, 22, 23, 24, 25, 26, 27, 28, 29, or 30 nucleotides in length, such as between 14 and 15, 16, 17, 18, 19, 20, 21, 22, 23, 24, 25, 26, 27, 28, 29, or 30 nucleotides in length, such as between 15 and 16, 17, 18, 19, 20, 21, 22, 23, 24, 25, 26, 27, 28, 29, or 30 nucleotides in length, such as between 16 and 17, 18, 19, 20, 21, 22, 23, 24, 25, 26, 27, 28, 29, or 30 nucleotides in length, such as between 17 and 18, 19, 20, 21, 22, 23, 24, 25, 26, 27, 28, 29, or 30 nucleotides in length, such as between 18 and 19, 20, 21, 22, 23, 24, 25, 26, 27, 28, 29, or 30 nucleotides in length, such as between 19 and 20, 21, 22, 23, 24, 25, 26, 27, 28, 29, or 30 nucleotides in length, such as between 20 and 21, 22, 23, 24, 25, 26, 27, 28, 29, or 30 nucleotides in length, such as between 21 and 22, 23, 24, 25, 26, 27, 28, 29, or 30 nucleotides in length, such as between 22 and 23, 24, 25, 26, 27, 28, 29, or 30 nucleotides in length, such as between 23 and 24, 25, 26, 27, 28, 29, or 30 nucleotides in length, such as between 24 and 25, 26, 27, 28, 29, or 30 nucleotides in length, such as between 25 and 26, 27, 28, 29, or 30 nucleotides in length, such as between 26 and 27, 28, 29, or 30 nucleotides in length, such as between 27 and 28, 29, or 30 nucleotides in length, such as between 28 and 29, or 30 nucleotides in length, such as between 29 and 30 nucleotides in length.

Preferably, the first contiguous nucleotide sequence of the "D guide" region of complementarity or the second contiguous nucleotide sequence of the "D' guide" region of complementarity are of between 7 and 15 nucleotides in length, and can be represented in a sequence with the term "N₇₋₁₅" ; or preferably the first contiguous nucleotide sequence of the "D guide" region of complementarity or the second contiguous nucleotide sequence of the "D' guide" region of complementarity are of between 12 and 30 nucleotides in length, and can be represented in a sequence with the term "N₁₂₋₃₀"

In some embodiments, the first contiguous nucleotide sequence of the "D guide" region of complementarity and/or the second contiguous nucleotide sequence of the "D' guide" region of complementarity is/are independently such as 7, 8, 9, 10, 11, 12, 13, 14, 15, 16, 17, 18, 19, 20, 21, 22, 23, 24, 25, 26, 27, 28, 29, or 30 nucleotides in length.

In some embodiments, when one of the first contiguous nucleotide sequence of the "D guide" region of complementarity or the second contiguous nucleotide sequence of the "D' guide" region of complementarity is between 7 and 15 nucleotides (such as between 7 and 8, 9, 10, 11, 12, 13, 14, or 15 nucleotides in length, such as between 8 and 9, 10, 11, 12, 13, 14, or 15 nucleotides in length, such as between 9 and 10, 11, 12, 13, 14, or 15 nucleotides in length, such as between 10 and 11, 12, 13, 14, or 15 nucleotides in length, such as between 11 and 12, 13, 14, or 15 nucleotides in length, such as between 12 and 13, 14, or 15 nucleotides in length, such as between 13 and 14, or 15 nucleotides in length, such as between 14 and or 15 nucleotides in length), the other one is between 12 and 30 nucleotides (such as between 12 and 13, 14, 15, 16, 17, 18, 19, 20, 21, 22, 23, 24, 25, 26, 27, 28, 29, or 30 nucleotides in length, such as between 13 and 14, 15, 16, 17, 18, 19, 20, 21, 22, 23, 24, 25, 26, 27, 28, 29, or 30 nucleotides in length, such as between 14 and 15, 16, 17, 18, 19, 20, 21, 22, 23, 24, 25, 26, 27, 28, 29, or 30 nucleotides in length, such as between 15 and 16, 17, 18, 19, 20, 21, 22, 23, 24, 25, 26, 27, 28, 29, or 30 nucleotides in length, such as between 16 and 17, 18, 19, 20, 21, 22, 23, 24, 25, 26, 27, 28, 29, or 30 nucleotides in length, such as between 17 and 18, 19, 20, 21, 22, 23, 24, 25, 26, 27, 28, 29, or 30 nucleotides in length, such as between 18 and 19, 20, 21, 22, 23, 24, 25, 26, 27, 28, 29, or 30 nucleotides in length, such as between 19 and 20, 21, 22, 23, 24, 25, 26, 27, 28, 29, or 30 nucleotides in length, such as between 20 and 21, 22, 23, 24, 25, 26, 27, 28, 29, or 30 nucleotides in length, such as between 21 and 22, 23, 24, 25, 26, 27, 28, 29, or 30 nucleotides in length, such as between 22 and 23, 24, 25, 26, 27, 28, 29, or 30 nucleotides in length, such as between 23 and 24, 25, 26, 27, 28, 29, or 30 nucleotides in length, such as between 24 and 25, 26, 27, 28, 29, or 30 nucleotides in length, such as between 25 and 26, 27, 28, 29, or 30 nucleotides in length, such as between 26 and 27, 28, 29, or 30 nucleotides in length, such as between 27 and 28, 29, or 30 nucleotides in length, such as between 28 and 29, or 30 nucleotides in length, such as between 29 and 30 nucleotides in length).

In some embodiments, the first contiguous nucleotide sequence of the "D guide" region of complementarity and/or the second contiguous nucleotide sequence of the "D' guide" region of complementarity is/are at least 70% complementary, such as at least 70%, 75% 80%, 85%, 90%, 95%, or 100% complementary, preferably at least 90% complementary, more preferably at least 95% complementary, even more preferably fully complementary to a same-length portion of the first and/or second target nucleic acid sequence of the target mRNA, respectively.

In some embodiments, the first target nucleic acid sequence comprises the polyA-tail of said target mRNA; and the second target nucleic acid sequence of the target mRNA is selected from the group consisting of the 5'UTR, a sequence overlapping the 5'UTR and the open reading frame (ORF), the open reading frame (ORF), a sequence overlapping the ORF and the 3'UTR, the 3'UTR, and a sequence overlapping 3'UTR and 5'UTR of the mRNA (e.g. when the target mRNA is circularized); and *vice versa.*

In some embodiments, the first target nucleic acid sequence comprises the polyA-tail of said target mRNA, and the "D guide" comprises a homo-polymeric sequence, preferably a poly-uridine sequence, such as at least 7 contiguous uridines, preferably between 7 and 12 uridines, such as between 7 and 8, 9, 10, 11, or 12 nucleotides in length, such as between 8 and 9, 10, 11, or 12 nucleotides in length, such as between 9 and 10, 11, or 12 nucleotides in length, such as between 10 and 11, or 12 nucleotides in length, such as between 11 and or 12 nucleotides in length.

In some embodiments, the second target nucleic acid sequence comprises the polyA-tail of said target mRNA, and the "D' guide" comprises a homo-polymeric sequence, preferably a poly-uridine sequence, such as at least 7 contiguous uridines, preferably between 7 and 12 uridines, such as between 7 and 8, 9, 10, 11, or 12 nucleotides in length, such as between 8 and 9, 10, 11, or 12 nucleotides in length, such as between 9 and 10, 11, or 12 nucleotides in length, such as between 10 and 11, or 12 nucleotides in length, such as between 11 and or 12 nucleotides in length

Figures 1a and 1b provide an overview of an example of a smartRNA with structural C/D box sequence elements identical to yeast U14 and similar to human snoRD86. The self-complementarity of the pseudo-symmetric smartRNA-scaffold allows the molecule to fold into the correct smartRNP-forming structure. In the folded smartRNA, the base paring of the 5' stem I and 3' stem I forms the stem I. Similarly, the 5' stem II and 3' stem II forms the stem II, which might comprise a loop structure. The C/D-box and C'/D'-box sequences flanks the antisense sequences and are required for smartRNP formation. The 'first' and 'second' antisense (AS) sequences correspond to the D'-guide and D-guide, e.g., the first AS corresponds to D'-guide, and the second AS corresponds to the D-guide, or *vice versa.*

In some embodiments the C/D box and C'/D' box sequence elements of the smartRNA are fully identical to endogenous C/D snoRNA sequences.

In some embodiments the C/D box and C'/D' box sequence elements of the smartRNA are non-naturally occurring C/D box sequences.

In some embodiments, stem II can be shortened or even omitted in a functional smartRNA. Moreover, in some embodiments, stem I can also be shortened - especially after introduction of stabilizing RNA modifications.

In some embodiments, the "loop" can be shortened or even omitted in a functional smartRNA.

Thus, in some embodiments, the smartRNA comprises or consists of one or two polynucleotide chains.

In some embodiments, the "C box", "D box", "C' box" and/or "D' box" nucleotide sequence of the smart RNA is selected from the sequences of Table 1 below.

**Table 1: SmartRNA CID box and C'/D' box sequences**

| **C box sequences** | **D box sequences** | **C' box sequences** | **D' box sequences** |
|---|---|---|---|
| ACGATGA | CTGA | AACATGT | AAGC |
| AGGATGA | | AAGACAC | AGAA |
| ATGAAGA | | AAGAGCA | AGGA |
| ATGAGGA | | AAGATGC | AGGC |
| ATGATGA | | ACAATGA | ATAA |
| ATGATGC | | ACAGTGA | ATGA |
| ATGATGG | | ACCATCA | ATGC |
| ATGATGT | | ACCATGT | CAGA |
| CGGATGA | | ACGATGA | CAGC |
| CTGATGA | | ACGGTGA | CAGG |
| GCGATGA | | ACTATGA | CATA |
| GGGATGA | | AGAAGGA | CCAA |
| GTCATGA | | AGCAGGG | CCGA |
| GTGAAGA | | AGCATTT | CGGA |
| GTGATGA | | AGGACGA | CGTA |
| GTGATGC | | AGGATGC | CTAA |
| GTGATGG | | ATAATGA | CTCA |
| GTGATGT | | ATCAGAA | CTCC |
| TCGATGA | | ATGAAAA | CTGA |
| TGATGA | | ATGAAAG | CTGC |
| TTGATGA | | ATGAACA | CTGT |
| | | ATGAACT | CTTA |
| | | ATGAAGA | CTTC |
| | | ATGAAGG | CTTG |
| | | ATGAAGT | GAGA |
| | | ATGAATA | GGGA |
| | | ATGAATG | GTAA |
| | | ATGAGAA | GTGA |
| | | ATGAGAC | GTGC |
| | | ATGAGAT | GTGG |
| | | ATGAGGA | TAGA |
| | | ATGAGGG | TCGA |
| | | ATGAGTG | TCGC |
| | | ATGATAA | TGGA |
| | | ATGATAC | TGGC |
| | | ATGATAT | TTGA |
| | | ATGATCT | TTGG |
| | | ATGATGA | TTGT |
| | | ATGATGC | |
| | | ATGATGG | |
| | | ATGATGT | |
| | | ATGATTA | |
| | | ATGATTT | |
| | | ATGCTCA | |
| | | ATGCTGA | |
| | | ATGGAGA | |
| | | ATGGAGG | |
| | | ATGGCGA | |
| | | ATGTAGA | |
| | | ATGTGGA | |
| | | ATGTGGT | |
| | | ATGTTGA | |
| | | ATGTTGG | |
| | | ATTATGA | |
| | | CACATGA | |
| | | CAGATTG | |
| | | CAGCTGA | |
| | | CTCATGA | |
| | | CTCATTC | |
| | | CTCTGGA | |
| | | CTGAAAG | |
| | | CTGAAGA | |
| | | CTGAAGC | |
| | | CTGACGA | |
| | | CTGACTC | |
| | | CTGAGAA | |
| | | CTGAGGA | |
| | | CTGAGGG | |
| | | CTGATGA | |
| | | CTGATGC | |
| | | CTGATGG | |
| | | CTGATGT | |
| | | CTGATTA | |
| | | CTGATTT | |
| | | CTGCGGA | |
| | | CTGCTGA | |
| | | CTGCTGT | |
| | | CTGGACA | |
| | | CTGTAGG | |
| | | CTGTGGA | |
| | | CTGTGGC | |
| | | CTGTTGA | |
| | | CTGTTGA | |
| | | CTGTTGG | |
| | | GACATGA | |
| | | GAGATGA | |
| | | GCAAGGA | |
| | | GCCAGAA | |
| | | GCCATGA | |
| | | GCCCTGT | |
| | | GCGACAA | |
| | | GCGATGA | |
| | | GGGAAGC | |
| | | GGGATGT | |
| | | GGGCTGA | |
| | | GTAAAGT | |
| | | GTCATTG | |
| | | GTCCTGG | |
| | | GTGAAAA | |
| | | GTGAAGA | |
| | | GTGAATA | |
| | | GTGACAA | |
| | | GTGACCA | |
| | | GTGACTG | |
| | | GTGAGAA | |
| | | GTGAGCA | |
| | | GTGAGGA | |
| | | GTGATAA | |
| | | GTGATCT | |
| | | GTGATGA | |
| | | GTGATGC | |
| | | GTGATGG | |
| | | GTGATGT | |
| | | GTGATTA | |
| | | GTGATTG | |
| | | GTGATTT | |
| | | GTGCTGA | |
| | | GTGCTGG | |
| | | GTGGAGA | |
| | | GTGGGGA | |
| | | GTGGTGA | |
| | | GTGTAGA | |
| | | GTGTGGA | |
| | | GTGTTCA | |
| | | GTGTTGA | |
| | | GTGTTGG | |
| | | GTTACTA | |
| | | GTTATGA | |
| | | TATACGA | |
| | | TCCAAGG | |
| | | TGGAAAT | |
| | | TGGAATA | |
| | | TGGATGA | |
| | | TTCATGA | |
| | | TTGAAAA | |
| | | TTGAAGA | |
| | | TTGACGA | |
| | | TTGACGG | |
| | | TTGATGA | |
| | | TTGATGT | |
| | | TTGATTA | |
| | | TTGATTG | |
| | | TTGATTT | |
| | | TTGCCAA | |
| | | TTGCTGA | |
| | | TTGGTGA | |
| | | TTGTAGA | |
| | | TTTATGA | |
| | | TTTCTGA | |

In some embodiments, the binding of the first contiguous nucleotide sequence of the "D guide" region of complementarity and the second contiguous nucleotide sequence of the "D' guide" region of complementarity, to the first and second target sequences, respectively, increases the 2'-O-methylation level of one or more adenosines of the polyA-tail of the target mRNA.

In some embodiments, the 2'-O-methylation of one or more adenosines of the poly-A tail is increased by at least 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or at least 1000% compared to a control (e.g. the adenosine 2'O-methylation level of the poly-A tail measure in the absence of a compound from the invention).

In some embodiments, the smartRNA is between 30 and 150 nucleotides in length, such as between 30 and 40, 50, 60, 70, 80, 90, 100, 110, 120, 130, 140, or 150 nucleotides in length, such as between 40 and 50, 60, 70, 80, 90, 100, 110, 120, 130, 140, or 150 nucleotides in length, such as between 50 and 60, 70, 80, 90, 100, 110, 120, 130, 140, or 150 nucleotides in length, such as between 60 and 70, 80, 90, 100, 110, 120, 130, 140, or 150 nucleotides in length, such as between 70 and 80, 90, 100, 110, 120, 130, 140, or 150 nucleotides in length, such as between 80 and 90, 100, 110, 120, 130, 140, or 150 nucleotides in length, such as between 90 and 100, 110, 120, 130, 140, or 150 nucleotides in length, such as between 100 and 110, 120, 130, 140, or 150 nucleotides in length, such as between 110 and 120, 130, 140, or 150 nucleotides in length, such as between 120 and 130, 140, or 150 nucleotides in length, such as between 130 and 140, or 150 nucleotides in length, such as between 140 and 150 nucleotides in length, preferably between 60 and 80 nucleotides in length, such as between 60 and 70 nucleotides in length or between 70 and 80 nucleotides in length.

In some embodiments, the smartRNA comprises at least one modified nucleotide.

In some embodiments, the smartRNA comprises at least one modified nucleotide comprising a modified sugar moiety; preferably wherein the modified sugar moiety is independently selected from a bicyclic sugar moiety or a non-bicyclic sugar moiety.

In some embodiments, the smartRNA comprises at least one modified nucleotide comprising a non-bicyclic sugar moiety.

In some embodiments, the smartRNA comprises at least one modified nucleotide comprising a non-bicyclic sugar moiety, wherein the non-bicyclic sugar moiety is independently selected from the group consisting of 2'-O-alkyl-RNA, 2'-O-methyl-RNA (2'OMe modified sugar), 2'-alkoxy-RNA, 2'-O-methoxyethyl-RNA, 2'-amino-DNA, 2'-fluoro-DNA /RNA (2'F modified sugar), arabino nucleic acid (ANA), 2'-fluoro-ANA, Glycol nucleic acid (GNA), inverted RNA (3'-3' linked, or 5'-5' linked), 2'-azido-DNA, 2'-O-propargyl-RNA, L-ribose based nucleotides (mirror image nucleotides), and unlocked nucleic acid (UNA); preferably wherein the non-bicyclic sugar moiety is independently selected from a 2'F modified sugar and a 2'OMe modified sugar.

In some embodiments, the smartRNA comprises at least one modified nucleotide comprising a bicyclic sugar moiety.

In some embodiments, the smartRNA comprises at least one modified nucleotide comprising a bicyclic sugar moiety, wherein the bicyclic sugar moiety is independently selected from the group consisting of locked nucleic acids (LNAs), bridged nucleic acids (BNAs) and analogues thereof, constrained ethyl nucleic acid (cEt), constrained ethyl BNAs (cET-BNAs), Tricyclo-DNA, and constrained methoxyethyl BNAs.

In some embodiments, the smartRNA comprises at least one nucleotide with a modified base, such as, isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine, 2-chloro-6-aminopurine, pseudouridine (Ψ), N1-methylspeudouridine (m1Ψ), 5-methylcytidine (m5C), N6-methyladenosine (m6A), 7-methylguanosine (m7G), 5-methoxyuridine, inosine, purine, xanthine, hypoxanthine, 2,2,7-trimethylguanosine (m3G/TMG cap), pyridin-4-one, pyridin-2-one, phenyl, 2,4,6-trimethoxy benzene, 3-methyl uracil, 2-thiouridine,s dihydrouridine, naphthyl, aminophenyl, 5-alkylcytidines, 5-fluoro-2'-deoxyuridine, 2',2'-difluoro-2'-deoxy nucleosides, 5-alkyluridines (e.g., ribothymidine), 5-halouridine (e.g., 5-bromouridine), 2',4'-difluorotoluyl nucleosides, 6-azapyrimidines, 6-alkylpyrimidines (e.g. 6-methyluridine), 5-nitroindole, and 3-nitropyrrole, preferably pseudouridine (Ψ), N1-methylspeudouridine (m1Ψ), and 5-methylcytidine (m5C).

In some embodiments, the smartRNA is a totalmer or a mixmer.

In some embodiments, the smartRNA comprises at least one modified internucleoside linkage.

In some embodiments, the smartRNA comprises at least one modified internucleoside linkage, wherein the modified internucleoside linkage is independently selected from the group consisting of phosphorothioates, chiral phosphorothioates, diphosphorothioates, phosphorodithioates, phosphoramidates, boranophosphates, methyl phosphonates, chiral methyl phosphonates, sulfonylphosphoramidates, methoxypropylphosphonates, 5'-vinylphosphonates, 5'-methylphosphonates, 5'-phosphorothioates, phosphodiesters, phosphotriesters. 5'-hydroxyphosphonates, Inverted nucleotide linkages (3'-3', and 5'-5'), and 3'-O-methylphosphonates internucleoside linkages.

In some embodiments, each internucleoside linkage of the smartRNA is either a phosphodiester internucleoside linkage, phosphorodithioate internucleoside linkage, or a phosphorothioate internucleoside linkage.

In some embodiments, the smartRNA comprises one or more chemically modified nucleotides, such as one or more nucleotide analogues, modified bases, modified backbones, and non-nucleotide linkages which are synthetic, naturally occuring, and non-naturally occurring; such as, phosphorothioates, chiral phosphorothioates, phosphorodithioates, phosphoramidates, methyl phosphonates, chiral methyl phosphonates, sulfonylphosphoramidates, methoxypropylphosphonates, 5'-vinylphosphonates, 5'-methylphosphonates, 5'-phosphorothioates, phosphodiesters, phosphotriesters, peptide-nucleic acids (PNAs), locked nucleic acids (LNAs), bridged nucleic acids (BNAs) and analogues thereof, constrained ethyl BNAs (cET-BNAs), constrained methoxyethyl BNAs, unlocked nucleic acids (UNAs), glycol nucleic acids (GNAs), phosphorodiamidate morpholino oligomers (PMOs), thiomorpholino oligomers (TMOs), ribonucleic acids (RNAs), 2'-deoxyribonucleic acids (DNAs), tricyclo-DNAs (tcDNAs), inverted nucleotides, 5'-5' reverse linkages, 3'-3' reverse linkages, L-ribose based nucleotides (mirror image nucleotides), 2',3'-dideoxy nucleotides, 2'-modified nucleotides such as, but not limited to, 2'-O-methyl (2'-O-Me), 2'-O-methoxyethyl (2'-O-MOE), 2'-fluoro (2'-F), 2'-arabino-fluoro (2'-Ara-F), 2'-methoxyethoxy, 2'-O-alkyl, 2'-O-alkenyl, 2'-O-alkynyl, 2'-O-benzyl, 2'-allyl, 2'-amino, 2'-azido, 2'-pyridinyl, 2'-O-propargyl, 2'-O-[2-(methylamino)-2-oxoethyl], 4'-thio, 2'-O-(N-methylcarbamate), nucleotides with modified bases such as, but not limited to, pseudouridine (Ψ), N1-methylspeudouridine (m1Ψ), 5-methylcytidine (m5C), N6-methyladenosine (m6A), 7-methylguanosine (m7G), 5-methoxyuridine, inosine, purine, xanthine, hypoxanthine, 2,2,7-trimethylguanosine (m3G/TMG cap), pyridin-4-one, pyridin-2-one, phenyl, 2,4,6-trimethoxy benzene, 3-methyl uracil, 2-thiouridine,s dihydrouridine, naphthyl, aminophenyl, 5-alkylcytidines, 5-fluoro-2'-deoxyuridine, 2',2'-difluoro-2'-deoxy nucleosides, 5-alkyluridines (e.g., ribothymidine), 5-halouridine (e.g., 5-bromouridine), 2',4'-difluorotoluyl nucleosides, 6-azapyrimidines, 6-alkylpyrimidines (e.g. 6-methyluridine), 5-nitroindole, 3-nitropyrrole, nucleosides or non-nucleoside linkers containing chemical handles such as, but not limited to, amines (e.g. aminohexyl), azides, alkynes, alkenes, acryls, thiols, hydrazines, aldehydes, biotin, digoxigenin, and others.

In some embodiments, the smartRNA is circularized, such as by at least partially annealing the "5' stem I" and the "3' stem I" nucleotide sequences to each other, or by covalently joining the 5'- and 3'-ends; preferably by covalently joining the 5'- and 3'- ends.

In some embodiments, the sequences complementary to each other (e.g. 5' stem I and 3' stem I sequences) comprises or consists of base pairs formed entirely or partially from base pairs selected from the group consisting of Watson-Crick base pairs, non-Watson-Crick base pairs, base pairs formed from non-natural and/or modified nucleotides.

In some embodiments, the sequences complementary to each other comprises or consists of base pairs formed entirely or partially from base pairs formed entirely or partially from Watson-Crick base pairs, such as guanine (G)-cytosine (C) base pairs, adenine (A)-thymine (T), and/or adenine (A) - uracil (U) base pairs.

In some embodiments, the sequences complementary to each other comprises or consists of base pairs formed entirely or partially from non-Watson-Crick base pairs, such as non-canonical base pairs, such as Hoogsteen base pair, such as G-A trans sugar edge, and/or Wobble base pairs.

In some embodiments, the non-canonical base pairs comprise U-U wobble base pairs, G-U wobble base pairs, and Hoogsteen/ sugar edge pairing A-G base pairs.

In one aspect, the smartRNA comprises (Scaffolds S1):
i. a "5' stem I" nucleotide sequence comprising or consisting of the nucleotide sequence GAUCAC or GGAUCAC;
ii. a "C-box" nucleotide sequence comprising or consisting of the nucleotide sequence GUGAUGA;
iii. a "D' guide" nucleotide sequence;
iv. a "D'-box" nucleotide sequence comprising or consisting of the nucleotide sequence CUGA;
v. a "5' stem II" nucleotide sequence comprising or consisting of the nucleotide sequence CCUCAG;
vi. optionally, a loop nucleotide sequence comprising or consisting of the nucleotide sequence GCCU;
vii. a "3' stem II" nucleotide sequence comprising or consisting of the nucleotide sequence CUGAGGG;
viii. a "C' box" nucleotide sequence comprising or consisting of the nucleotide sequence GUGAUGA;
ix. a "D guide" nucleotide sequence;
x. a "D-box" nucleotide sequence comprising or consisting of the nucleotide sequence CUGA; and
xi. a "3' stem I" nucleotide sequence comprising or consisting of the nucleotide sequence GUGAUC or GUGAUCC;

wherein the "D guide" nucleotide sequence comprises or consist of a first contiguous nucleotide sequence which is complementary to a same-length portion of a first target nucleic acid sequence of a target mRNA;
wherein the "D' guide" nucleotide sequence comprises a second contiguous nucleotide sequence which is complementary to a same-length portion of a second target nucleic acid sequence of the same target mRNA; and
wherein the first or second target nucleic acid sequence comprises the polyA-tail of said target mRNA.

In one aspect, the smartRNA comprises the sequences of Table 2a.

**Table 2a: Scaffolds S1 - Structure sequences**

| | |
|---|---|
| **5' stem 1** | (G)GAUCAC |
| **C box"** | GUGAUGA |
| **D' box** | CUGA |
| **5' stem 2** | CCUCAG |
| **Loop** | GCCU |
| **3' stem 2** | CUGAGGG |
| **C' box** | GUGAUGA |
| **D box** | CUGA |
| **3' stem 1** | GUGAUC(C) |

| | |
|---|---|
| *Nucleobases in parenthesis are optional.* | |

In one aspect, the smartRNA comprises:
i. a "5' stem I" nucleotide sequence comprising or consisting of the nucleotide sequence GGUUGGGUCA (SEQ ID NO: 34);
ii. a "C-box" nucleotide sequence comprising or consisting of the nucleotide sequence AUGAUGA;
iii. a "D' guide" nucleotide sequence;
iv. a "D'-box" nucleotide sequence comprising or consisting of the nucleotide sequence CUGA;
v. optionally, a loop nucleotide sequence comprising or consisting of the nucleotide sequence AGAGAG;
vi. a "C' box" nucleotide sequence comprising or consisting of the nucleotide sequence GUGAUGA;
vii. a "D guide" nucleotide sequence;
viii. a "D-box" nucleotide sequence comprising or consisting of the nucleotide sequence CUGA; and
ix. a "3' stem I" nucleotide sequence comprising or consisting of the nucleotide sequence GUCCCAGCCU (SEQ ID NO: 35);

wherein the "D guide" nucleotide sequence comprises or consist of a first contiguous nucleotide sequence which is complementary to a same-length portion of a first target nucleic acid sequence of a target mRNA;
wherein the "D' guide" nucleotide sequence comprises a second contiguous nucleotide sequence which is complementary to a same-length portion of a second target nucleic acid sequence of the same target mRNA; and
wherein the first or second target nucleic acid sequence comprises the polyA-tail of said target mRNA.

In one aspect, the smartRNA comprises the sequences of Table 2b.

**Table 2b: Scaffolds 115-2 - Structure sequences**

| | |
|---|---|
| **5' stem 1** | GGUUGGGUCA (SEQ ID NO: 34) |
| **C box"** | AUGAUGA |
| **D' box** | CUGA |
| **5' stem 2** | / |
| **Loop** | AGAGAG |
| **3' stem 2** | / |
| **C' box** | GUGAUGA |
| **D box** | CUGA |
| **3' stem 1** | GUCCCAGCCU (SEQ ID NO: 35) |

In one aspect, the smartRNA comprises:
i. a "5' stem I" nucleotide sequence comprising or consisting of the nucleotide sequence 
GGAAAGAGGAAC, GGAGCUGAGUCC, GGAGGCUUUGCA, GGAUACUUUCCU,
GGAUUGCUCACU, GGAUUGUGGCCA, GGCCCCCUUCCU, GGCCUAUCUCUC,
GGCUGCUCACCA, GGCUUUACAGUA, GGGCUGGCAUAU, GGGGAUCACG,
GGGGCUGGAUUU, GGGGGCGGGGCG, GGGGGGAUCACG, GGGGUUGGAUCG,
GGGGUUGGGUCA, or GGUGCCCUGCCU (SEQ ID NO: 36-53);
ii. a "C-box" nucleotide sequence comprising or consisting of the nucleotide sequence GUGAUGA, AUGAUGA, AUGAGGA, CUGAUGA, or AGGAUGA;
iii. a "D' guide" nucleotide sequence;
iv. a "D'-box" nucleotide sequence comprising or consisting of the nucleotide sequence CUGA, CAGA, UUGA, or CCGA;
v. optionally, a "5' stem II" nucleotide sequence comprising or consisting of the nucleotide sequence UCA, AG, CCUCAG, or UUUUAUGUGC (SEQ ID NO: 72);
vi. optionally, a loop nucleotide sequence comprising or consisting of the nucleotide sequence GUGUCG, UCGACU, AUGUG, GCAAAGAAAGGCCUUU (SEQ ID NO: 73), GC, AGAGAG, GCUCC, GCCU, ACCAC, GUCACC, GAGUA, AAUGUUUUUCCUU (SEQ ID NO: 74), GUGCAAUCA, UUCU, GCUGCU, GACACC, CAGAUCGACU (SEQ ID NO: 75), or UCAAUGU;
vii. optionally, a "3' stem II" nucleotide sequence comprising or consisting of the nucleotide sequence UGA, CU, CUGAGGG, or GCU;
viii. a "C' box" nucleotide sequence comprising or consisting of the nucleotide sequence GUGCUGA, AUGUUGA, CUGAAAG, GUGUGGA, AUGAUGA, GUGAUGA, GUGAGGA, AUGAUUU, AACAUGU, AUGUUGG, CUGAUGU, GUGAUGG, GUGAUUU, AUGAUGG, or GUGACUG;
ix. a "D guide" nucleotide sequence;
x. a "D-box" nucleotide sequence comprising or consisting of the nucleotide sequence CUGA; and
xi. a "3' stem I" nucleotide sequence comprising or consisting of the nucleotide sequence

GCUGCCGCCCCAUAG, UAUGCCAGCUCAUAG, GCUGUGAGCCCAUAG,
AACUCAGUUUCAUAG, AGUCCAGCCUCAUAG, GUCCCAGCCUCAUAG,
GGAGAUGGGCCAUAG, GUGAUCCCCCAUAG, UGCUGUUUGCCAUAG,
GGCCCAGCCUCAUAG, GUCCUCUGACCAUAG, UGGUGGCACACAUAG,
GUGAGCAUCUCAUAG, GCAAUGCCUGCAUAG, GUGAUCACAUAG,
GGCCAAAUUGCAUAG, AGAAGAGAUGCAUAG, or GGAAGGGAGGCAUAG (SEQ ID
NO: 54-71);

wherein the "D guide" nucleotide sequence comprises or consist of a first contiguous nucleotide sequence which is complementary to a same-length portion of a first target nucleic acid sequence of a target mRNA;
wherein the "D' guide" nucleotide sequence comprises a second contiguous nucleotide sequence which is complementary to a same-length portion of a second target nucleic acid sequence of the same target mRNA; and
wherein the first or second target nucleic acid sequence comprises the polyA-tail of said target mRNA.

In one aspect, the smartRNA comprises the sequences of Table 2c and, optionally, the sequences of Table 2d.

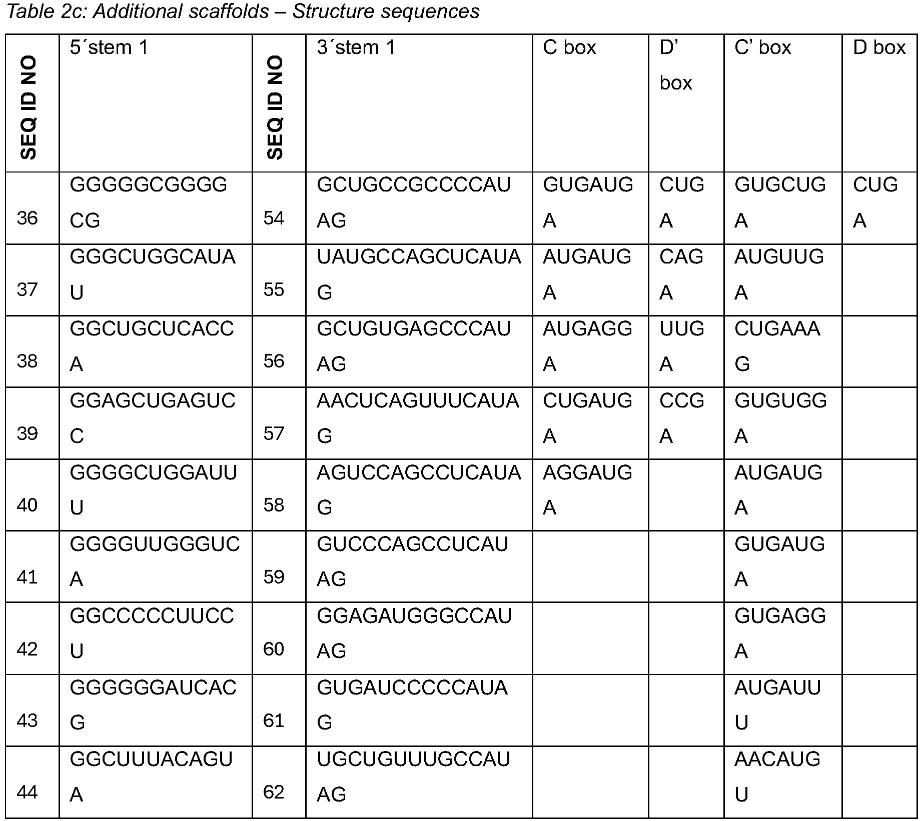

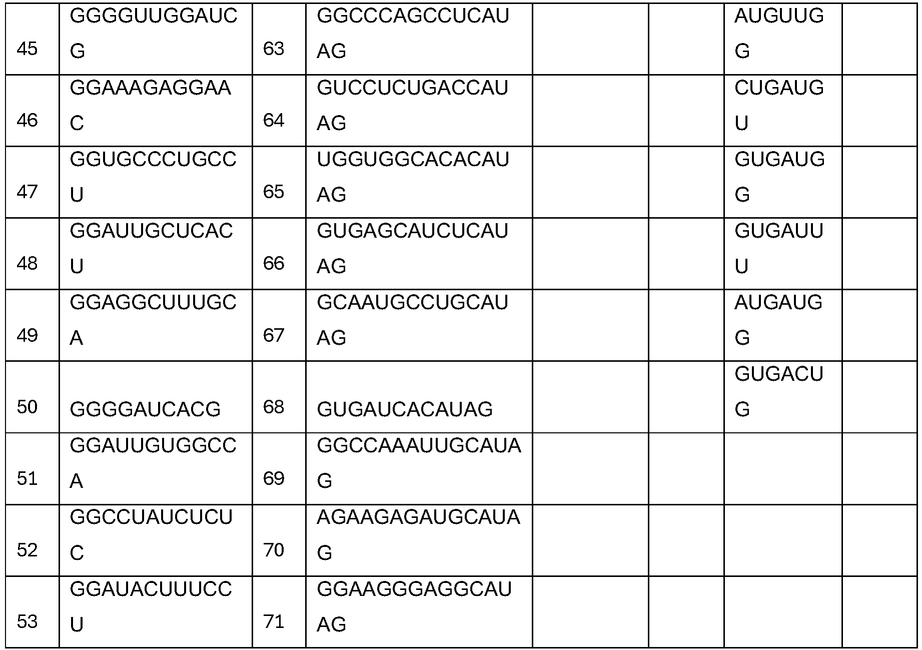

**Table 2d: Additional scaffolds - Optional structure sequences**

| **5'stem 2** | **loop** | **3'stem 2** |
|---|---|---|
| UCA | GUGUCG | UGA |
| AG | UCGACU | CU |
| CCUCAG | AUGUG | CUGAGGG |
| UUUUAUGUGC (SEQ ID NO: 72) | GCAAAGAAAGGCCUUU (SEQ ID NO: 73) | GCU |
| | GC | |
| | AGAGAG | |
| | GCUCC | |
| | GCCU | |
| | ACCAC | |
| | GUCACC | |
| | GAGUA | |
| | AAUGUUUUUCCUU (SEQ ID NO: 74) | |
| | GUGCAAUCA | |
| | UUCU | |
| | GCUGCU | |
| | GACACC | |
| | CAGAUCGACU (SEQ ID NO: 75) | |
| | UCAAUGU | |

In some embodiments, the "D' guide" nucleotide sequence is selected from the group consisting of SEQ ID NO: 76 to 84, and/or any one of the sequences of Table 3.

In some embodiments, the "D' guide" nucleotide sequence is selected from the group consisting of the nucleotide sequences of SEQ ID NO: 76 to 84, and/or the nucleotide sequences of any one of the sequences of Table 3.

In some embodiments, the "D guide" nucleotide sequence is selected from the group consisting of SEQ ID NO: 76 to 84 and/or any one of the sequences of Table 3.

In some embodiments, the "D guide" nucleotide sequence is selected from the group consisting of the nucleotide sequences of SEQ ID NO: 76 to 84, and/or the nucleotide sequences of any one of the sequences of Table 3.

**Table 3: Scaffolds - "D' guide" and/or "D guide" sequences**

| **SEQ ID NO** | **"D' guide" and/or "D guide" nucleotide sequence** |
|---|---|
| 76 | GAUUUUUUUUUUU |
| 77 | AGAGUCACAGUUUGCUGACU |
| 78 | AUAGUCACAUAUAAUAAACU |
| 79 | GACUCCAAACAUUUGAAUGU |
| 80 | AUGGCUAAACAAAGUGCAGU |
| 81 | AACUAUCUAUAAAUGGUACU |
| 82 | GAAAAAGUACUAUGGACAGU |
| 83 | UUCAAUUCAGUUAGUGCAGU |
| 84 | UAAGUAGUUUUAAGGAAAAU |

In one aspect, the compound of the invention comprises or consists of a small artificial RNA (smartRNA) oligonucleotide comprising, or consists of, a sequence selected from the group consisting of SEQ ID NO: 7 to 10.

In one aspect, the compound of the invention comprises or consists of a small artificial RNA (smartRNA) oligonucleotide comprising, or consists of, the nucleotide sequence of a sequence selected from the group consisting of SEQ ID NO: 7 to 10.

In one aspect, the compound of the invention comprises or consists of a small artificial RNA (smartRNA) oligonucleotide comprising, or consists of, a sequence selected from the group consisting of SEQ ID NO: 85 to 100 and/or SEQ ID NO: 101 to 116.

In one aspect, the compound of the invention comprises or consists of a small artificial RNA (smartRNA) oligonucleotide comprising, or consists of, the nucleotide sequence of a sequence selected from the group consisting of SEQ ID NO: 85 to 100 and/or SEQ ID NO: 101 to 116

In one aspect, the compound of the invention comprises or consists of a small artificial RNA (smartRNA) oligonucleotide comprising, or consists of, a sequence selected Table 4a.

In one aspect, the compound of the invention comprises or consists of a small artificial RNA (smartRNA) oligonucleotide comprising, or consists of, the nucleotide sequence of a sequence selected Table 4a.

**Table 4a: Scaffolds S1**

| SEQ ID NO | Compound ID | Sequence (5'-3') |
|---|---|---|
| 85 | STX001 | GGAUCACG GUGAUGA GAUUUUUUUUUUU CUGA CCUCAG GCCU CUGAGGG GUGAUGA AGAGUCACAGUUUGCUGACU CUGA GUGAUCC |
| 86 | STX002 | GGAUCACG GUGAUGA AGAGUCACAGUUUGCUGACU CUGA CCUCAG GCCU CUGAGGG GUGAUGA GAUUUUUUUUUUU CUGA GUGAUCC |
| 87 | STX003 | GGAUCACG GUGAUGA AUAGUCACAUAUAAUAAACU CUGA CCUCAG GCCU CUGAGGG GUGAUGA GAUUUUUUUUUUU CUGA GUGAUCC |
| 88 | STX004 | GGAUCACG GUGAUGA GAUUUUUUUUUUU CUGA CCUCAG GCCU CUGAGGG GUGAUGA AUAGUCACAUAUAAUAAACU CUGA GUGAUCC |
| 89 | STX005 | GGAUCACG GUGAUGA GAUUUUUUUUUUU CUGA CCUCAG GCCU CUGAGGG GUGAUGA GACUCCAAACAUUUGAAUGU CUGA GUGAUCC |
| 90 | STX006 | GGAUCACG GUGAUGA GACUCCAAACAUUUGAAUGU CUGA CCUCAG GCCU CUGAGGG GUGAUGA GAUUUUUUUUUUU CUGA GUGAUCC |
| 91 | STX007 | GGAUCACG GUGAUGA AUGGCUAAACAAAGUGCAGU CUGA CCUCAG GCCU CUGAGGG GUGAUGA GAUUUUUUUUUUU CUGA GUGAUCC |
| 92 | STX008 | GGAUCACG GUGAUGA GAUUUUUUUUUUU CUGA CCUCAG GCCU CUGAGGG GUGAUGA AUGGCUAAACAAAGUGCAGU CUGA GUGAUCC |
| 93 | STX009 | GGAUCACG GUGAUGA AACUAUCUAUAAAUGGUACU CUGA CCUCAG GCCU CUGAGGG GUGAUGA GAUUUUUUUUUUU CUGA GUGAUCC |
| 94 | STX010 | GGAUCACG GUGAUGA GAUUUUUUUUUUU CUGA CCUCAG GCCU CUGAGGG GUGAUGA AACUAUCUAUAAAUGGUACU CUGA GUGAUCC |
| 95 | STX011 | GGAUCACG GUGAUGA GAAAAAGUACUAUGGACAGU CUGA CCUCAG GCCU CUGAGGG GUGAUGA GAUUUUUUUUUUU CUGA GUGAUCC |
| 96 | STX012 | GGAUCACG GUGAUGA GAUUUUUUUUUUU CUGA CCUCAG GCCU CUGAGGG GUGAUGA GAAAAAGUACUAUGGACAGU CUGA GUGAUCC |
| 97 | STX013 | GGAUCACG GUGAUGA GAUUUUUUUUUUU CUGA CCUCAG GCCU CUGAGGG GUGAUGA UUCAAUUCAGUUAGUGCAGU CUGA GUGAUCC |
| 98 | STX014 | GGAUCACG GUGAUGA UUCAAUUCAGUUAGUGCAGU CUGA CCUCAG GCCU CUGAGGG GUGAUGA GAUUUUUUUUUUU CUGA GUGAUCC |
| 99 | STX015 | GGAUCACG GUGAUGA UAAGUAGUUUUAAGGAAAAU CUGA CCUCAG GCCU CUGAGGG GUGAUGA GAUUUUUUUUUUU CUGA GUGAUCC |
| 100 | STX016 | GGAUCACG GUGAUGA GAUUUUUUUUUUU CUGA CCUCAG GCCU CUGAGGG GUGAUGA UAAGUAGUUUUAAGGAAAAU CUGA GUGAUCC |
| 101 | STX001b | GAUCACG GUGAUGA GAUUUUUUUUUUU CUGA CCUCAG GCCU CUGAGGG GUGAUGA AGAGUCACAGUUUGCUGACU CUGA GUGAUC |
| 102 | STX002b | GAUCACG GUGAUGA AGAGUCACAGUUUGCUGACU CUGA CCUCAG GCCU CUGAGGG GUGAUGA GAUUUUUUUUUUU CUGA GUGAUC |
| 103 | STX003b | GAUCACG GUGAUGA AUAGUCACAUAUAAUAAACU CUGA CCUCAG GCCU CUGAGGG GUGAUGA GAUUUUUUUUUUU CUGA GUGAUC |
| 104 | STX004b | GAUCACG GUGAUGA GAUUUUUUUUUUU CUGA CCUCAG GCCU CUGAGGG GUGAUGA AUAGUCACAUAUAAUAAACU CUGA GUGAUC |
| 105 | STX005b | GAUCACG GUGAUGA GAUUUUUUUUUUU CUGA CCUCAG GCCU CUGAGGG GUGAUGA GACUCCAAACAUUUGAAUGU CUGA GUGAUC |
| 106 | STX006b | GAUCACG GUGAUGA GACUCCAAACAUUUGAAUGU CUGA CCUCAG GCCU CUGAGGG GUGAUGA GAUUUUUUUUUUU CUGA GUGAUC |
| 107 | STX007b | GAUCACG GUGAUGA AUGGCUAAACAAAGUGCAGU CUGA CCUCAG GCCU CUGAGGG GUGAUGA GAUUUUUUUUUUU CUGA GUGAUC |
| 108 | STX008b | GAUCACG GUGAUGA GAUUUUUUUUUUU CUGA CCUCAG GCCU CUGAGGG GUGAUGA AUGGCUAAACAAAGUGCAGU CUGA GUGAUC |
| 109 | STX009b | GAUCACG GUGAUGA AACUAUCUAUAAAUGGUACU CUGA CCUCAG GCCU CUGAGGG GUGAUGA GAUUUUUUUUUUU CUGA GUGAUC |
| 110 | STX010b | GAUCACG GUGAUGA GAUUUUUUUUUUU CUGA CCUCAG GCCU CUGAGGG GUGAUGA AACUAUCUAUAAAUGGUACU CUGA GUGAUC |
| 111 | STX011b | GAUCACG GUGAUGA GAAAAAGUACUAUGGACAGU CUGA CCUCAG GCCU CUGAGGG GUGAUGA GAUUUUUUUUUUU CUGA GUGAUCC |
| 112 | STX012b | GAUCACG GUGAUGA GAUUUUUUUUUUU CUGA CCUCAG GCCU CUGAGGG GUGAUGA GAAAAAGUACUAUGGACAGU CUGA GUGAUC |
| 113 | STX013b | GGAUCACG GUGAUGA GAUUUUUUUUUUU CUGA CCUCAG GCCU CUGAGGG GUGAUGA UUCAAUUCAGUUAGUGCAGU CUGA GUGAUC |
| 114 | STX014b | GAUCACG GUGAUGA UUCAAUUCAGUUAGUGCAGU CUGA CCUCAG GCCU CUGAGGG GUGAUGA GAUUUUUUUUUUU CUGA GUGAUC |
| 115 | STX015b | GAUCACG GUGAUGA UAAGUAGUUUUAAGGAAAAU CUGA CCUCAG GCCU CUGAGGG GUGAUGA GAUUUUUUUUUUU CUGA GUGAUC |
| 116 | STX016b | GAUCACG GUGAUGA GAUUUUUUUUUUU CUGA CCUCAG GCCU CUGAGGG GUGAUGA UAAGUAGUUUUAAGGAAAAU CUGA GUGAUC |

| | | |
|---|---|---|
| *Note: the spaces inside the sequences in the table above are only intended to make the sequences easier to read and to identify the different structure sequences. It should therefore be understood that these spaces are absent from the actual sequences, and should not be considered as nicks.* | | |

In one aspect, the compound of the invention comprises or consists of a small artificial RNA (smartRNA) oligonucleotide comprising, or consists of, a sequence selected from the group consisting of SEQ ID NO: 117 to 132.

In one aspect, the compound of the invention comprises or consists of a small artificial RNA (smartRNA) oligonucleotide comprising, or consists of, the nucleotide sequence of a sequence selected from the group consisting of SEQ ID NO: 117 to 132.

In one aspect, the compound of the invention comprises or consists of a small artificial RNA (smartRNA) oligonucleotide comprising, or consists of, a sequence selected Table 4b.

In one aspect, the compound of the invention comprises or consists of a small artificial RNA (smartRNA) oligonucleotide comprising, or consists of, the nucleotide sequence of a sequence selected Table 4b.

**Table 4b: Scaffolds 115-2**

| SEQ ID NO | Compound ID | Sequence (5'-3') |
|---|---|---|
| 117 | STX017 | GGUUGGGUCA AUGAUGA GAUUUUUUUUUUU CUGA AGAGAG GUGAUGA AGAGUCACAGUUUGCUGACU CUGA GUCCCAGCCU |
| 118 | STX018 | GGUUGGGUCA AUGAUGA AGAGUCACAGUUUGCUGACU CUGA AGAGAG GUGAUGA GAUUUUUUUUUUU CUGA GUCCCAGCCU |
| 119 | STX019 | GGUUGGGUCA AUGAUGA AUAGUCACAUAUAAUAAACU CUGA AGAGAG GUGAUGA GAUUUUUUUUUUU CUGA GUCCCAGCCU |
| 120 | STX020 | GGUUGGGUCA AUGAUGA GAUUUUUUUUUUU CUGA AGAGAG GUGAUGA AUAGUCACAUAUAAUAAACU CUGA GUCCCAGCCU |
| 121 | STX021 | GGUUGGGUCA AUGAUGA GAUUUUUUUUUUU CUGA AGAGAG GUGAUGA GACUCCAAACAUUUGAAUGU CUGA GUCCCAGCCU |
| 122 | STX022 | GGUUGGGUCA AUGAUGA GACUCCAAACAUUUGAAUGU CUGA AGAGAG GUGAUGA GAUUUUUUUUUUU CUGA GUCCCAGCCU |
| 123 | STX023 | GGUUGGGUCA AUGAUGA AUGGCUAAACAAAGUGCAGU CUGA AGAGAG GUGAUGA GAUUUUUUUUUUU CUGA GUCCCAGCCU |
| 124 | STX024 | GGUUGGGUCA AUGAUGA GAUUUUUUUUUUU CUGA AGAGAG GUGAUGA AUGGCUAAACAAAGUGCAGU CUGA GUCCCAGCCU |
| 125 | STX025 | GGUUGGGUCA AUGAUGA AACUAUCUAUAAAUGGUACU CUGA AGAGAG GUGAUGA GAUUUUUUUUUUU CUGA GUCCCAGCCU |
| 126 | STX026 | GGUUGGGUCA AUGAUGA GAUUUUUUUUUUU CUGA AGAGAG GUGAUGA AACUAUCUAUAAAUGGUACU CUGA GUCCCAGCCU |
| 127 | STX027 | GGUUGGGUCA AUGAUGA GAAAAAGUACUAUGGACAGU CUGA AGAGAG GUGAUGA GAUUUUUUUUUUU CUGA GUCCCAGCCU |
| 128 | STX028 | GGUUGGGUCA AUGAUGA GAUUUUUUUUUUU CUGA AGAGAG GUGAUGA GAAAAAGUACUAUGGACAGU CUGA GUCCCAGCCU |
| 129 | STX029 | GGUUGGGUCA AUGAUGA GAUUUUUUUUUUU CUGA AGAGAG GUGAUGA UUCAAUUCAGUUAGUGCAGU CUGA GUCCCAGCCU |
| 130 | STX030 | GGUUGGGUCA AUGAUGA UUCAAUUCAGUUAGUGCAGU CUGA AGAGAG GUGAUGA GAUUUUUUUUUUU CUGA GUCCCAGCCU |
| 131 | STX031 | GGUUGGGUCA AUGAUGA UAAGUAGUUUUAAGGAAAAU CUGA AGAGAG GUGAUGA GAUUUUUUUUUUU CUGA GUCCCAGCCU |
| 132 | STX032 | GGUUGGUCA AUGAUGA GAUUUUUUUUUU CUGA AGAGAG GUGAUGA UAAGUAGUUUAAGGAAAAU CUGA GUCCCAGCCU |

| | | |
|---|---|---|
| *Note: the spaces inside the sequences in the table above are only intended to make the sequences easier to read and to identify the different structure sequences. It should therefore be understood that these spaces are absent from the actual sequences, and should not be considered as nicks.* | | |

In one aspect, the compound of the invention comprises or consists of a small artificial RNA (smartRNA) oligonucleotide comprising, or consists of, a sequence selected from the group consisting of SEQ ID NO: 133 to 169.

In one aspect, the compound of the invention comprises or consists of a small artificial RNA (smartRNA) oligonucleotide comprising, or consists of, the nucleotide sequence of a sequence selected from the group consisting of SEQ ID NO: 133 to 169.

In one aspect, the compound of the invention comprises or consists of a small artificial RNA (smartRNA) oligonucleotide comprising, or consists of, a sequence selected Table 4c.

In one aspect, the compound of the invention comprises or consists of a small artificial RNA (smartRNA) oligonucleotide comprising, or consists of, the nucleotide sequence of a sequence selected Table 4c.

**Table 4c: Additional scaffolds**

| SEQ ID NO | Compound ID | Sequence (5'-3') |
|---|---|---|
| 133 | STX038* | GGGGGCGGGGCG GUGAUGA GAUUUUUUUUUUU CUGA GUGUCG GUGCUGA AGAGUCACAGUUUGCUGACU CUGA GCUGCCGCCCCAUAG |
| 134 | STX039* | GGGGGCGGGGCG GUGAUGA AGAGUCACAGUUUGCUGACU CUGA GUGUCG GUGCUGA GAUUUUUUUUUUU CUGA GCUGCCGCCCCAUAG |
| 135 | STX040* | GGGCUGGCAUAU AUGAUGA GAUUUUUUUUUUU CAGA UCGACU AUGUUGA AGAGUCACAGUUUGCUGACU CUGA UAUGCCAGCUCAUAG |
| 136 | STX041* | GGGCUGGCAUAU AUGAUGA AGAGUCACAGUUUGCUGACU CAGA UCGACU AUGUUGA GAUUUUUUUUUUU CUGA UAUGCCAGCUCAUAG |
| 137 | STX042 | GGCUGCUCACCA GUGAUGA GAUUUUUUUUUUU CUGA UCA AUGUG UGA CUGAAAG AGAGUCACAGUUUGCUGACU CUGA GCUGUGAGCCCAUAG |
| 138 | STX043 | GGCUGCUCACCA GUGAUGA AGAGUCACAGUUUGCUGACU CUGA UCA AUGUG UGA CUGAAAG GAUUUUUUUUUUU CUGA GCUGUGAGCCCAUAG |
| 139 | STX044 | GGAGCUGAGUCC AUGAUGA GAUUUUUUUUUUU CUGA AG GCAAAGAAAGGCCUUU CU GUGUGGA AGAGUCACAGUUUGCUGACU CUGA AACUCAGUUUCAUAG |
| 140 | STX045 | GGAGCUGAGUCC AUGAUGA AGAGUCACAGUUUGCUGACU CUGA AG GCAAAGAAAGGCCUUU CU GUGUGGA GAUUUUUUUUUUU CUGA AACUCAGUUUCAUAG |
| 141 | STX046* | GGGGCUGGAUUU GUGAUGA GAUUUUUUUUUUU CUGA GC AUGAUGA AGAGUCACAGUUUGCUGACU CUGA AGUCCAGCCUCAUAG |
| 142 | STX047* | GGGGCUGGAUUU GUGAUGA AGAGUCACAGUUUGCUGACU CUGA GC AUGAUGA GAUUUUUUUUUUU CUGA AGUCCAGCCUCAUAG |
| 143 | STX048* | GGGGUUGGGUCA AUGAUGA GAUUUUUUUUUUU CUGA AGAGAG GUGAUGA AGAGUCACAGUUUGCUGACU CUGA GUCCCAGCCUCAUAG |
| 144 | STX049* | GGGGUUGGGUCA AUGAUGA AGAGUCACAGUUUGCUGACU CUGA AGAGAG GUGAUGA GAUUUUUUUUUUU CUGA GUCCCAGCCUCAUAG |
| 145 | STX050* | GGCCCCCUUCCU AUGAUGA GAUUUUUUUUUU CUGA GCUCC GUGAGGA AGAGUCACAGUUUGCUGACU CUGA GGAGAUGGCCAUAG |
| 146 | STX051* | GGCCCCCUUCCU AUGAUGA AGAGUCACAGUUUGCUGACU CUGA GCUCC GUGAGGA GAUUUUUUUUUUU CUGA GGAGAUGGGCCAUAG |
| 147 | STX052 | GGGGGGAUCACG GUGAUGA GAUUUUUUUUUUU CUGA CCUCAG GCCU CUGAGGG GUGAUGA AGAGUCACAGUUUGCUGACU CUGA GUGAUCCCCCAUAG |
| 148 | STX053 | GGGGGGAUCACG GUGAUGA AGAGUCACAGUUUGCUGACU CUGA CCUCAG GCCU CUGAGGG GUGAUGA GAUUUUUUUUUUU CUGA GUGAUCCCCCAUAG |
| 149 | STX054* | GGCUUUACAGUA GUGAUGA GAUUUUUUUUUUU CUGA ACCAC AUGAUUU AGAGUCACAGUUUGCUGACU CUGA UGCUGUUUGCCAUAG |
| 150 | STX055* | GGCUUUACAGUA GUGAUGA AGAGUCACAGUUUGCUGACU CUGA ACCAC AUGAUUU GAUUUUUUUUUU CUGA UGCUGUUGCCAUAG |
| 151 | STX056* | GGGGUUGGAUCG AUGAUGA GAUUUUUUUUUUU CUGA AGAGAG GUGAUGA AGAGUCACAGUUUGCUGACU CUGA GGCCCAGCCUCAUAG |
| 152 | STX057* | GGGGUUGGAUCG AUGAUGA AGAGUCACAGUUUGCUGACU CUGA AGAGAG GUGAUGA GAUUUUUUUUUUU CUGA GGCCCAGCCUCAUAG |
| 153 | STX058* | GGAAAGAGGAAC AUGAGGA GAUUUUUUUUUUU UUGA GUCACC AACAUGU AGAGUCACAGUUUGCUGACU CUGA GUCCUCUGACCAUAG |
| 154 | STX059* | GGAAAGAGGAAC AUGAGGA AGAGUCACAGUUUGCUGACU UUGA GUCACC AACAUGU GAUUUUUUUUUUU CUGA GUCCUCUGACCAUAG |
| 155 | STX060* | GGUGCCCUGCCU CUGAUGA GAUUUUUUUUUUU CUGA GAGUA AUGAUGA AGAGUCACAGUUUGCUGACU CUGA UGGUGGCACACAUAG |
| 156 | STX061* | GGUGCCCUGCCU CUGAUGA AGAGUCACAGUUUGCUGACU CUGA GAGUA AUGAUGA GAUUUUUUUUUUU CUGA UGGUGGCACACAUAG |
| 157 | STX065* | GGAUUGCUCACU AUGAUGA AGAGUCACAGUUUGCUGACU CAGA AAUGUUUUUCCUU AUGUUGG GAUUUUUUUUUUU CUGA GUGAGCAUCUCAUAG |
| 158 | STX066* | GGAGGCUUUGCA GUGAUGA GAUUUUUUUUUUU CUGA GUGCAAUCA CUGAUGU AGAGUCACAGUUUGCUGACU CUGA GCAAUGCCUGCAUAG |
| 159 | STX067* | GGAGGCUUUGCA GUGAUGA AGAGUCACAGUUUGCUGACU CUGA GUGCAAUCA CUGAUGU GAUUUUUUUUUUU CUGA GCAAUGCCUGCAUAG |
| 160 | STX068 | GGGGAUCACG GUGAUGA GAUUUUUUUUUUU CUGA CCUCAG GCCU CUGAGGG GUGAUGA AGAGUCACAGUUUGCUGACU CUGA GUGAUCACAUAG |
| 161 | STX069 | GGGGAUCACG GUGAUGA AGAGUCACAGUUUGCUGACU CUGA CCUCAG GCCU CUGAGGG GUGAUGA GAUUUUUUUUUUU CUGA GUGAUCACAUAG |
| 162 | STX070 | GGAUUGUGGCCA AGGAUGA GAUUUUUUUUUUU CUGA UUUUAUGUGC UUCU GCU GUGAUGG AGAGUCACAGUUUGCUGACU CUGA GGCCAAAUUGCAUAG |
| 163 | STX071 | GGAUUGUGGCCA AGGAUGA AGAGUCACAGUUUGCUGACU CUGA UUUUAUGUGC UUCU GCU GUGAUGG GAUUUUUUUUUUU CUGA GGCCAAAUUGCAUAG |
| 164 | STX073* | GGCCUAUCUCUC AUGAUGA AGAGUCACAGUUUGCUGACU CUGA GCUGCU GUGAUUU GAUUUUUUUUUUU CUGA AGAAGAGAUGCAUAG |
| 165 | STX077* | GGAUACUUUCCU CUGAUGA AGAGUCACAGUUUGCUGACU CUGA GACACC AUGAUGG GAUUUUUUUUUUU CUGA GGAAGGGAGGCAUAG |
| 166 | STX078* | GGGCUGGCAUAU AUGAUGA GAUUUUUUUUUUU CCGA CAGAUCGACU AUGUUGA AGAGUCACAGUUUGCUGACU CUGA UAUGCCAGCUCAUAG |
| 167 | STX079* | GGGCUGGCAUAU AUGAUGA AGAGUCACAGUUUGCUGACU CCGA CAGAUCGACU AUGUUGA GAUUUUUUUUUUU CUGA UAUGCCAGCUCAUAG |
| 168 | STX080* | GGCUGCUCACCA GUGAUGA GAUUUUUUUUUUU CUGA UCAAUGU GUGACUG AGAGUCACAGUUUGCUGACU CUGA GCUGUGAGCCCAUAG |
| 169 | STX081* | GGCUGCUCACCA GUGAUGA AGAGUCACAGUUUGCUGACU CUGA UCAAUGU GUGACUG GAUUUUUUUUUUU CUGA GCUGUGAGCCCAUAG |

| | | |
|---|---|---|
| **Scaffolds without stem 2 sequences (5' and 3' stem* 2 *sequences)* *Note: the spaces inside the sequences in the table above are only intended to make the sequences easier to read and to identify the different structure sequences. It should therefore be understood that these spaces are absent from the actual sequences, and should not be considered as nicks.* | | |

In one aspect, the compound of the invention comprises or consists of a small artificial RNA (smartRNA) oligonucleotide comprising, or consists of, a sequence selected from the group consisting of SEQ ID NO: 170 to 171, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, a sequence selected in Table 5a, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, the nucleotide sequence of a sequence selected from the group consisting of SEQ ID NO: 170 to 171, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, the nucleotide sequence of a sequence selected in Table 5a, wherein the term "N₇₋₃₀" corresponds to the "D guide" or "D' guide" sequence, comprising or consisting of a contiguous nucleotide sequence of 7 to 30 contiguous nucleotides in length.

Preferably, the "D guide" and the "D' guide" (represented by the terms "N₇₋₃₀") comprise or consist of a contiguous nucleotide sequence of between 7 to 15, or 12 to 30 contiguous nucleotides in length.

More preferably, when the "D guide" comprises or consists of a contiguous nucleotide sequence of between 7 to 15 contiguous nucleotides in length, the "D' guide" comprises or consists of a contiguous nucleotide sequence of between 12 to 30 contiguous nucleotides in length; and *vice versa.*

**Table 5a: Scaffolds S1 - Scaffold sequences with generic "D guide" or "D' guide" sequences**

| SEQ ID NO | Design | Sequence (5'-3') |
|---|---|---|
| 170 | S1 | GGAUCACG GUGAUGA N7-30 CUGA CCUCAG GCCU CUGAGGG GUGAUGA N7-30 CUGA GUGAUCC |
| 171 | S1 | GAUCACG GUGAUGA N7-30 CUGA CCUCAG GCCU CUGAGGG GUGAUGA N7-30 CUGA GUGAUC |

| | | |
|---|---|---|
| *Note: the spaces inside the sequences in the table above are only intended to make the sequences easier to read and to identify the different structure sequences. It should therefore be understood that these spaces are absent from the actual sequences, and should not be considered as nicks.* | | |

In one aspect, the compound of the invention comprises or consists of a small artificial RNA (smartRNA) oligonucleotide comprising, or consists of, a sequence of SEQ ID NO: 172, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, the nucleotide sequence of a sequence of SEQ ID NO: 172, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, a sequence selected in Table 5b, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, the nucleotide sequence of a sequence selected in Table 5b, wherein the term "N₇₋₃₀" corresponds to the "D guide" or "D' guide" sequence, comprising or consisting of a contiguous nucleotide sequence of 7 to 30 contiguous nucleotides in length.

Preferably, the "D guide" and the "D' guide" (represented by the terms "N₇-30") comprise or consist of a contiguous nucleotide sequence of between 7 to 15, or 12 to 30 contiguous nucleotides in length.

More preferably, when the "D guide" comprises or consists of a contiguous nucleotide sequence of between 7 to 15 contiguous nucleotides in length, the "D' guide" comprises or consists of a contiguous nucleotide sequence of between 12 to 30 contiguous nucleotides in length; and *vice versa.*

**Table 5b: Scaffolds S115-2 - Scaffold sequences with generic "D guide" or "D' guide" sequences**

| SEQ ID NO | Design | Sequence (5'-3') |
|---|---|---|
| 172 | S115-2 | GGUUGGGUCA AUGAUGA N7-30 CUGA AGAGAG GUGAUGA N7-30 CUGA GUCCCAGCCU |

| | | |
|---|---|---|
| *Note: the spaces inside the sequences in the table above are only intended to make the sequences easier to read and to identify the different structure sequences. It should therefore be understood that these spaces are absent from the actual sequences, and should not be considered as nicks.* | | |

In one aspect, the compound of the invention comprises or consists of a small artificial RNA (smartRNA) oligonucleotide comprising, or consists of, a sequence selected from the group consisting of SEQ ID NO: 173 to 192, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, the nucleotide sequence of a sequence selected from the group consisting of SEQ ID NO: 173 to 192, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, a sequence selected Table 5c, wherein the term "N₇₋₃₀" corresponds to the "D guide" or "D' guide" sequence, comprising or consisting of a contiguous nucleotide sequence of 7 to 30 contiguous nucleotides in length.

In one aspect, the compound of the invention comprises or consists of a small artificial RNA (smartRNA) oligonucleotide comprising, or consists of, the nucleotide sequence of a sequence selected Table 5c, wherein the term "N₇₋₃₀" corresponds to the "D guide" or "D' guide" sequence, comprising or consisting of a contiguous nucleotide sequence of 7 to 30 contiguous nucleotides in length.

Preferably, the "D guide" and the "D' guide" (represented by the terms "N₇₋₃₀") comprise or consist of a contiguous nucleotide sequence of between 7 to 15, or 12 to 30 contiguous nucleotides in length.

More preferably, when the "D guide" comprises or consists of a contiguous nucleotide sequence of between 7 to 15 contiguous nucleotides in length, the "D' guide" comprises or consists of a contiguous nucleotide sequence of between 12 to 30 contiguous nucleotides in length; and *vice versa.*

**Table 5c: Additional scaffolds - Scaffold sequences with generic "D guide" or "D' guide" sequences**

| SEQ ID NO | Design | Sequence (5'-3') |
|---|---|---|
| 173 | STX038 | GGGGGCGGGGCG GUGAUGA N7-30 CUGA GUGUCG GUGCUGA N7- 30 CUGA GCUGCCGCCCCAUAG |
| 174 | STX040 | GGGCUGGCAUAU AUGAUGA N7-30 CAGA UCGACU AUGUUGA N7-30 CUGA UAUGCCAGCUCAUAG |
| 175 | STX042 | GGCUGCUCACCA GUGAUGA N7-30 CUGA UCA AUGUG UGA CUGAAAG N7-30 CUGA GCUGUGAGCCCAUAG |
| 176 | STX044 | GGAGCUGAGUCC AUGAUGA N7-30 CUGA AG GCAAAGAAAGGCCUUU CU GUGUGGA N7-30 CUGA AACUCAGUUUCAUAG |
| 177 | STX046 | GGGGCUGGAUUU GUGAUGA N7-30 CUGA GC AUGAUGA N7-30 CUGA AGUCCAGCCUCAUAG |
| 178 | STX048 | GGGGUUGGGUCA AUGAUGA N7-30 CUGA AGAGAG GUGAUGA N7-30 CUGA GUCCCAGCCUCAUAG |
| 179 | STX050 | GGCCCCCUUCCU AUGAUGA N7-30 CUGA GCUCC GUGAGGA N7-30 CUGA GGAGAUGGGCCAUAG |
| 180 | STX052 | GGGGGGAUCACG GUGAUGA N7-30 CUGA CCUCAG GCCU CUGAGGG GUGAUGA N7-30 CUGA GUGAUCCCCCAUAG |
| 181 | STX054 | GGCUUUACAGUA GUGAUGA N7-30 CUGA ACCAC AUGAUUU N7-30 CUGA UGCUGUUUGCCAUAG |
| 182 | STX056 | GGGGUUGGAUCG AUGAUGA N7-30 CUGA  AGAGAG  GUGAUGA N7- 30 CUGA GGCCCAGCCUCAUAG |
| 183 | STX058 | GGAAAGAGGAAC AUGAGGA N7-30 UUGA  GUCACC  AACAUGU N7- 30 CUGA GUCCUCUGACCAUAG |
| 184 | STX060 | GGUGCCCUGCCU CUGAUGA N7-30 CUGA GAGUA AUGAUGA N7-30 CUGA UGGUGGCACACAUAG |
| 185 | STX065 | GGAUUGCUCACU AUGAUGA N7-30 CAGA AAUGUUUUUCCUU AUGUUGG N7-30 CUGA GUGAGCAUCUCAUAG |
| 186 | STX066 | GGAGGCUUUGCA GUGAUGA N7-30 CUGA  GUGCAAUCA CUGAUGU N7-30 CUGA GCAAUGCCUGCAUAG |
| 187 | STX068 | GGGGAUCACG GUGAUGA N7-30 CUGA CCUCAG GCCU CUGAGGG GUGAUGA N7-30 CUGA GUGAUCACAUAG |
| 188 | STX070 | GGAUUGUGGCCA AGGAUGA N7-30 CUGA UUUUAUGUGC UUCU GCU GUGAUGG N7-30 CUGA GGCCAAAUUGCAUAG |
| 189 | STX073 | GGCCUAUCUCUC AUGAUGA N7-30 CUGA  GCUGCU  GUGAUUU N7- 30 CUGA AGAAGAGAUGCAUAG |
| 190 | STX077 | GGAUACUUUCCU CUGAUGA N7-30 CUGA  GACACC  AUGAUGG N7- 30 CUGA GGAAGGGAGGCAUAG |
| 191 | STX078 | GGGCUGGCAUAU AUGAUGA N7-30 CCGA  CAGAUCGACU AUGUUGA N7-30 CUGA UAUGCCAGCUCAUAG |
| 192 | STX080 | GGCUGCUCACCA GUGAUGA N7-30 CUGA UCAAUGU GUGACUG N7-30 CUGA GCUGUGAGCCCAUAG |

| | | |
|---|---|---|
| *Note: the spaces inside the sequences in the table above are only intended to make the sequences easier to read and to identify the different structure sequences. It should therefore be understood that these spaces are absent from the actual sequences, and should not be considered as nicks.* | | |

### Conjugates

In some embodiments, the compound is covalently attached to at least one conjugate moiety.

In some embodiments, a conjugate moiety is covalently attached to the smartRNA, such as covalently attached at the 3'-end of the smartRNA and/or covalently attached at the 5'-end of the smartRNA, and/or covalently attached at any position between the 5' and 3' end.

The conjugate moiety may be independently selected from the group consisting of carbohydrates, fatty acids, cell surface receptor ligands, drug substances, hormones, lipophilic substances, polymers, proteins, peptides, toxins (e.g. bacterial toxins), vitamins, viral proteins (e.g. capsids) and combinations thereof.

In some embodiments, the conjugate moiety is independently selected from a fatty acid, such as a C10, C12, C14, C16, or C18 fatty acid, preferably a C16 fatty acid, more preferably palmitic acid.

The conjugate moiety may be attached to the compound via a conjugate linker, such as e.g. a cleavable conjugate linker. A cleavable conjugate linker may comprise 1 to 3 linker nucleotides.

In some embodiments, the compound is covalently attached to at least one conjugate moiety via a C6 amino linker.

In some embodiments, the compound is directly attached to at least one conjugate moiety.

In some embodiments, the compound is encapsulated in a lipid-based delivery vehicle, covalently linked to or encapsulated in a dendrimer, or conjugated to an aptamer.

In some embodiment, the compound is in the form of a pharmaceutically acceptable salt; preferably a sodium salt or a potassium salt.

### SmartRNP complex

A smartRNA according to the invention is capable of assembling with endogenous RNA binding protein (RNP) complex, thereby forming a smartRNP complex.

The complex's assembly is necessary for the introduction of a chemical modification on the target mRNA.

In some embodiments, a smartRNA assembles, when the smartRNA is introduced into a cell, with endogenous core proteins to form a smartRNP enzymatic complex that targets and modifies a target mRNA by modifying, *i.e*., by 2'-O-methylating, one or more adenosine nucleotides in the polyA-tail of said target mRNA. In some embodiments, the modifications mediated by the smartRNA increase the stability of the target mRNA by preventing deadenylation of the polyA tail of said mRNA, thus allowing additional rounds of translation, and thereby leading to increased levels of the encoded protein. This is particularly useful to increase the expression of a protein which is not sufficiently expressed in a cell.

In some embodiments, the target mRNA encodes a functional protein.

In some embodiments, the smartRNA is capable of forming a smartRNP by assembling with at least one of the endogenous proteins selected from the group consisting of:
- Fibrillarin, such as Fibrillarin as set forth in SEQ ID NO: 1;
- NOP56, such as NOP56 as set forth in SEQ ID NO: 2;
- NOP58, such as NOP58 as set forth in SEQ ID NO: 3; and
- SNU13, such as SNU13 as set forth in SEQ ID NO: 4.

In some embodiments, the smartRNA is capable of forming a smartRNP by assembling with all of the following endogenous proteins:
- Fibrillarin, such as Fibrillarin as set forth in SEQ ID NO: 1;
- NOP56, such as NOP56 as set forth in SEQ ID NO: 2;
- NOP58, such as NOP58 as set forth in SEQ ID NO: 3; and
- SNU13, such as SNU13 as set forth in SEQ ID NO: 4.

### Target mRNA modification

The abundance of a cytoplasmic mRNA is largely governed by two opposing processes: translation and degradation. The mRNA degradation process is initiated, while translation is ongoing, by deadenylation factors that gradually gain access to and shorten the protein-bound polyA-tail. Translation is brought to a halt when the polyA-tail is reduced to a length of ~30 nucleotides, and the mRNA is subjected to complete degradation.

An mRNA can be modified by introducing 2'-O-methylations *in vivo.* The full spectrum of the effects of said modification is still not understood, but it has been shown that it can interfere with efficiency of many enzymes that act on RNA- including ribonucleases.

Thus, 2'-O-methylated nucleotides may be also introduced into RNA oligonucleotides to increase their stability. The inventors of the present disclosure have found that the smartRNA described herein is capable of increasing 2'-O-methylation levels of the polyA-tail of the target mRNA molecule, hereby increasing the total amount of protein produced.

Thus, in some embodiments, the binding of the first and second antisense elements of the smartRNA to the first and second target sequences - wherein one of the first and second target sequence is a sequence comprised in the poly-A tail of the target mRNA- increases the 2'-O-methylation level of the polyA-tail of the target mRNA molecule.

In some embodiments, the binding of the first and second contiguous nucleotide sequence (of the D guide and D' guide sequences) to the first and second target nucleic acid sequence of the target mRNA, respectively, increases the 2'-O-methylation level of one or more adenosines of the polyA-tail of the target mRNA.

In some embodiments, the binding of the first and second contiguous nucleotide sequence - of the smartRNA contained in a smartRNP complex - to the first and second target nucleic acid sequence of the target mRNA, respectively, reduces deadenylation of the target mRNA.

Consequently, the binding of the first and second contiguous nucleotide sequence to the first and second target nucleic acid sequence of the target mRNA, respectively, increases the stability of the target mRNA.

Consequently, the binding of the first and second contiguous nucleotide sequence to the first and second target nucleic acid sequence of the target mRNA, respectively, reduces degradation of the target mRNA.

Consequently, the binding of the first and second contiguous nucleotide sequence to the first and second target nucleic acid sequence of the target mRNA, respectively, increases the expression of the protein encoded by the target mRNA.

In some embodiments, the binding of the first and second contiguous nucleotide sequence of the smartRNA contained in a smartRNP complex to the first and second target nucleic acid sequence of the target mRNA, respectively, increases the 2'-O-methylation level of one or more adenosines of the polyA-tail of the target mRNA.

Consequently, the 2'-O-methylation of one or more adenosines of the polyA-tail of the target mRNA increases the stability of the target mRNA.

Consequently, the increase of the 2'-O-methylation of one or more adenosines of the polyA-tail of the target mRNA reduces the deadenylation of the target mRNA.

Consequently, the increase of the 2'-O-methylation of one or more adenosines of the polyA-tail of the target mRNA increases the expression of the protein encoded by the target mRNA.

Consequently, the increase of the 2'-O-methylation of one or more adenosines of the polyA-tail of the target mRNA reduces the degradation of the target mRNA.

The compound is capable of increasing or upregulating the expression of a target mRNA, such as capable of increasing the expression of target mRNA by at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 550, 600, 650, 700, 750, 800, 850, 900, 950, or at least 1000%, compared to a control, wherein the control is a cell that has not been exposed to the compound.

The compound is capable of increasing or upregulating the expression of a target protein, such as capable of increasing the expression of target protein by at least 10, 15, 20, 25, 30, 35, 40, 45, 50, 55, 60, 65, 70, 75, 80, 85, 90, 95, 100, 110, 120, 130, 140, 150, 160, 170, 180, 190, or at least 200%, compared to a control, wherein the control is a cell that has not been exposed to the compound.

### Vector and Host Cell

In one aspect, the present disclosure is directed to a vector comprising a nucleotide sequence encoding the smartRNA as described herein.

In some embodiments, the vector is an adeno-associated virus (AAV) or lentiviral vector.

In another aspect, the present disclosure is directed to a host cell expressing the smartRNA as described herein.

### Composition

In another aspect, the present disclosure is directed to a composition comprising the smartRNA as described herein, the vector as described herein, or the host cell as described herein.

In some embodiments of the present disclosure, the composition further comprises a pharmaceutically acceptable diluent, solvent, carrier, salt and/or adjuvant. The pharmaceutically acceptable carriers can be either solid or liquid, such as aqueous. A solid carrier can be one or more excipients which may also act as diluents, flavoring agents, solubilizers, lubricants, suspending agents, binders, preservatives, wetting agents, tablet disintegrating agents, or an encapsulating material. An aqueous diluent or solvent can be e.g. phosphate buffered saline (PBS).

In some embodiments, multiple smartRNAs may be required to achieve a desired therapeutic effect. Thus, in some embodiments, the composition further comprises one or more additional smartRNAs as described herein.

The smartRNA can be formulated as a pharmaceutical composition and administered to a mammalian host, such as a human patient in a variety of forms adapted to the chosen route of administration. Thus, in some embodiments, the composition is a pharmaceutical composition.

### Medicament

In one aspect, the present disclosure is directed to a compound, vector, host cell, or composition as disclosed herein for use as a medicament.

In another aspect, the present disclosure is directed to a compound, vector, host cell, or composition as disclosed herein for use in preventing, treating and/or ameliorating a medical condition affecting a subject.

In another aspect, the present disclosure is directed to the use of a compound, vector, host cell, or composition as disclosed herein for the preparation of a medicament for preventing, treating and/or ameliorating a medical condition affecting a subject.

In another aspect, the present disclosure is directed to a method for preventing, treating and/or ameliorating a medical condition affecting a subject comprising administering a therapeutically or prophylactically effective amount of a compound, vector, host cell, or composition as disclosed herein.

In some embodiments, the medical condition is characterized by deficient protein expression of the protein encoded by the target mRNA.

In some embodiments, the medical condition is selected from the group consisting of haploinsufficiency disorders; cancer; immunological disorders; cardiovascular diseases; respiratory diseases; metabolic diseases; infectious diseases; digestive diseases; and neurological diseases, preferably a haploinsufficiency disorder.

In some embodiments, the haploinsufficiency disorder and/or neurological disease is selected from the group consisting of GRN frontotemporal dementia (GRN-FTD), palmoplantar keratoderma, punctate type 1A; progressive familial intrahepatic cholestasis type 3;low phospholipid associated cholelithiasis; adrenoleukodystrophy;X-linked cerebral adrenoleukodystrophy;isolated adrenal insufficiency;adrenomyeloneuropathy; telangiectasia, hereditary hemorrhagic, type 2;hereditary hemorrhagic telangiectasia; ADNP-related multiple congenital anomalies - intellectual disability - autism spectrum disorder; non-syndromic X-linked intellectual disability;FRAXE intellectual disability; AHDC1-related intellectual disability - obstructive sleep apnea - mild dysmorphism syndrome; parietal foramina 2; Brugada syndrome;long QT syndrome;complex neurodevelopmental disorder;catecholaminergic polymorphic ventricular tachycardia; KBG syndrome; syndromic intellectual disability; hypogonadotropic hypogonadism 1 with or without anosmia; X-linked syndromic intellectual disability;syndromic X-linked intellectual disability 5; classic or attenuated familial adenomatous polyposis;gastric adenocarcinoma and proximal polyposis of the stomach;APC-related attenuated familial adenomatous polyposis;classic familial adenomatous polyposis;attenuated familial adenomatous polyposis;familial adenomatous polyposis 1; hypercholesterolemia, autosomal dominant, type B;familial hypobetalipoproteinemia 1;homozygous familial hypercholesterolemia; Kennedy disease;androgen insensitivity syndrome; short stature, rhizomelic, with microcephaly, micrognathia, and developmental delay; X-linked complex neurodevelopmental disorder; Coffin-Siris syndrome;intellectual disability, autosomal dominant 14; Coffin-Siris syndrome;Coffin-Siris syndrome 1; Coffin-Siris syndrome;Coffin-Siris syndrome 6; X-linked chondrodysplasia punctata 1; developmental and epileptic encephalopathy;X-linked complex neurodevelopmental disorder;X-linked lissencephaly with abnormal genitalia; syndromic complex neurodevelopmental disorder;complex neurodevelopmental disorder; Bohring-Opitz syndrome; syndromic intellectual disability;severe feeding difficulties-failure to thrive-microcephaly due to ASXL3 deficiency syndrome; hereditary nonpolyposis colon cancer;hereditary breast carcinoma;familial ovarian cancer;ataxia telangiectasia; hemiplegic migraine-developmental and epileptic encephalopathy spectrum; Menkes disease;X-linked distal spinal muscular atrophy type 3; ATR-X-related syndrome;alpha thalassemia-X-linked intellectual disability syndrome; syndromic intellectual disability;autism spectrum disorder due to AUTS2 deficiency; diabetes insipidus, nephrogenic, X-linked; oligodontia-cancer predisposition syndrome; myofibrillar myopathy;dilated cardiomyopathy;myofibrillar myopathy 6;dilated cardiomyopathy 1HH; BAP1-related tumor predisposition syndrome; hereditary breast carcinoma;hereditary nonpolyposis colon cancer;familial ovarian cancer; severe motor and intellectual disabilities-sensorineural deafness-dystonia syndrome; Dias-Logan syndrome; microphthalmia, syndromic 2; pulmonary arterial hypertension juvenile polyposis syndrome;generalized juvenile polyposis/juvenile polyposis coli; pulmonary arterial hypertension;congenital heart disease;primary pulmonary hypertension;pulmonary hypertension, primary, 1 ;obsolete idiopathic and/or familial pulmonary arterial hypertension; syndromic intellectual disability; Fanconi anemia, complementation group S;breast-ovarian cancer, familial, susceptibility to, 1;hereditary breast ovarian cancer syndrome; Fanconi anemia complementation group D1;breast-ovarian cancer, familial, susceptibility to, 2;Fanconi anemia complementation group A;Fanconi anemia;hereditary breast ovarian cancer syndrome; Fanconi anemia complementation group J;familial ovarian cancer;hereditary breast carcinoma;Fanconi anemia;Fanconi anemia complementation group G; intellectual developmental disorder with dysmorphic facies and ptosis; X-linked syndromic intellectual disability;X-linked intellectual disability; isolated growth hormone deficiency type III;Bruton-type agammaglobulinemia; episodic ataxia type 2; cerebellar dysfunction with variable cognitive and behavioral abnormalities; X-linked syndromic intellectual disability;syndromic X-linked intellectual disability Najm type; hyper-IgM syndrome type 1; hyperparathyroidism 2 with jaw tumors;hyperparathyroidism 1;parathyroid gland carcinoma; CDH1-related diffuse gastric and lobular breast cancer syndrome;familial ovarian cancer;hereditary diffuse gastric adenocarcinoma; CDKL5 disorder;developmental and epileptic encephalopathy, 2; multiple endocrine neoplasia type 4;hereditary nonpolyposis colon cancer; Beckwith-Wiedemann syndrome; melanoma-pancreatic cancer syndrome;familial atypical multiple mole melanoma syndrome;melanoma, cutaneous malignant, susceptibility to, 2; complex neurodevelopmental disorder;developmental and epileptic encephalopathy 94; CHARGE syndrome; complex neurodevelopmental disorder; hereditary nonpolyposis colon cancer;hereditary breast carcinoma;familial ovarian cancer; choroideremia; isolated congenital megalocornea; complex neurodevelopmental disorder; Dent disease type 1; complex neurodevelopmental disorder; X-linked complex neurodevelopmental disorder;intellectual disability, X-linked, syndromic, Houge type; complex neurodevelopmental disorder;holoprosencephaly 12 with or without pancreatic agenesis; osteogenesis imperfecta type 1;osteogenesis imperfecta type 2;osteogenesis imperfecta type 3;combined osteogenesis imperfecta and Ehlers-Danlos syndrome 1;Ehlers-Danlos syndrome, arthrochalasia type;Caffey disease;Ehlers-Danlos syndrome, classic type, 1;osteogenesis imperfecta type 4; spondyloepiphyseal dysplasia, Stanescu type;achondrogenesis type II;platyspondylic dysplasia, Torrance type;spondylometaphyseal dysplasia;spondyloepiphyseal dysplasia congenita;Kniest dysplasia;spondyloperipheral dysplasia;Stickler syndrome type 1;spondyloepiphyseal dysplasia with metatarsal shortening; Ehlers-Danlos syndrome, vascular type;autosomal dominant Ehlers-Danlos syndrome, vascular type; Alport syndrome;X-linked Alport syndrome; Ehlers-Danlos syndrome, classic type;Ehlers-Danlos syndrome, classic type, 1; Rubinstein-Taybi syndrome;Rubinstein-Taybi syndrome due to CREBBP mutations; syndromic intellectual disability; hereditary nonpolyposis colon cancer;CTNNA1-related diffuse gastric and lobular breast cancer syndrome;hereditary gastric cancer; severe intellectual disability-progressive spastic diplegia syndrome; complex neurodevelopmental disorder;pseudohypoaldosteronism type 2E;neurodevelopmental disorder with or without autism or seizures; X-linked intellectual disability, Cabezas type; granulomatous disease, chronic, X-linked; Brooke-Spiegler syndrome;frontotemporal dementia and/or amyotrophic lateral sclerosis 8; lissencephaly spectrum disorders;lissencephaly type 1 due to doublecortin gene mutation; X-linked syndromic intellectual disability;intellectual disability, X-linked 102; DDX41-related hematologic malignancy predisposition syndrome; DICER1-related tumor predisposition;pleuropulmonary blastoma;obsolete DICER1 syndrome;rhabdomyosarcoma;goiter, multinodular 1, with or without Sertoli-Leydig cell tumors; non-syndromic X-linked intellectual disability; complex neurodevelopmental disorder; neurodevelopmental disorder with nonspecific brain abnormalities and with or without seizures; progressive muscular dystrophy;dilated cardiomyopathy 3B;Duchenne muscular dystrophy; hypertrophic cardiomyopathy;arrhythmogenic cardiomyopathy with wooly hair and keratoderma;dilated cardiomyopathy;arrhythmogenic right ventricular dysplasia 8;arrhythmogenic right ventricular cardiomyopathy;familial isolated arrhythmogenic right ventricular dysplasia; complex neurodevelopmental disorder;DYRK1A-related intellectual disability syndrome; hypotonia, ataxia, and delayed development syndrome; MEND syndrome;X-linked chondrodysplasia punctata 2; X-linked hypohidrotic ectodermal dysplasia; craniofrontonasal syndrome; mandibulofacial dysostosis-microcephaly syndrome; Kleefstra syndrome;Kleefstra syndrome 1; supravalvular aortic stenosis; telangiectasia, hereditary hemorrhagic, type 1;juvenile polyposis syndrome;hereditary hemorrhagic telangiectasia; Rubinstein-Taybi syndrome due to EP300 haploinsufficiency; craniosynostosis 4; thrombocytopenia 5; exostoses, multiple, type 1; exostoses, multiple, type 2; branchio-oto-renal syndrome;branchiootorenal syndrome 1; nonsyndromic genetic hearing loss;dilated cardiomyopathy 1J;autosomal dominant nonsyndromic hearing loss 10; hemophilia A;hemophilia; hemophilia B;thrombophilia, X-linked, due to factor 9 defect;hemophilia; Fanconi anemia complementation group B; Marfan syndrome;familial thoracic aortic aneurysm and aortic dissection;Shprintzen-Goldberg syndrome; Aarskog-Scott syndrome, X-linked; LADD syndrome; Hartsfield-Bixler-Demyer syndrome;osteoglophonic dwarfism;Pfeiffer syndrome type 1;hypogonadotropic hypogonadism 2 with or without anosmia; hereditary leiomyomatosis and renal cell cancer; obsolete Birt-Hogg-Dube syndrome;Birt-Hogg-Dube syndrome 1;Birt-Hogg-Dube syndrome; inherited ichthyosis; familial thoracic aortic aneurysm and aortic dissection;periventricular nodular heterotopia;heterotopia, periventricular, X-linked dominant; myofibrillar myopathy 5;dilated cardiomyopathy;myofibrillar myopathy; fragile X syndrome; anterior segment dysgenesis 3;Axenfeld-Rieger syndrome type 3; lymphedema-distichiasis syndrome; alveolar capillary dysplasia with misalignment of pulmonary veins; FOXG1 disorder; blepharophimosis, ptosis, and epicanthus inversus syndrome; intellectual disability-severe speech delay-mild dysmorphism syndrome;congenital heart disease; nystagmus 1, congenital, X-linked; exudative vitreoretinopathy;exudative vitreoretinopathy 1; GATA2 deficiency with susceptibility to MDS/AML;deafness-lymphedema-leukemia syndrome; hypoparathyroidism-deafness-renal disease syndrome; structural congenital heart disease, multiple types - GATA4;atrial septal defect 2; GATA6-related congenital heart disease with or without pancreatic agenesis or neonatal diabetes;pancreatic hypoplasia-diabetes-congenital heart disease syndrome; severe intellectual disability-poor language-strabismus-grimacing face-long fingers syndrome; GTP cyclohydrolase I deficiency;dopa-responsive dystonia;dystonia 5; brachydactyly type C; inborn glycerol kinase deficiency; Fabry disease; holoprosencephaly 9; Greig cephalopolysyndactyly syndrome; glomuvenous malformation; pseudopseudohypoparathyroidism; Simpson-Golabi-Behmel syndrome;Simpson-Golabi-Behmel syndrome type 1; complex neurodevelopmental disorder; complex neurodevelopmental disorder;intellectual disability, autosomal dominant 6; frontotemporal dementia and/or amyotrophic lateral sclerosis; ; linear skin defects with multiple congenital anomalies 1; Cornelia de Lange syndrome;Cornelia de Lange syndrome 5; intellectual disability, autosomal dominant 43; acute intermittent porphyria; monogenic diabetes;maturity-onset diabetes of the young;maturity-onset diabetes of the young type 3; renal cysts and diabetes syndrome; neurodevelopmental disorder-craniofacial dysmorphism-cardiac defect-hip dysplasia syndrome; synpolydactyly type 1; Lesch-Nyhan syndrome; mucopolysaccharidosis type 2;mucopolysaccharidosis type 2, severe form;mucopolysaccharidosis type 2, attenuated form; growth delay due to insulin-like growth factor I resistance; incontinentia pigmenti;IKBKG-related immunodeficiency with or without ectodermal dysplasia; non-syndromic X-linked intellectual disability;intellectual disability, X-linked 21; X-linked complex neurodevelopmental disorder;intellectual disability, X-linked 1; van der Woude syndrome 1; Alagille syndrome due to a JAG1 point mutation; Koolen-de Vries syndrome; syndromic intellectual disability;autosomal dominant intellectual disability-craniofacial anomalies-cardiac defects syndrome; RASopathy;KAT6B-related multiple congenital anomalies syndrome;blepharophimosis - intellectual disability syndrome, SBBYS type; Brugada syndrome;long QT syndrome;short QT syndrome;familial long QT syndrome;long QT syndrome 2; Jervell and Lange-Nielsen syndrome; hypertrophic cardiomyopathy;long QT syndrome;short QT syndrome;familial long QT syndrome;long QT syndrome 1; neonatal-onset developmental and epileptic encephalopathy;complex neurodevelopmental disorder;neonatal encephalopathy with non-epileptic myoclonus;seizures, benign familial neonatal, 1; X-linked syndromic intellectual disability; Kabuki syndrome 2; microcephaly with or without chorioretinopathy, lymphedema, or intellectual disability; gastrointestinal stromal tumor;piebaldism; Wiedemann-Steiner syndrome; complex neurodevelopmental disorder with motor features;dystonia 28, childhood-onset; syndromic intellectual disability; Kabuki syndrome 1; complex neurodevelopmental disorder;O'Donnell-Luria-Rodan syndrome; complex neurodevelopmental disorder; L1 syndrome;X-linked hydrocephalus with stenosis of the aqueduct of Sylvius; Danon disease; hypercholesterolemia, familial, 1;homozygous familial hypercholesterolemia; Buschke-Ollendorff syndrome; arrhythmogenic right ventricular cardiomyopathy;dilated cardiomyopathy;dilated cardiomyopathy 1A;Emery-Dreifuss muscular dystrophy 2, autosomal dominant;autosomal dominant Emery-Dreifuss muscular dystrophy; Emery-Dreifuss muscular dystrophy; nail-patella syndrome; X-linked intellectual disability;X-linked immunodeficiency with magnesium defect, Epstein-Barr virus infection and neoplasia; hereditary pheochromocytoma-paraganglioma;pheochromocytoma; complex neurodevelopmental disorder;intellectual disability, autosomal dominant 1; Rett syndrome;syndromic X-linked intellectual disability Lubs type; syndromic intellectual disability;congenital heart disease; complex neurodevelopmental disorder;intellectual disability, autosomal dominant 20; syndromic intellectual disability; multiple endocrine neoplasia type 1; X-linked Opitz G/BBB syndrome; Waardenburg syndrome type 2;Waardenburg syndrome type 2A; mismatch repair cancer syndrome 1;Lynch syndrome;hereditary breast carcinoma;Lynch syndrome 2;mismatch repair cancer syndrome; Currarino triad; mismatch repair cancer syndrome 1;Lynch syndrome;hereditary breast carcinoma;Lynch syndrome 1;mismatch repair cancer syndrome 2;mismatch repair cancer syndrome; mismatch repair cancer syndrome 1;Lynch syndrome;hereditary breast carcinoma;Lynch syndrome 5;mismatch repair cancer syndrome 3;mismatch repair cancer syndrome; Basilicata-Akhtar syndrome; tooth agenesis, selective, 1; X-linked myotubular myopathy; dilated cardiomyopathy;arrhythmogenic right ventricular cardiomyopathy;hypertrophic cardiomyopathy;familial hypertrophic cardiomyopathy;hypertrophic cardiomyopathy 4; Feingold syndrome type 1; syndromic intellectual disability;intellectual disability, autosomal dominant 39; syndromic intellectual disability; complex neurodevelopmental disorder; complex neurodevelopmental disorder; Norrie disease; X-linked complex neurodevelopmental disorder;X-linked intellectual disability, Cantagrel type; neurofibromatosis type 1 ;familial ovarian cancer; neurofibromatosis type 2; brain malformations with or without urinary tract defects;chromosome 1 p32-p31 deletion syndrome; Marshall-Smith syndrome;Malan overgrowth syndrome; Nance-Horan syndrome; Cornelia de Lange syndrome;Cornelia de Lange syndrome 1; dilated cardiomyopathy;NKX2.5-related congenital, conduction and myopathic heart disease; NOG-related symphalangism spectrum disorder;multiple synostoses syndrome 1; focal epilepsy; X-linked adrenal hypoplasia congenita; Bosch-Boonstra-Schaaf optic atrophy syndrome; autosomal dominant pseudohypoaldosteronism type 1;pseudohyperaldosteronism type 2; complex neurodevelopmental disorder; 46,XY sex reversal 3; complex neurodevelopmental disorder; Sotos syndrome; syndromic intellectual disability; CK syndrome;CHILD syndrome; complex neurodevelopmental disorder; NYX-related retinopathy;congenital stationary night blindness 1A; oculocerebrorenal syndrome; ciliopathy;orofaciodigital syndrome I; X-linked intellectual disability-cerebellar hypoplasia syndrome; ornithine carbamoyltransferase deficiency; syndromic microphthalmia type 5; classic lissencephaly; focal segmental glomerulosclerosis 7;renal coloboma syndrome; Waardenburg syndrome;Waardenburg syndrome type 1; PAX6-related ocular dysgenesis;aniridia 1; tooth agenesis, selective, 3; congenital anomalies of kidney and urinary tract syndrome with or without hearing loss, abnormal ears, or developmental delay; X-linked complex neurodevelopmental disorder; Leigh syndrome;pyruvate dehydrogenase deficiency; X-linked hypophosphatemic rickets;X-linked dominant hypophosphatemic rickets; complex neurodevelopmental disorder; Borjeson-Forssman-Lehmann syndrome; syndromic X-linked intellectual disability Siderius type; PHIP-related behavioral problems-intellectual disability-obesity-dysmorphic features syndrome; Axenfeld-Rieger syndrome; autosomal dominant polycystic kidney disease;autosomal recessive polycystic kidney disease;polycystic kidney disease 1; autosomal dominant polycystic kidney disease;polycystic kidney disease 2; Brugada syndrome 1;arrhythmogenic right ventricular cardiomyopathy;dilated cardiomyopathy;catecholaminergic polymorphic ventricular tachycardia;arrhythmogenic right ventricular dysplasia 9;familial isolated arrhythmogenic right ventricular dysplasia; Pelizeaus-Merzbacher spectrum disorder; Charcot-Marie-Tooth disease type 1A;hereditary neuropathy with liability to pressure palsies;Charcot-Marie-Tooth disease type 1 ;Charcot-Marie-Tooth disease type 1E; mismatch repair cancer syndrome 1;Lynch syndrome;hereditary breast carcinoma;Lynch syndrome 4;mismatch repair cancer syndrome;mismatch repair cancer syndrome 4; intellectual disability-microcephaly-strabismus-behavioral abnormalities syndrome; Treacher Collins syndrome 2; focal dermal hypoplasia; nonsyndromic genetic hearing loss;autosomal dominant nonsyndromic hearing loss 15; Renpenning syndrome; PRPS1 deficiency disorder;phosphoribosylpyrophosphate synthetase superactivity; neuroocular syndrome; infantile convulsions and choreoathetosis;self-limited familial infantile epilepsy; nevoid basal cell carcinoma syndrome; non-syndromic X-linked intellectual disability;autism, susceptibility to, X-linked 4; PTEN hamartoma tumor syndrome;multiple exostoses with spastic tetraparesis; brachydactyly type E2;mesomelic dysplasia; Noonan syndrome with multiple lentigines;cardiofaciocutaneous syndrome;Costello syndrome;Noonan syndrome;LEOPARD syndrome 2;LEOPARD syndrome 3;LEOPARD syndrome 1;Noonan syndrome 1;metachondromatosis; syndromic intellectual disability; complex neurodevelopmental disorder;PURA-related severe neonatal hypotonia-seizures-encephalopathy syndrome due to a point mutation; syndromic intellectual disability; early-onset parkinsonism-intellectual disability syndrome;intellectual disability, X-linked 72; Cornelia de Lange syndrome; hereditary breast carcinoma;Fanconi anemia complementation group O;familial ovarian cancer;breast-ovarian cancer, familial, susceptibility to, 3; hereditary breast carcinoma;breast-ovarian cancer, familial, susceptibility to, 4;familial ovarian cancer; Smith-Magenis syndrome; Noonan syndrome;capillary malformation-arteriovenous malformation 1; retinoblastoma;hereditary retinoblastoma; multiple endocrine neoplasia type 2A;multiple endocrine neoplasia type 2B;familial medullary thyroid carcinoma;Hirschsprung disease, susceptibility to, 1; RP2-related retinopathy;retinitis pigmentosa 2; Diamond-Blackfan anemia 4; Diamond-Blackfan anemia;Diamond-Blackfan anemia 1; Diamond-Blackfan anemia;Diamond-Blackfan anemia 3; Diamond-Blackfan anemia 10; Coffin-Lowry syndrome; X-linked retinoschisis; hereditary thrombocytopenia and hematologic cancer predisposition syndrome;hereditary thrombocytopenia and hematological cancer predisposition syndrome associated with RUNX1;microcephaly, corpus callosum dysgenesis, and cleft lip/palate; Duane-radial ray syndrome; SATB2 associated disorder; generalized epilepsy with febrile seizures plus;Dravet syndrome;developmental and epileptic encephalopathy;familial hemiplegic migraine;generalized epilepsy with febrile seizures plus, type 2;developmental and epileptic encephalopathy, 6; complex neurodevelopmental disorder; Brugada syndrome;arrhythmogenic right ventricular cardiomyopathy;dilated cardiomyopathy;familial long QT syndrome;catecholaminergic polymorphic ventricular tachycardia;short QT syndrome;Brugada syndrome 1;long QT syndrome 3;long QT syndrome; hereditary pheochromocytoma-paraganglioma;paraganglioma;paragangliomas 2; hereditary pheochromocytoma-paraganglioma;mitochondrial disease;paraganglioma;pheochromocytoma;paragangliomas 4; hereditary pheochromocytoma-paraganglioma;mitochondrial disease;paragangliomas 3;paraganglioma; hereditary pheochromocytoma-paraganglioma;mitochondrial disease;pheochromocytoma;paraganglioma;paragangliomas 1; complex neurodevelopmental disorder;Schinzel-Giedion syndrome;intellectual disability, autosomal dominant 29; schizophrenia; SETD2-related neurodevelopmental disorder without or with macrocephaly/overgrowth;SETD2-related microcephaly-severe intellectual disability-multiple congenital anomalies syndrome;complex neurodevelopmental disorder; syndromic complex neurodevelopmental disorder;intellectual disability-facial dysmorphism syndrome due to SETD5 haploinsufficiency; SF3B4-related acrofacial dysostosis;Nager acrofacial dysostosis; myoclonic dystonia 11; X-linked lymphoproliferative disease due to SH2D1A deficiency; complex neurodevelopmental disorder; Phelan-McDermid syndrome; holoprosencephaly 3; Leri-Weill dyschondrosteosis; SIN3A-related intellectual disability syndrome;chromosome 15q24 deletion syndrome; holoprosencephaly 2; Allan-Herndon-Dudley syndrome; GLUT1 deficiency syndrome;childhood onset GLUT1 deficiency syndrome 2; X-linked complex neurodevelopmental disorder;SLC35A2-congenital disorder of glycosylation; complex neurodevelopmental disorder; creatine transporter deficiency;cerebral creatine deficiency syndrome; Christianson syndrome; aneurysm-osteoarthritis syndrome;familial thoracic aortic aneurysm and aortic dissection;Loeys-Dietz syndrome; juvenile polyposis/hereditary hemorrhagic telangiectasia syndrome;Myhre syndrome;pulmonary arterial hypertension juvenile polyposis syndrome;generalized juvenile polyposis/juvenile polyposis coli; rhabdoid tumor predisposition syndrome 1;Coffin-Siris syndrome;familial rhabdoid tumor; X-linked complex neurodevelopmental disorder;Cornelia de Lange syndrome 2; syndromic X-linked intellectual disability Snyder type; ZTTK syndrome; Waardenburg syndrome type 4C; intellectual disability, autosomal dominant 27; anophthalmia/microphthalmia-esophageal atresia syndrome; Lamb-Shaffer syndrome; campomelic dysplasia;isolated Pierre-Robin syndrome;Cooks syndrome; hereditary spastic paraplegia 4; hereditary chronic pancreatitis; Legius syndrome; complex neurodevelopmental disorder; 46,XY sex reversal 1; X-linked syndromic intellectual disability; familial ovarian cancer;Peutz-Jeghers syndrome; recessive X-linked ichthyosis; developmental and epileptic encephalopathy;developmental and epileptic encephalopathy, 4; X-linked complex neurodevelopmental disorder; SYNCRIP-related neurodevelopmental disorder; complex neurodevelopmental disorder; syndromic intellectual disability; complex neurodevelopmental disorder;intellectual disability, autosomal dominant 41; complex neurodevelopmental disorder; cleft palate with or without ankyloglossia, X-linked; ulnar-mammary syndrome; pulmonary arterial hypertension;coxopodopatellar syndrome; Holt-Oram syndrome; TCF12-related craniosynostosis; developmental delay with variable intellectual impairment and behavioral abnormalities;complex neurodevelopmental disorder; Pitt-Hopkins syndrome; Treacher-Collins syndrome;Treacher Collins syndrome 1; Char syndrome; Loeys-Dietz syndrome;multiple self-healing squamous epithelioma;familial thoracic aortic aneurysm and aortic dissection;Loeys-Dietz syndrome 1; holoprosencephaly 4; deafness dystonia syndrome; hereditary pheochromocytoma-paraganglioma;pheochromocytoma; complex neurodevelopmental disorder; Li-Fraumeni syndrome; spondyloepiphyseal dysplasia tarda, X-linked; syndromic intellectual disability;micrognathia-recurrent infections-behavioral abnormalities-mild intellectual disability syndrome; complex neurodevelopmental disorder;Clark-Baraitser syndrome; trichorhinophalangeal syndrome type I; tuberous sclerosis;tuberous sclerosis 1; tuberous sclerosis;tuberous sclerosis 2; dilated cardiomyopathy;hypertrophic cardiomyopathy;arrhythmogenic right ventricular cardiomyopathy;tibial muscular dystrophy;myopathy, myofibrillar, 9, with early respiratory failure;TTN-related myopathy;dilated cardiomyopathy 1G; Saethre-Chotzen syndrome;Sweeney-Cox syndrome;TWIST1-related craniosynostosis; syndromic X-linked intellectual disability Nascimento type; Angelman syndrome; X-linked complex neurodevelopmental disorder;syndromic X-linked intellectual disability 14; Hao-Fountain syndrome; X-linked syndromic intellectual disability;intellectual disability, X-linked 99; von Hippel-Lindau disease; DeSanto-Shinawi syndrome;DeSanto-Shinawi syndrome due to WAC point mutation; syndromic intellectual disability;complex neurodevelopmental disorder; Skraban-Deardorff syndrome; X-linked complex neurodevelopmental disorder;neurodegeneration with brain iron accumulation 5; Wilms tumor 1;Denys-Drash syndrome; X-linked lymphoproliferative disease due to XIAP deficiency; X inactivation, familial skewed, 1; complex neurodevelopmental disorder;intellectual disability, autosomal dominant 22; X-linked syndromic intellectual disability;Wieacker-Wolff syndrome; syndromic X-linked intellectual disability Raymond type; Mowat-Wilson syndrome; holoprosencephaly; heterotaxy, visceral, 1, X-linked; syndromic complex neurodevelopmental disorder; and weiss-kruszka syndrome.

In some embodiments, the haploinsufficiency disorder and/or neurological disease is caused by haploinsufficiency of a gene selected from the group consisting of:*AAGAB; ABCB4; ABCD1; ACVRL1; ADNP; AFF2; AHDC1; ALX4; ANK2; ANKRD11; ANKRD17; ANOS1; AP1S2; APC; APOB; AR; ARCN1; ARHGEF9; ARID1A; ARID1B; ARID2; ARSL; ARX; ASH1L; ASXL1; ASXL3; ATM; ATP1A2; ATP7A; ATRX; AUTS2; AVPR2; AXIN2; BAG3; BAP1; BARD1; BCAP31; BCL11A; BCOR; BMPR1A; BMPR2; BPTF; BRCA1; BRCA2; BRIP1; BRPF1; BRWD3; BTK; CACNA1A; CAMTA1; CASK; CD40LG; CDC73; CDH1; CDKL5; CDKN1B; CDKN1C; CDKN2A; CHD2; CHD7; CHD8; CHEK2; CHM; CHRDL1; CIC; CLCN5; CLTC; CNKSR2; CNOT1; COL1A1; COL2A1; COL3A1; COL4A5; COL5A1; CREBBP; CTCF; CTNNA1; CTNNB1; CUL3; CUL4B; CYBB; CYLD; DCX; DDX3X; DDX41; DICER1; DLG3; DLG4; DLL1; DMD; DSP; DYRK1A; EBF3; EBP; EDA; EFNB1; EFTUD2; EHMT1; ELN; ENG; EP300; ERF; ETV6; EXT1; EXT2; EYA1; EYA4; F8; F9; FANCB; FBN1; FGD1; FGF10; FGFR1; FH; FLCN; FLG; FLNA; FLNC; FMR1; FOXC1; FOXC2; FOXF1; FOXG1; FOXL2; FOXP1; FRMD7; FZD4; GATA2; GATA3; GATA4; GATA6; GATAD2B; GCH1; GDF5; GK; GLA; GLI2; GLI3; GLMN; GNAS; GPC3; GRIN2A; GRIN2B; GRN; HCCS; HDAC8; HIVEP2; HMBS; HNF1A; HNF1B; HNRNPK; HOXD13; HPRT1; IDS; IGF1R; IKBKG; IL1RAPL1; IQSEC2; IRF6; JAG1; KANSL 1; KAT6A; KAT6B; KCNH2; KCNQ1; KCNQ2; KDM5C; KDM6A; KIF11; KIT; KMT2A; KMT2B; KMT2C; KMT2D; KMT2E; KMT5B; L1CAM; LAMP2; LDLR; LEMD3; LMNA; LMX1B; MAGT1; MAX; MBD5; MECP2; MED13L; MEF2C; MEIS2; MEN1; MID1; MITF; MLH1; MNX1; MSH2; MSH6; MSL3; MSX1; MTM1; MYBPC3; MYCN; MYT1L; NAA15; NBEA; NCKAP1; NDP; NEXMIF; NF1; NF2; NFIA; NFIX; NHS; NIPBL; NKX2-5; NOG; NPRL3; NR081; NR2F1; NR3C2; NR4A2; NR5A1; NRXN1; NSD1; NSD2; NSDHL; NUS1; NYX; OCRL; OFD1; OPHN1; OTC; OTX2; PAFAH1B1; PAX2; PAX3; PAX6; PAX9; PBX1; PCDH19; PDHA1; PHEX; PHF21A; PHF6; PHF8; PHIP; PITX2; PKD1; PKD2; PKP2; PLP1; PMP22; PMS2; POGZ; POLR1D; PORCN; POU4F3; PQBP1; PRPS1; PRR12; PRRT2; PTCH1; PTCHD1; PTEN; PTHLH; PTPN11; PUF60; PURA; QRICH1; RAB39B; RAD21; RAD51C; RAD51D; RAI1; RASA1; RB1; RET; RP2; RPS17; RPS19; RPS24; RPS26; RPS6KA3; RS1; RUNX1; SALL4; SATB2; SCN1A; SCN2A; SCN5A; SDHAF2; SDHB; SDHC; SDHD; SETBP1; SETD1A; SETD2; SETD5; SF3B4; SGCE; SH2D1A; SHANK2; SHANK3; SHH; SHOX; SIN3A; SIX3; SLC16A2; SLC2A1; SLC35A2; SLC6A1; SLC6A8; SLC9A6; SMAD3; SMAD4; SMARCB1; SMC1A; SMS; SON; SOX10; SOX11; SOX2; SOX5; SOX9; SPAST; SPINK1; SPRED1; SRRM2; SRY; STAG2; STK11; STS; STXBP1; SYN1; SYNCRIP; SYNGAP1; TAOK1; TBL1XR1; TBR1; TBX22; TBX3; TBX4; TBX5; TCF12; TCF20; TCF4; TCOF1; TFAP2B; TGFBR1; TGIF1; TIMM8A; TMEM127; TNRC6B; TP53; TRAPPC2; TRIO; TRIP12; TRPS1; TSC1; TSC2; TTN; TWIST1; UBE2A; UBE3A; UPF3B; USP7; USP9X; VHL; WAC; WDFY3; WDR26; WDR45; WT1; XIAP; XIST; ZBTB18; ZC4H2; ZDHHC9; ZEB2; ZIC2; ZIC3; ZMYND11;* and *ZNF462.*

In some embodiments, preventing, treating and/or ameliorating a medical condition affecting a subject further comprises the administration of one or more additional therapeutic agents, such as agonist therapeutic agents.

In some embodiments, preventing, treating and/or ameliorating a medical condition affecting a subject comprises administering a therapeutically or prophylactically effective amount of the compound, vector, host cell, or composition as described herein to the subject affected by the medical condition.

### Methods and use of smartRNA

The smartRNA described herein is capable of promoting a chemical modification, such as 2'-O-methylation, of one or more adenosines of the polyA-tail of the target mRNA molecule. The chemical modification can interfere with efficiency of many enzymes that act on RNA- including ribonucleases, thereby increasing target mRNA stability.

Thus, in another aspect, the present disclosure concerns a method of modulating the stability of a target mRNA, such as increasing the stability of a target mRNA, the method comprising the step of contacting the mRNA with a compound, a vector, a host cell, or a composition from the present disclosure.

In another aspect, the present disclosure concerns a method of increasing or upregulating the expression of a target mRNA, the method comprising the step of contacting the mRNA with a compound, a vector, a host cell, or a composition from the present disclosure.

In some embodiments, the expression of the target mRNA is increased or upregulated by at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 550, 600, 650, 700, 750, 800, 850, 900, 950, or at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 1000%, compared to a control, such as compared to a cell that has not been exposed to the compound.

In another aspect, the present disclosure concerns a method of increasing or upregulating the expression of a target protein expressed by a target mRNA, the method comprising the step of contacting the mRNA with a compound, a vector, a host cell, or a composition from the present disclosure.

In some embodiments, the expression of the target protein is increased or upregulated by at least 10, 15, 20, 25, 30, 35, 40, 45, 50, 55, 60, 65, 70, 75, 80, 85, 90, 95, 100, 110, 120, 130, 140, 150, 160, 170, 180, 190, or at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 200%, compared to a control.

In some embodiments, the target mRNA and/or the target protein originates from the transcription of one or more of the following genes: *AAGAB; ABCB4; ABCD1; ACVRL1; ADNP; AFF2; AHDC1; ALX4; ANK2; ANKRD11; ANKRD17; ANOS1; AP1S2; APC; APOB; AR; ARCN1; ARHGEF9; ARID1A; ARID1B; ARID2; ARSL; ARX; ASH1L; ASXL1; ASXL3; ATM; ATP1A2; ATP7A; ATRX; AUTS2; AVPR2; AXIN2; BAG3; BAP1; BARD1; BCAP31; BCL11A; BCOR; BMPR1A; BMPR2; BPTF; BRCA1; BRCA2; BRIP1; BRPF1; BRWD3; BTK; CACNA1A; CAMTA1; CASK; CD40LG; CDC73; CDH1; CDKL5; CDKN1B; CDKN1C; CDKN2A; CHD2; CHD7; CHD8; CHEK2; CHM; CHRDL1; CIC; CLCN5; CLTC; CNKSR2; CNOT1; COL1A1; COL2A1; COL3A1; COL4A5; COL5A1; CREBBP; CTCF; CTNNA1; CTNNB1; CUL3; CUL4B; CYBB; CYLD; DCX; DDX3X; DDX41; DICER1; DLG3; DLG4; DLL1; DMD; DSP; DYRK1A; EBF3; EBP; EDA; EFNB1; EFTUD2; EHMT1; ELN; ENG; EP300; ERF; ETV6; EXT1; EXT2; EYA1; EYA4; F8; F9; FANCB; FBN1; FGD1; FGF10; FGFR1; FH; FLCN; FLG; FLNA; FLNC; FMR1; FOXC1; FOXC2; FOXF1; FOXG1; FOXL2; FOXP1; FRMD7; FZD4; GATA2; GATA3; GATA4; GATA6; GATAD2B; GCH1; GDF5; GK; GLA; GLI2; GLI3; GLMN; GNAS; GPC3; GRIN2A; GRIN2B; GRN; HCCS; HDAC8; HIVEP2; HMBS; HNF1A; HNF1B; HNRNPK; HOXD13; HPRT1; IDS; IGF1R; IKBKG; IL1RAPL1; IQSEC2; IRF6; JAG1; KANSL1; KAT6A; KAT6B; KCNH2; KCNQ1; KCNQ2; KDM5C; KDM6A; KIF11; KIT; KMT2A; KMT2B; KMT2C; KMT2D; KMT2E; KMT5B; L1CAM; LAMP2; LDLR; LEMD3; LMNA; LMX1B; MAGT1; MAX; MBD5; MECP2; MED13L; MEF2C; MEIS2; MEN1; MID1; MITF; MLH1; MNX1; MSH2; MSH6; MSL3; MSX1; MTM1; MYBPC3; MYCN; MYT1L; NAA15; NBEA; NCKAP1; NDP; NEXMIF; NF1; NF2; NFIA; NFIX; NHS; NIPBL; NKX2-5; NOG; NPRL3; NR081; NR2F1; NR3C2; NR4A2; NR5A1; NRXN1; NSD1; NSD2; NSDHL; NUS1; NYX; OCRL; OFD1; OPHN1; OTC; OTX2; PAFAH1B1; PAX2; PAX3; PAX6; PAX9; PBX1; PCDH19; PDHA1; PHEX; PHF21A; PHF6; PHF8; PHIP; PITX2; PKD1; PKD2; PKP2; PLP1; PMP22; PMS2; POGZ; POLR1D; PORCN; POU4F3; PQBP1; PRPS1; PRR12; PRRT2; PTCH1; PTCHD1; PTEN; PTHLH; PTPN11; PUF60; PURA; QRICH1; RAB39B; RAD21; RAD51C; RAD51D; RAI1; RASA 1; RB1; RET; RP2; RPS17; RPS19; RPS24; RPS26; RPS6KA3; RS1; RUNX1; SALL4; SATB2; SCN1A; SCN2A; SCN5A; SDHAF2; SDHB; SDHC; SDHD; SETBP1; SETD1A; SETD2; SETD5; SF3B4; SGCE; SH2D1A; SHANK2; SHANK3; SHH; SHOX; SIN3A; SIX3; SLC16A2; SLC2A1; SLC35A2; SLC6A1; SLC6A8; SLC9A6; SMAD3; SMAD4; SMARCB1; SMC1A; SMS; SON; SOX10; SOX11; SOX2; SOX5; SOX9; SPAST; SPINK1; SPRED1; SRRM2; SRY; STAG2; STK11; STS; STXBP1; SYN1; SYNCRIP; SYNGAP1; TAOK1; TBL1XR1; TBR1; TBX22; TBX3; TBX4; TBX5; TCF12; TCF20; TCF4; TCOF1; TFAP2B; TGFBR1; TGIF1; TIMM8A; TMEM127; TNRC6B; TP53; TRAPPC2; TRIO; TRIP12; TRPS1; TSC1; TSC2; TTN; TWIST1; UBE2A; UBE3A; UPF3B; USP7; USP9X; VHL; WAC; WDFY3; WDR26; WDR45; WT1; XIAP; XIST; ZBTB18; ZC4H2; ZDHHC9; ZEB2; ZIC2; ZIC3; ZMYND11;* and/or *ZNF462.*

In some embodiments, the method is an *in vitro* method.

In some embodiments, the method is an *in vivo* method.

### Kit

In one aspect, the present disclosure is directed to a kit comprising a compound, vector, host cell, or composition as described herein.

In another aspect, the present disclosure is directed to a kit comprising a compound, vector, host cell, or composition as described herein, and instructions for use.

In some embodiments, the kit further comprises one or more additional therapeutic agents, such as agonist therapeutic agents.

### EXAMPLES

### Example 1: Singular 2'-O-methylations in polyA-tail halts deadenylation and increase protein expression

**Aim:** To address whether 2'-O-methylation in the polyA-tail of a target mRNA delays the degradation of the mRNA and allows additional translation and protein production.

**Materials and Methods:** four artificial RNAs were constructed consisting of a body of 20 nucleotides (SEQ ID NO: 32 - GGAAAUUGCAUCGCAUUGGG) at the 5'-end, followed by a polyA-tail at the 3'-end with an exact length of 60 adenosines with (1) only regular adenosines (pA₆₀ no methyl) (SEQ ID NO: 23), (2) 2'-O-methyl at position 60 (pA₆₀ methyl @ 60) (SEQ ID NO: 24), (3) 2'-O-methyl at position 30 (pA₆₀ methyl @ 30) (SEQ ID NO: 25), and (4) 2'-O-methyl at a "random" position (pA₆₀ methyl @ random) (SEQ ID NO: 26-31). The former three pA₆₀-RNAs were synthesized by the phosphoramidite method with 2'-O-silyl protection, whereas the latter was produced as follows: a 20mer body RNA (GGAAAUUGCAUCGCAUUGGG) (SEQ ID NO: 32) was mixed with 10mer RNAs A₁₀ and A₁₀@6 (where @6 indicates a 2'-O-methylated adenosine at the sixth position) were mixed in a 1:5:1 molar ratio and annealed to a DNA-scaffold followed by ligation using T4 RNA ligase 2. Subsequently, 80mers were gel-purified. Thus, the produced RNAs contain pA₆₀-tails with on average one 2'-O-methylated adenosine equally distributed between positions 6, 16, 26, 36, 46 and 56.

The purified polyA-tailed 80mer RNAs were then ligated (using a DNA-scaffold and T4 RNA ligase 2) to a 20mer RNA oligo with a Cy5-group at the 5'-end (Cy5-GUCCUUUCCUAAUAAAAUGA) (SEQ ID NO: 33) thus producing the 100mer Cy5-labelled and pA₆₀-containing RNA substrates to be used in *in vitro* deadenylation assays. An otherwise identical Cy3-labelled version with the non-methylated pA₆₀-tail was produced and used as a control substrate in all reactions (SEQ ID NO: 22).

Deadenylation assays were performed in cell extracts essentially as described in Torabi *et al.,* 2021, by separately adding the four Cy5-substrate RNAs and Cy3-control RNA with HeLa S100 extracts followed by incubation at 37°C. Samples of identical volumes were taken out after 0, 7.5, 15, 30, 60 and 120 minutes of incubation, placed on ice and the reaction was immediately stopped by addition of Trizol reagent. RNAs were purified according to the Trizol protocol, and subsequently separated on polyacrylamide gels followed by visualization and quantification using a Typhoon imager.

**Results:** Introduction of a 2'-O-methylation in the polyA-tail at any of the mentioned positions slows down deadenylation (**Figure 2**).

When the same polyA-tailed 80mer RNAs were appended to m7G-capped eGFP-encoding mRNA bodies and co-transfected with a m7G-capped mKATE2 with a non-methylated polyA-tail into HeLa cells for 24 hours, increased eGFP protein expression was measured for @60 and @random, but not for @30 (**Figure 3**). Close inspection of the quantified data in **Figure 2** revealed that the levels of RNA with a polyA-tail longer than 30 adenosines were approximately the same over the profiled period for unmodified and @30, while @60 and @random had elevated and similar amounts of the longer tails (**Figure 2**).

The eGFP RNA body was *in vitro* transcribed using T7 polymerase and gel purified followed by m7G capping using the Vaccinia Capping System. The artificial polyA-tails were ligated to the eGFP RNA body using the same method as described for the Cy5-labelled RNA above. mKATE2 including a template-encoded regular non-methylated pA₆₀-tail was similarly *in vitro* transcribed, capped and purified.

eGFP and mKATE2 expression was measured in individual cells by flow cytometry and the relative expression is depicted in Figure 3.

**Conclusion:** It was concluded that mRNAs with 2'-O-methyl in the polyA-tail downstream of nucleotide 30 lead to increased protein production. It is known in the art that translation is inhibited if the polyA-tail is less than approximately 30 nucleotides and the data presented in Figure 2 and Figure 3 is consistent with this, since translation of @30 is similar to the unmodified mRNA.

### Example 2 transfected smartRNA assembles with endogenous protein factors

**Aim:** to address whether smartRNAs can be taken up by human cells and recruit their protein partners to form enzymatically active smartRNPs targeting and modifying an mRNA of choice.

**Materials and Methods:** an 80-base smartRNA (S1/smart_S1) (SEQ ID NO: 6) was designed based on common C/D-box snoRNA features and an optimized scaffold design. Various mutants of S1 were also constructed to assess the importance of different structural elements of S1: (1) S1_CC'mut = smart_S1 with C- and C'-box mutated (SEQ ID NO: 15) and (2) S1_Dmut = smart_S1 with D-box mutated (SEQ ID NO: 16).

smart_S1 and mutants were produced by T7 transcription of PCR-generated DNA templates followed by gel purification. The smartRNA constructs were transfected into HEK293 Flp-In T-Rex cells that either expressed a 3xFLAG tagged version of NOP56 (*NOP56-3xFLAG*) or not (*EMPTY*)*.* The same quantity of smartRNA variants were transfected. 6×10⁶ cells were seeded in P15 cell culture dishes in DMEM containing 10% FBS. 12h before transfection, expression from the Flp-In locus was induced by addition of 100ng/ml tetracycline. The smartRNAs constructs were heated to 95C for 2 min followed by snap-cooling at 4C and subsequent transfection into HEK Flp-In T-Rex cells at a concentration of 10nM using Lipofectamine2000 according to the manufacturer's protocol. The media was replaced with DMEM containing 10% FBS, 1% penicillin/streptavidin and 100ng/ml tetracycline 6h after transfection.

24 hours after transfection, cells were lysed, and the lysates were subjected to immunoprecipitation using M2 anti-FLAG antibody with and without addition of ribosomal RNA from *E*. *coli* as competitor. The lysates (INPUT) and immunoprecipitates (IP) were subjected to northern blotting and probed with probes directed towards the smart_S1 RNA and derivatives. The endogenous box C/D snoRNA snoRD22 (SEQ ID NO: 5) was probed as a positive control.

**Results and Conclusion:** Pulldown of the smartRNA with NOP56 is indicative of a fully assembled smartRNP complex. Thus, the smartRNA interacts with intended RNP protein partners, and this interaction is dependent on signature elements of the smartRNA, since point mutations in the C/C'- or D-box elements abolished the interaction completely (**Figure 4**).

### Example 3 smartRNAs with homo-polymeric U-stretches and long opposing AS elements are stable when expressed from plasmids transfected into cells and assemble with endogenous protein factors.

**Aim:** To address whether a smartRNA that is configured with homo-polymeric AS elements is still able to form a functional smartRNP.

**Materials and Methods:** an *in vivo* expression system was used to simultaneously test the design for a massive parallel screening procedure. The expression system relies on a vector encoding the open reading frame for fluorescent protein mKATE2 interrupted by an artificial intronic sequence that includes an artificial smartRNA. Thus, after transcription, the exons are spliced together thereby producing an mRNA encoding mKATE2, inside the cells. At the same time, splicing allows the liberation and processing of the smartRNA.

Two smartRNAs were tested (i) with 10-nucleotides homo-polymeric pyrimidine (pU₁₀ or pU₁₀) D'-guide AS element (SEQ ID NO: 7) or the D-guide AS element (SEQ ID NO: 8) or a mixed nucleotides (pR₁₀) D'-guide AS element (SEQ ID NO: 13) or D-guide AS element (SEQ ID NO: 14) with an opposing stretch, comprising the AS element, of 12 nucleotides, and (ii) with 10-nucleotides homo-polymeric uridine AS element at either the D- (SEQ ID NO: 10) or the D'-position (SEQ ID NO: 9) with an opposing stretch, comprising the AS element, of 23 nucleotides. Thus, while the sequence stretch comprising the AS element is 23 nucleotides, the actual AS-element is 20 nucleotides. Similarly, the 10 nucleotide AS-element is also in a 13 nucleotide stretch, as three nucleotides are not part of the AS-element, but part of sequence that constitutes the D- and D'-guide.

**Results:** The experiment, which is illustrated in **Figure 5****,** demonstrated that smartRNAs with pU₁₀ (or pC₁₀ which is simply used here as a control) were expressed at respectable levels in HeLa Kyoto cells albeit at lower levels than the pR₁₀, both when they were placed at the D- or the D'-position. Moreover, it was demonstrated that for a pU₁₀ smartRNA, the opposing AS element can be up to 20 nucleotides long and still allow stable expression.

The RNA immunoprecipitation (RIP) experiment shown in **Figure 6** further illustrated that the modified smartRNAs interacts with the endogenous C/D-box snoRNP core proteins creating the smartRNP at levels comparable to a 'normal' smartRNA (the S1/smartRNA_S1).

**Conclusion:** The experiment shows that a smartRNA configured with homo-polymeric AS elements to fit to the concept is still able to form a functional smartRNP. Furthermore, this indicates smartRNA AS element opposing the pU₁₀ can be up to 20 nucleotides long without compromising the capability of forming smartRNP complex.

### Example 4. smartRNAs targeting SCN1A increase eGFP protein levels in cells containing SCN1A target sequence

### Aim:

To show that smartRNAs can increase protein levels in cells containing the relevant target sequence.

### Materials and methods:

A stable HEK293 Flp-In T-Rex reporter cell line was generated that expresses an mRNA consisting of the eGFP401 open reading frame fused to the full human SCN1A 3'UTR and polyA sequence (Figure 7). Downstream of the SCN1A polyA signal there is a backup β-globin polyAsignal. eGFP401 encodes standard eGFP, but with codons that render the mRNA more unstable than the typically used version (2.3-2.6-fold difference in half-lives; PMID: 32275854 and personal communication with the authors). The reporter gene, which is driven by the chicken β-actin promoter, is inserted into the AAVS1 safe harbor locus (PMID: 33293588) by use of CRISPR/Cas9.

The cells were checked for correct expression of the construct using nanopore sequences (MinION, Nanopore).

SmartRNAs targeting the human SCN1A 3'UTR and the SCN1A polyAsequence were designed (SEQ ID: 85 to 100, or Tables 4a; SEQ ID NO: 117-132 or Table 4b, and SEQ ID NO: 133 to 169, or Table 4c). The smartRNAs were generated by in vitro transcription (HighYield T7 RNA synthesis kit, Jena), PAGE analyzed and gel-purified (ZR small-RNA PAGE Recovery kit, Zymo), treated with RppH enzyme (NEB) to remove pyrophosphate, and trizol extracted (direct-zol kit, Zymo).

The smartRNAs were transfected into the cells in forward transfection using RNAiMAX (Thermo). Cells were seeded in DMEM containing 10% FBS in 24-well plates at 50.000 cells/well, left to settle for 24 hours, transfected using 1,5 uL RNAiMAX (pre-incubated with the smartRNA) per 500 uL medium. The cells were then incubated for 72 hours before harvest.

Cells were harvested, fixed by fixation buffer (Biotium) and stained by live/dead stain (405 nm excitation, Thermo) according to manufacturer's recommendations. The cells were analyzed on a flow cytometer (Attune NxT 4, Thermo), gated on live cells and the GFP MFI was calculated for a minimum of 20.000 events.

### Results:

smartRNAs based on either the original S1 scaffold (SEQ IDs: 85 to 100, or Table 4a) or the SNORD115-22 scaffold (SEQ IDs: 117 to 132, or table 4b), or a variety of human snoRNA scaffolds (SEQ ID: 133-169, or table 4c) and targeting the human SCN1A 3'UTR and polyA sequence were shown to increase the amount of eGFP signal in HEK293 Flp-In T-Rex cells expressing the relevant target sequence (figure 8 and 9) when transfected in at 10 nM.

### ITEMS

1. A compound comprising a small artificial RNA (smartRNA) oligonucleotide comprising or consisting of a partially double stranded ribonucleic acid (dsRNA), the partially dsRNA comprising or consisting of a sense strand and an antisense strand,
   wherein the sense strand comprises or consists of a first contiguous nucleotide sequence comprising or consisting of, from 5' to 3':
      - a "stem I" nucleotide sequence comprising a "stem I" region of complementarity;
      - a "C box" nucleotide sequence comprising a "C box" region of complementarity;
      - a "D' guide" nucleotide sequence comprising a "D' guide" region of complementarity;
      - a "D' box" nucleotide sequence comprising a "D' box" region of complementarity;
      - a "stem II" nucleotide sequence comprising a "stem II" region of complementarity;
   wherein the antisense strand comprises or consists of a second contiguous nucleotide sequence comprising or consisting of, from 5' to 3':
      - a "stem II" nucleotide sequence comprising a "stem II" region of complementarity;
      - a "C' box" nucleotide sequence comprising a "C' box" region of complementarity
      - a "D guide" nucleotide sequence comprising a "D guide" region of complementarity;
      - a "D box" nucleotide sequence comprising a "D box" region of complementarity;
      - a "stem I" nucleotide sequence comprising a "stem I" region of complementarity;
   wherein the sense strand "stem I" and the antisense strand "stem I" regions of complementarity are at least partially complementary to each other and thereby can anneal at least partially to each other to form an at least partially double-stranded "stem I" secondary structure;
   wherein the "C box" and the "D box" regions of complementarity are at least partially complementary to each other and thereby can anneal at least partially to each other to form an at least partially double-stranded "C/D box" secondary structure;
   wherein the "D' box" and the "C' box" regions of complementarity are at least partially complementary to each other and thereby can anneal at least partially to each other to form an at least partially double-stranded "C'/D' box" secondary structure;
   wherein the sense strand "stem II" and the antisense strand "stem II" regions of complementarity are at least partially complementary to each other and thereby can anneal at least partially to each other to form an at least partially double-stranded "stem II" secondary structure;
   wherein the "D guide" region of complementarity comprises or consist of a first contiguous nucleotide sequence which is complementary to a same-length portion of a first target nucleic acid sequence of a target mRNA;
   wherein the "D' guide" region of complementarity comprises or consist of a second contiguous nucleotide sequence which is complementary to a same-length portion of a second target nucleic acid sequence of the same target mRNA; and
   wherein the first or second target nucleic acid sequence comprises the polyA-tail of said target mRNA.
2. A compound comprising a small artificial RNA (smartRNA) oligonucleotide comprising or consisting of a partially double stranded ribonucleic acid (dsRNA), the partially dsRNA comprising or consisting of a sense strand and an antisense strand,
   wherein the sense strand comprises or consists of a first contiguous nucleotide sequence comprising or consisting of, from 5' to 3':
      - a "stem I" nucleotide sequence comprising a "stem I" region of complementarity;
      - a "C box" nucleotide sequence comprising a "C box" region of complementarity;
      - a "D' guide" nucleotide sequence comprising a "D' guide" region of complementarity;
      - a "D' box" nucleotide sequence comprising a "D' box" region of complementarity;
   wherein the antisense strand comprises or consists of a second contiguous nucleotide sequence comprising or consisting of, from 5' to 3':
      - a "C' box" nucleotide sequence comprising a "C' box" region of complementarity
      - a "D guide" nucleotide sequence comprising a "D guide" region of complementarity;
      - a "D box" nucleotide sequence comprising a "D box" region of complementarity;
      - a "stem I" nucleotide sequence comprising a "stem I" region of complementarity;
   wherein the sense strand "stem I" and the antisense strand "stem I" regions of complementarity are at least partially complementary to each other and thereby can anneal at least partially to each other to form an at least partially double-stranded "stem I" secondary structure;
   wherein the "C box" and the "D box" regions of complementarity are at least partially complementary to each other and thereby can anneal at least partially to each other to form an at least partially double-stranded "C/D box" secondary structure;
   wherein the "D' box" and the "C' box" regions of complementarity are at least partially complementary to each other and thereby can anneal at least partially to each other to form an at least partially double-stranded "C'/D' box" secondary structure;
   wherein the "D guide" region of complementarity comprises or consist of a first contiguous nucleotide sequence which is complementary to a same-length portion of a first target nucleic acid sequence of a target mRNA;
   wherein the "D' guide" region of complementarity comprises or consist of a second contiguous nucleotide sequence which is complementary to a same-length portion of a second target nucleic acid sequence of the same target mRNA; and
   wherein the first or second target nucleic acid sequence comprises the polyA-tail of said target mRNA.
3. A compound comprising a small artificial RNA (smartRNA) oligonucleotide comprising or consisting of, from 5' to 3':
   - a "5' stem I" nucleotide sequence comprising a "5' stem I" region of complementarity;
   - a "C box" nucleotide sequence comprising a "C box" region of complementarity;
   - a "D' guide" nucleotide sequence comprising a "D' guide" region of complementarity;
   - a "D' box" nucleotide sequence comprising a "D' box" region of complementarity
   - a "5' stem II" nucleotide sequence comprising a "5' stem II" region of complementarity;
   - optionally a "loop" nucleotide sequence;
   - a "3' stem II" nucleotide sequence comprising a "3' stem II" region of complementarity;
   - a "C' box" nucleotide sequence comprising a "C' box" region of complementarity
   - a "D guide" nucleotide sequence comprising a "D guide" region of complementarity;
   - a "D box" nucleotide sequence comprising a "D box" region of complementarity;
   - a "3' stem I" nucleotide sequence comprising a "3' stem I" region of complementarity;

   wherein the "5' stem I" and the "3' stem I" regions of complementarity are at least partially complementary to each other and thereby can anneal at least partially to each other to form an at least partially double-stranded "stem I" secondary structure;
   wherein the "C box" and the "D box" regions of complementarity are at least partially complementary to each other and thereby can anneal at least partially to each other to form an at least partially double-stranded "C/D box" secondary structure;
   wherein the "D' box" and the "C' box" regions of complementarity are at least partially complementary to each other and thereby can anneal at least partially to each other to form an at least partially double-stranded "C'/D' box" secondary structure;
   wherein the "5' stem II" and the 3' stem II" regions of complementarity are at least partially complementary to each other and thereby can anneal at least partially to each other to form an at least partially double-stranded "stem II" secondary structure;
   wherein the "D guide" region of complementarity comprises or consist of a first contiguous nucleotide sequence which is complementary to a same-length portion of a first target nucleic acid sequence of a target mRNA;
   wherein the "D' guide" region of complementarity comprises or consist of a second contiguous nucleotide sequence which is complementary to a same-length portion of a second target nucleic acid sequence of the same target mRNA; and
   wherein the first or second target nucleic acid sequence comprises the polyA-tail of said target mRNA.
4. A compound comprising a small artificial RNA (smartRNA) oligonucleotide comprising or consisting of, from 5' to 3':
   - a "5' stem I" nucleotide sequence comprising a "5' stem I" region of complementarity;
   - a "C box" nucleotide sequence comprising a "C box" region of complementarity;
   - a "D' guide" nucleotide sequence comprising a "D' guide" region of complementarity;
   - a "D' box" nucleotide sequence comprising a "D' box" region of complementarity
   - optionally a "loop" nucleotide sequence;
   - a "C' box" nucleotide sequence comprising a "C' box" region of complementarity
   - a "D guide" nucleotide sequence comprising a "D guide" region of complementarity;
   - a "D box" nucleotide sequence comprising a "D box" region of complementarity;
   - a "3' stem I" nucleotide sequence comprising a "3' stem I" region of complementarity;

   wherein the "5' stem I" and the "3' stem I" regions of complementarity are at least partially complementary to each other and thereby can anneal at least partially to each other to form an at least partially double-stranded "stem I" secondary structure;
   wherein the "C box" and the "D box" regions of complementarity are at least partially complementary to each other and thereby can anneal at least partially to each other to form an at least partially double-stranded "C/D box" secondary structure;
   wherein the "D' box" and the "C' box" regions of complementarity are at least partially complementary to each other and thereby can anneal at least partially to each other to form an at least partially double-stranded "C'/D' box" secondary structure;
   wherein the "D guide" region of complementarity comprises or consist of a first contiguous nucleotide sequence which is complementary to a same-length portion of a first target nucleic acid sequence of a target mRNA;
   wherein the "D' guide" region of complementarity comprises or consist of a second contiguous nucleotide sequence which is complementary to a same-length portion of a second target nucleic acid sequence of the same target mRNA; and
   wherein the first or second target nucleic acid sequence comprises the polyA-tail of said target mRNA.
5. The compound according to any of the preceding items, wherein:
   - the "stem I" nucleotide sequences and/or regions of complementarity are independently between 0 and 30 nucleotides in length, such as between 0 and 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length; preferably independently between 0 and 12 nucleotides in length, such as preferably independently between 6 and 12 nucleotides in length, more preferably between 7 and 10 nucleotides in length;
   - the "C box" and "D box" nucleotide sequences and/or regions of complementarity are independently between 0 and 30 nucleotides in length, such as between 0 and 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length; preferably between 4 and 8 nucleotides in length;
   - the "D' box" and "C' box" nucleotide sequences and/or regions of complementarity are independently between 1 and 30 nucleotides in length, such as between 1 and 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, preferably between 4 and 8 nucleotides in length,
   - the "stem II" nucleotide sequences and/or regions of complementarity are independently between 0 and 30 nucleotides in length, such as between 0 and 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length; preferably between 0 and 7 nucleotides in length; such as between 1 and 7 nucleotides in length; and/or
   - the "loop" nucleotide sequence is between 0 and 30 nucleotides in length, such as between 0 and 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length, such as 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides in length; preferably between 0 and 7 nucleotides in length, such as between 1 and 7 nucleotides in length;
6. The compound according to any of the preceding items, wherein:
   - the "stem I" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other;
   - the "C box" and the "D box" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other;
   - the "D' box" and the "C' box" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other; and/or
   - the "stem II" regions of complementarity are at least 70% complementary, such as at least 70%, 75%, 80%, 85%, 90%, 95%, or 100% complementary, preferably at least 90% complementary, more preferably at least 95% complementary, even more preferably fully complementary to each other.
7. The compound according to any of the preceding items, wherein:
   - the "5' stem I" and the "3' stem I" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other;
   - the "C box" and the "D box" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other;
   - the "D' box" and the "C' box" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other, and/or
   - the "5' stem II" and the 3' stem II" regions of complementarity can anneal and/or anneal at least 70% to each other, such as at least 70%, 75%, 80%, 85%, 90%, 95%, or 100% complementary, preferably at least 90% to each other, more preferably at least 95% to each other, even more preferably fully to each other.
8. The compound according to any of the preceding items, wherein the first contiguous nucleotide sequence of the "D guide" region of complementarity and/or the second contiguous nucleotide sequence of the "D' guide" region of complementarity is/are independently of at least 7 nucleotides in length, preferably between 7 and 30 nucleotides in length, such as between 7 and 8, 9, 10, 11, 12, 13, 14, 15, 16, 17, 18, 19, 20, 21, 22, 23, 24, 25, 26, 27, 28, 29, 30 nucleotides in length, such as 7, 8, 9, 10, 11, 12, 13, 14, 15, 16, 17, 18, 19, 20, 21, 22, 23, 24, 25, 26, 27, 28, 29, or 30 nucleotides in length.
9. The compound according to any of the preceding items, wherein one of the first contiguous nucleotide sequence of the "D guide" region of complementarity or the second contiguous nucleotide sequence of the "D' guide" region of complementarity is between 7 and 15 nucleotides, and the other one is between 12 and 30 nucleotides.
10. The compound according to any of the preceding items, wherein the first contiguous nucleotide sequence of the "D guide" region of complementarity and/or the second contiguous nucleotide sequence of the "D' guide" region of complementarity is/are at least 70% complementary, such as at least 70%, 75% 80%, 85%, 90%, 95%, or 100% complementary, preferably at least 90% complementary, more preferably at least 95% complementary, even more preferably fully complementary to a same-length portion of the first and/or second target nucleic acid sequence of the target mRNA, respectively.
11. The compound according to any of the preceding items, wherein the first target nucleic acid sequence comprises the polyA-tail of said target mRNA, and wherein the second target nucleic acid sequence of the target mRNA is selected from the group consisting of the 5'UTR, the 5'UTR and the open reading frame (ORF), the open reading frame (ORF), the open reading frame and the 3'UTR, the 3'UTR, and the 3'UTR and 5'UTR of the mRNA.
12. The compound according to any of the preceding items, wherein the second target nucleic acid sequence comprises the polyA-tail of said target mRNA, and wherein the first target nucleic acid sequence of the target mRNA is selected from the group consisting of the 5'UTR, the 5'UTR and the open reading frame (ORF), the open reading frame (ORF), the open reading frame and the 3'UTR, the 3'UTR, and the 3'UTR and 5'UTR of the mRNA
13. The compound according to any of the preceding items, wherein the first target nucleic acid sequence comprises the polyA-tail of said target mRNA, and wherein the "D guide" comprises a homo-polymeric sequence, preferably a poly-uridine sequence, such as at least 7 contiguous uridines, preferably between 7 and 12 uridines.
14. The compound according any of the preceding items, wherein the second target nucleic acid sequence comprises the polyA-tail of said target mRNA, and wherein the "D' guide" comprises a homo-polymeric sequence, preferably a poly-uridine sequence such as at least 7 contiguous uridines, preferably between 7 and 12 uridines.
15. The compound according to any one of the preceding items, wherein the "C box" nucleotide sequence of the smart RNA is selected from the "C box" sequences of Table 1.
16. The compound according to any one of the preceding items, wherein the "D box" nucleotide sequence of the smart RNA is selected from the "D box" sequences of Table 1.
17. The compound according to any one of the preceding items, wherein the "C' box" nucleotide sequence of the smart RNA is selected from the "C' box" sequences of Table 1.
18. The compound according to any one of the preceding items, wherein the "D' box" nucleotide sequence of the smart RNA is selected from the "D' box" sequences of Table 1.
19. The compound according to any one of the preceding items, wherein the binding of the first contiguous nucleotide sequence of the "D guide" region of complementarity and the second contiguous nucleotide sequence of the "D' guide" region of complementarity, to the first and second target sequences, respectively, increases the 2'-O-methylation level of one or more adenosines of the polyA-tail of the target mRNA..
20. The compound according to item 15, wherein the 2'-O-methylation of one or more adenosines of the poly-A tail is increased by at least 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or at least 1000% compared to a control.
21. The compound according to any one of the preceding items, wherein the smartRNA is between 30 and 150 nucleotides in length, such as between 30 and 40, 50, 60, 70, 80, 90, 100, 110, 120, 130, 140, or 150 nucleotides in length, such as between 40 and 50, 60, 70, 80, 90, 100, 110, 120, 130, 140, or 150 nucleotides in length, such as between 50 and 60, 70, 80, 90, 100, 110, 120, 130, 140, or 150 nucleotides in length, such as between 60 and 70, 80, 90, 100, 110, 120, 130, 140, or 150 nucleotides in length, such as between 70 and 80, 90, 100, 110, 120, 130, 140, or 150 nucleotides in length, such as between 80 and 90, 100, 110, 120, 130, 140, or 150 nucleotides in length, such as between 90 and 100, 110, 120, 130, 140, or 150 nucleotides in length, such as between 100 and 110, 120, 130, 140, or 150 nucleotides in length, such as between 110 and 120, 130, 140, or 150 nucleotides in length, such as between 120 and 130, 140, or 150 nucleotides in length, such as between 130 and 140, or 150 nucleotides in length, such as between 140 and 150 nucleotides in length, preferably between 60 and 80 nucleotides in length, such as between 60 and 70 nucleotides in length or between 70 and 80 nucleotides in length.
22. The compound according to any one of the preceding items, wherein the smartRNA comprises at least one modified nucleotide.
23. The compound according to any one of the preceding items, wherein the smartRNA comprises at least one modified nucleotide comprising a modified sugar moiety; preferably wherein the modified sugar moiety is independently selected from a bicyclic sugar moiety or a non-bicyclic sugar moiety.
24. The compound according to any one of the preceding items, wherein the smartRNA comprises at least one modified nucleotide comprising a non-bicyclic sugar moiety.
25. The compound according to any one of the preceding items, wherein the smartRNA comprises at least one modified nucleotide comprising a non-bicyclic sugar moiety, wherein the non-bicyclic sugar moiety is independently selected from the group consisting of 2'-O-alkyl-RNA, 2'-O-methyl-RNA (2'OMe modified sugar), 2'-alkoxy-RNA, 2'-O-methoxyethyl-RNA, 2'-amino-DNA, 2'-fluoro-DNA (2'F modified sugar), arabino nucleic acid (ANA), 2'-fluoro-ANA, Glycol nucleic acid (GNA), inverted RNA (3'-3' linked, or 5'-5' linked), 2'-azido-DNA, 2'-O-propargyl-RNA, L-ribose based nucleotides (mirror image nucleotides), and unlocked nucleic acid (UNA); preferably wherein the non-bicyclic sugar moiety is independently selected from a 2'F modified sugar and a 2'OMe modified sugar.
26. The compound according to any one of the preceding items, wherein the smartRNA comprises at least one modified nucleotide comprising a bicyclic sugar moiety.
27. The compound according to any one of the preceding items, wherein the smartRNA comprises at least one modified nucleotide comprising a bicyclic sugar moiety, wherein the bicyclic sugar moiety is independently selected from the group consisting of locked nucleic acids (LNAs), bridged nucleic acids (BNAs) and analogues thereof, constrained ethyl nucleic acid (cEt), constrained ethyl BNAs (cET-BNAs), Tricyclo-DNA, and constrained methoxyethyl BNAs.
28. The compound according to any one of the preceding items, wherein the smartRNA comprises at least one nucleotide with a modified base, such as, isocytosine, pseudoisocytosine, 5-methyl cytosine, 5-thiozolo-cytosine, 5-propynyl-cytosine, 5-propynyl-uracil, 5-bromouracil 5-thiazolo-uracil, 2-thio-uracil, 2'thio-thymine, inosine, diaminopurine, 6-aminopurine, 2-aminopurine, 2,6-diaminopurine, 2-chloro-6-aminopurine, pseudouridine (Ψ), N1-methylspeudouridine (m1Ψ), 5-methylcytidine (m5C), N6-methyladenosine (m6A), 7-methylguanosine (m7G), 5-methoxyuridine, inosine, purine, xanthine, hypoxanthine, 2,2,7-trimethylguanosine (m3G/TMG cap), pyridin-4-one, pyridin-2-one, phenyl, 2,4,6-trimethoxy benzene, 3-methyl uracil, 2-thiouridine,s dihydrouridine, naphthyl, aminophenyl, 5-alkylcytidines, 5-fluoro-2'-deoxyuridine, 2',2'-difluoro-2'-deoxy nucleosides, 5-alkyluridines (e.g., ribothymidine), 5-halouridine (e.g., 5-bromouridine), 2',4'-difluorotoluyl nucleosides, 6-azapyrimidines, 6-alkylpyrimidines (e.g. 6-methyluridine), 5-nitroindole, and 3-nitropyrrole, preferably pseudouridine (Ψ), N1-methylspeudouridine (m1Ψ), and 5-methylcytidine (m5C).
29. The compound according to any one of the preceding items, wherein the smartRNA is a totalmer or a mixmer.
30. The compound according to any one of the preceding items, wherein the smartRNA comprises at least one modified internucleoside linkage.
31. The compound according to any one of the preceding items, wherein the smartRNA comprises at least one modified internucleoside linkage, wherein the modified internucleoside linkage is independently selected from the group consisting of phosphorothioates, chiral phosphorothioates, diphosphorothioates, phosphorodithioates, phosphoramidates, boranophosphates, methyl phosphonates, chiral methyl phosphonates, sulfonylphosphoramidates, methoxypropylphosphonates, 5'-vinylphosphonates, 5'-methylphosphonates, 5'-phosphorothioates, phosphodiesters, phosphotriesters. 5'-hydroxyphosphonates, Inverted nucleotide linkages (3'-3', and 5'-5'), and 3'-O-methylphosphonates internucleoside linkages.
32. The compound according to any one of the preceding items, wherein each internucleoside linkage of the smartRNA is either a phosphodiester internucleoside linkage, phosphorodithioate internucleoside linkage, or a phosphorothioate internucleoside linkage.
33. The compound according to any one of the preceding items, wherein the smartRNA comprises one or more chemically modified nucleotides, such as one or more nucleotide analogues, modified bases, modified backbones, and non-nucleotide linkages which are synthetic, naturally occuring, and non-naturally occurring; such as, phosphorothioates, chiral phosphorothioates, phosphorodithioates, phosphoramidates, methyl phosphonates, chiral methyl phosphonates, sulfonylphosphoramidates, methoxypropylphosphonates, 5'-vinylphosphonates, 5'-methylphosphonates, 5'-phosphorothioates, phosphodiesters, phosphotriesters, peptide-nucleic acids (PNAs), locked nucleic acids (LNAs), bridged nucleic acids (BNAs) and analogues thereof, constrained ethyl BNAs (cET-BNAs), constrained methoxyethyl BNAs, unlocked nucleic acids (UNAs), glycol nucleic acids (GNAs), phosphorodiamidate morpholino oligomers (PMOs), thiomorpholino oligomers (TMOs), ribonucleic acids (RNAs), 2'-deoxyribonucleic acids (DNAs), tricyclo-DNAs (tcDNAs), inverted nucleotides, 5'-5' reverse linkages, 3'-3' reverse linkages, L-ribose based nucleotides (mirror image nucleotides), 2',3'-dideoxy nucleotides, 2'-modified nucleotides such as, but not limited to, 2'-O-methyl (2'-O-Me), 2'-O-methoxyethyl (2'-O-MOE), 2'-fluoro (2'-F), 2'-arabino-fluoro (2'-Ara-F), 2'-methoxyethoxy, 2'-O-alkyl, 2'-O-alkenyl, 2'-O-alkynyl, 2'-O-benzyl, 2'-allyl, 2'-amino, 2'-azido, 2'-pyridinyl, 2'-O-propargyl, 2'-O-[2-(methylamino)-2-oxoethyl], 4'-thio, 2'-O-(N-methylcarbamate), nucleotides with modified bases such as, but not limited to, pseudouridine (Ψ), N1-methylspeudouridine (m1Ψ), 5-methylcytidine (m5C), N6-methyladenosine (m6A), 7-methylguanosine (m7G), 5-methoxyuridine, inosine, purine, xanthine, hypoxanthine, 2,2,7-trimethylguanosine (m3G/TMG cap), pyridin-4-one, pyridin-2-one, phenyl, 2,4,6-trimethoxy benzene, 3-methyl uracil, 2-thiouridine,s dihydrouridine, naphthyl, aminophenyl, 5-alkylcytidines, 5-fluoro-2'-deoxyuridine, 2',2'-difluoro-2'-deoxy nucleosides, 5-alkyluridines (e.g., ribothymidine), 5-halouridine (e.g., 5-bromouridine), 2',4'-difluorotoluyl nucleosides, 6-azapyrimidines, 6-alkylpyrimidines (e.g. 6-methyluridine), 5-nitroindole, 3-nitropyrrole, nucleosides or non-nucleoside linkers containing chemical handles such as, but not limited to, amines (e.g. aminohexyl), azides, alkynes, alkenes, acryls, thiols, hydrazines, aldehydes, biotin, digoxigenin, and others.
34. The compound according to any one of the preceding items, wherein the smartRNA is circularized.
35. The compound according to any one of the preceding items, wherein the smartRNA is circularized by at least partially annealing the "5' stem I" and the "3' stem I" nucleotide sequences to each other, or by covalently joining the 5'- and 3'- ends; preferably by covalently joining the 5'- and 3'- ends.
36. The compound according to any one of the preceding items, wherein the smartRNA comprises one or more nick.
37. The compound according to item 36, wherein the one or more nick is located / are independently located between the sense strand "stem I" or the "5' stem I" and the "C box" region of complementarity; between the "C box" and the "D' guide" region of complementarity; between the "D' guide" and the "D' box" region of complementarity; between the "D' box" and the sense strand "stem II" or the "5' stem II" region of complementarity; between the sense strand "stem II" or the "5' stem II" region of complementarity and the loop nucleotide sequence; between the loop nucleotide sequence and the antisense strand "stem II" or the "3' stem II" region of complementarity; between the antisense strand "stem II" or the "3' stem II" and the "C' box" region of complementarity; between the "C' box" and the "D guide" region of complementarity; between the "D guide" and the "D box" region of complementarity; and/or between the "D box" and the antisense strand "stem I" or the "3' stem I" region of complementarity.
38. The compound according to any one of the preceding items, wherein the complementary comprises or consists of base pairs formed entirely or partially from base pairs selected from the group consisting of Watson-Crick base pairs, non-Watson-Crick base pairs, base pairs formed from non-natural and/or modified nucleotides.
39. The compound according to item 38, wherein the complementarity comprises or consists of base pairs formed entirely or partially from Watson-Crick base pairs, such as guanine (G)-cytosine (C) base pairs, adenine (A)-thymine (T), and/or adenine (A) - uracil (U) base pairs.
40. The compound according to item 38 or 39, wherein the complementarity comprises or consists of base pairs formed entirely or partially from non-Watson-Crick base pairs, such as non-canonical base pairs, such as Hoogsteen base pair, such as G-A trans sugar edge, and/or Wobble base pairs.
41. The compound according to item 40, wherein the non-canonical base pairs comprise U-U wobble base pairs, G-U wobble base pairs, Hoogsteen A-G base pairs
42. The compound according to any one of the preceding items, further comprising endogenous RNA binding protein (RNP) complex, thereby forming a smartRNP.
43. The compound according to item 42, wherein the endogenous RNA binding proteins (RNP) are selected from the group consisting of:
   i. Fibrillarin, such as Fibrillarin as set forth in SEQ ID NO: 1;
   ii. NOP56, such as NOP56 as set forth in SEQ ID NO: 2;
   iii. NOP58, such as NOP58 as set forth in SEQ ID NO: 3; and
   iv. SNU13, such as SNU13 as set forth in SEQ ID NO: 4.
44. The compound according to any one of items 1-43, wherein the smartRNA comprises:
   i. a "5' stem I" nucleotide sequence comprising or consisting of the nucleotide sequence GAUCAC or GGAUCAC;
   ii. a "C-box" nucleotide sequence comprising or consisting of the nucleotide sequence GUGAUGA;
   iii. a "D' guide" nucleotide sequence;
   iv. a "D'-box" nucleotide sequence comprising or consisting of the nucleotide sequence CUGA;
   v. a "5' stem II" nucleotide sequence comprising or consisting of the nucleotide sequence CCUCAG;
   vi. optionally, a loop nucleotide sequence comprising or consisting of the nucleotide sequence GCCU;
   vii. a "3' stem II" nucleotide sequence comprising or consisting of the nucleotide sequence CUGAGGG;
   viii. a "C' box" nucleotide sequence comprising or consisting of the nucleotide sequence GUGAUGA;
   ix. a "D guide" nucleotide sequence;
   x. a "D-box" nucleotide sequence comprising or consisting of the nucleotide sequence CUGA; and
   xi. a "3' stem I" nucleotide sequence comprising or consisting of the nucleotide sequence GUGAUC or GUGAUCC;

   wherein the "D guide" nucleotide sequence comprises or consist of a first contiguous nucleotide sequence which is complementary to a same-length portion of a first target nucleic acid sequence of a target mRNA;
   wherein the "D' guide" nucleotide sequence comprises a second contiguous nucleotide sequence which is complementary to a same-length portion of a second target nucleic acid sequence of the same target mRNA; and
   wherein the first or second target nucleic acid sequence comprises the polyA-tail of said target mRNA.
45. The compound according to any one of items 1-43, wherein the smartRNA comprises the sequences of Table 2a.
46. The compound according to any one of the preceding items, wherein the smartRNA comprises:
   i. a "5' stem I" nucleotide sequence comprising or consisting of the nucleotide sequence GGUUGGGUCA(SEQ ID NO: 34);
   ii. a "C-box" nucleotide sequence comprising or consisting of the nucleotide sequence AUGAUGA;
   iii. a "D' guide" nucleotide sequence;
   iv. a "D'-box" nucleotide sequence comprising or consisting of the nucleotide sequence CUGA;
   v. optionally, a loop nucleotide sequence comprising or consisting of the nucleotide sequence AGAGAG;
   vi. a "C' box" nucleotide sequence comprising or consisting of the nucleotide sequence GUGAUGA;
   vii. a "D guide" nucleotide sequence;
   viii. a "D-box" nucleotide sequence comprising or consisting of the nucleotide sequence CUGA; and
   ix. a "3' stem I" nucleotide sequence comprising or consisting of the nucleotide sequence GUCCCAGCCU (SEQ ID NO: 35);

   wherein the "D guide" nucleotide sequence comprises or consist of a first contiguous nucleotide sequence which is complementary to a same-length portion of a first target nucleic acid sequence of a target mRNA;
   wherein the "D' guide" nucleotide sequence comprises a second contiguous nucleotide sequence which is complementary to a same-length portion of a second target nucleic acid sequence of the same target mRNA; and
   wherein the first or second target nucleic acid sequence comprises the polyA-tail of said target mRNA.
47. The compound according to any one of items 1-43, wherein the smartRNA comprises the sequences of Table 2b.
48. The compound according to any one of the preceding items, wherein the smartRNA comprises:
   i. a "5' stem I" nucleotide sequence comprising or consisting of the nucleotide sequence 
GGAAAGAGGAAC, GGAGCUGAGUCC, GGAGGCUUUGCA, GGAUACUUUCCU,
GGAUUGCUCACU, GGAUUGUGGCCA, GGCCCCCUUCCU, GGCCUAUCUCUC,
GGCUGCUCACCA, GGCUUUACAGUA, GGGCUGGCAUAU, GGGGAUCACG,
GGGGCUGGAUUU, GGGGGCGGGGCG, GGGGGGAUCACG, GGGGUUGGAUCG,
GGGGUUGGGUCA, or GGUGCCCUGCCU (SEQ ID NO: 36-53);
   ii. a "C-box" nucleotide sequence comprising or consisting of the nucleotide sequence GUGAUGA, AUGAUGA, AUGAGGA, CUGAUGA, or AGGAUGA;
   iii. a "D' guide" nucleotide sequence;
   iv. a "D'-box" nucleotide sequence comprising or consisting of the nucleotide sequence CUGA, CAGA, UUGA, or CCGA;
   v. optionally, a "5' stem II" nucleotide sequence comprising or consisting of the nucleotide sequence UCA, AG, CCUCAG, or UUUUAUGUGC (SEQ ID NO: 72);
   vi. optionally, a "loop" nucleotide sequence comprising or consisting of the nucleotide sequence GUGUCG, UCGACU, AUGUG, GCAAAGAAAGGCCUUU (SEQ ID NO: 73), GC, AGAGAG, GCUCC, GCCU, ACCAC, GUCACC, GAGUA, AAUGUUUUUCCUU (SEQ ID NO: 74), GUGCAAUCA, UUCU, GCUGCU, GACACC, CAGAUCGACU (SEQ ID NO: 75), or UCAAUGU;
   vii. optionally, a "3' stem II" nucleotide sequence comprising or consisting of the nucleotide sequence UGA, CU, CUGAGGG, or GCU;
   viii. a "C' box" nucleotide sequence comprising or consisting of the nucleotide sequence GUGCUGA, AUGUUGA, CUGAAAG, GUGUGGA, AUGAUGA, GUGAUGA, GUGAGGA, AUGAUUU, AACAUGU, AUGUUGG, CUGAUGU, GUGAUGG, GUGAUUU, AUGAUGG, or GUGACUG;
   ix. a "D guide" nucleotide sequence;
   x. a "D-box" nucleotide sequence comprising or consisting of the nucleotide sequence CUGA; and
   xi. a "3' stem I" nucleotide sequence comprising or consisting of the nucleotide sequence

GCUGCCGCCCCAUAG, UAUGCCAGCUCAUAG, GCUGUGAGCCCAUAG,
AACUCAGUUUCAUAG, AGUCCAGCCUCAUAG, GUCCCAGCCUCAUAG,
GGAGAUGGGCCAUAG, GUGAUCCCCCAUAG, UGCUGUUUGCCAUAG,
GGCCCAGCCUCAUAG, GUCCUCUGACCAUAG, UGGUGGCACACAUAG,
GUGAGCAUCUCAUAG, GCAAUGCCUGCAUAG, GUGAUCACAUAG,
GGCCAAAUUGCAUAG, AGAAGAGAUGCAUAG, or GGAAGGGAGGCAUAG (SEQ ID NO:
54-71);

   wherein the "D guide" nucleotide sequence comprises or consist of a first contiguous nucleotide sequence which is complementary to a same-length portion of a first target nucleic acid sequence of a target mRNA;
   wherein the "D' guide" nucleotide sequence comprises a second contiguous nucleotide sequence which is complementary to a same-length portion of a second target nucleic acid sequence of the same target mRNA; and
   wherein the first or second target nucleic acid sequence comprises the polyA-tail of said target mRNA.
49. The compound according to any one of items 1-43, wherein the smartRNA comprises the sequences of Table 2c and, optionally, the sequences of Table 2d.
50. The compound according to any one of items 44 to 49, wherein
   i) the "D' guide" nucleotide sequence comprises, or consists of:
      - a nucleotide sequence selected from the group consisting of the nucleotide sequences of SEQ ID NO: 76 to 84, or
      - a sequence selected from the group consisting of SEQ ID NO: 76 to 84; and/or
   ii) the "D guide" nucleotide sequence comprises, of consists of:
      - a nucleotide sequence selected from the group consisting of the nucleotide sequences of SEQ ID NO: 76 to 84, or
      - a sequence selected from the group consisting of SEQ ID NO: 76 to 84.
51. The compound according to any one of items 44 to 49, wherein
   i) the "D' guide" nucleotide sequence comprises, or consists of:
      - a nucleotide sequence selected from the group of the nucleotide sequence of the sequences of Table 3, or
      - a sequence selected from the group consisting of the sequences of Table 3; and/or
   ii) the "D guide" nucleotide sequence comprises, or consists of:
      - a nucleotide sequence selected from the group of the nucleotide sequence of the sequence of Table 3, or
      - a sequence selected from the group consisting of the sequences of Table 3.
52. A compound comprising a small artificial RNA (smartRNA) oligonucleotide comprising, or consisting of:
   - a nucleotide sequence selected from the group consisting of the nucleotide sequences of SEQ ID NO: 7 to 10; or
   - a sequence selected from the group consisting of SEQ ID NO: 7 to 10.
53. A compound comprising a small artificial RNA (smartRNA) oligonucleotide comprising, or consists of:
   - a nucleotide sequence selected from the group of the nucleotide sequences of SEQ ID NO: 85 to 116, or
   - a sequence selected from the group of SEQ ID NO: 85 to 116.
54. A compound comprising a small artificial RNA (smartRNA) oligonucleotide comprising, or consists of:
   - a nucleotide sequence selected from any one of the nucleotide sequences of Table 4a
   - a sequence selected from any one of the sequences of Table 4a.
55. A compound comprising a small artificial RNA (smartRNA) oligonucleotide comprising, or consists of:
   - a nucleotide sequence selected from the group of the nucleotide sequences of SEQ ID NO: 117 to 132, or
   - a sequence selected from the group of SEQ ID NO: 117 to 132.
56. A compound comprising a small artificial RNA (smartRNA) oligonucleotide comprising, or consists of:
   - a nucleotide sequence selected from any one of the nucleotide sequences of Table 4b, or
   - a sequence selected from any one of the sequences of Table 4b.
57. A compound comprising a small artificial RNA (smartRNA) oligonucleotide comprising, or consists of:
   - a nucleotide sequence selected from the group of the nucleotide sequences of SEQ ID NO: 133 to 169, or
   - a sequence selected from the group of SEQ ID NO: 133 to 169.
58. A compound comprising a small artificial RNA (smartRNA) oligonucleotide comprising, or consists of:
   - a nucleotide sequence selected from any one of the nucleotide sequences of Table 4c, or
   - a sequence selected from any one of the sequences of Table 4c.
59. A compound comprising a small artificial RNA (smartRNA) oligonucleotide comprising, or consists of
   - a nucleotide sequence selected from the group of the nucleotide sequences of SEQ ID NO: 170 to 171, or
   - a sequence selected from the group of SEQ ID NO: 170 to 171.
60. A compound comprising a small artificial RNA (smartRNA) oligonucleotide comprising, or consists of:
   - a nucleotide sequence selected from any one of the nucleotide sequences of Table 5a, or
   - a sequence selected from any one of the sequences of Table 5a.
61. A compound comprising a small artificial RNA (smartRNA) oligonucleotide comprising, or consists of
   - the nucleotide sequence of SEQ ID NO: 172, or
   - the sequence of SEQ ID NO: 172.
62. A compound comprising a small artificial RNA (smartRNA) oligonucleotide comprising, or consists of:
   - a nucleotide sequence selected from any one of the nucleotide sequences of Table 5b, or
   - a sequence selected from any one of the sequences of Table 5b.
63. A compound comprising a small artificial RNA (smartRNA) oligonucleotide comprising, or consists of
   - a nucleotide sequence selected from the group of the nucleotide sequences of SEQ ID NO: 173 to 192, or
   - a sequence selected from the group of SEQ ID NO: 173 to 192.
64. A compound comprising a small artificial RNA (smartRNA) oligonucleotide comprising, or consists of:
   - a nucleotide sequence selected from any one of the nucleotide sequences of Table 5c, or
   - a sequence selected from any one of the sequences of Table 5c.
65. The compound according to any one of the preceding items, wherein the compound is covalently attached to at least one conjugate moiety.
66. The compound according to any one of the preceding items, wherein a conjugate moiety is covalently attached to the smartRNA, such as covalently attached at the 3'-end of the smartRNA and/or covalently attached at the 5'-end of the smartRNA, and/or covalently attached at any position between the 5' and 3' end.
67. The compound according to any one of the preceding items, wherein the compound is covalently attached to at least one conjugate moiety independently selected from the group consisting of carbohydrates, fatty acids, cell surface receptor ligands, drug substances, hormones, lipophilic substances, polymers, proteins, peptides, toxins (e.g. bacterial toxins), vitamins, viral proteins (e.g. capsids) and combinations thereof.
68. The compound according to any one of the preceding items, wherein the compound is covalently attached to at least one conjugate moiety independently selected from a fatty acid, such as a C10, C12, C14, C16, or C18 fatty acid, preferably a C16 fatty acid, more preferably palmitic acid.
69. The compound according to any one of the preceding items, wherein the compound is covalently attached to at least one conjugate moiety via a conjugate linker.
70. The compound according to any one of the preceding items, wherein the compound is covalently attached to at least one conjugate moiety via a cleavable conjugate linker.
71. The compound according to any one of the preceding items, wherein the compound is covalently attached to at least one conjugate moiety via a cleavable conjugate linker which comprises 1 to 3 linker nucleotides.
72. The compound according to any one of the preceding items, wherein the compound is covalently attached to at least one conjugate moiety via a C6 amino linker.
73. The compound according to any one of the preceding items, wherein the compound is directly attached to at least one conjugate moiety.
74. The compound according to any one of the preceding items, wherein the compound is encapsulated in a lipid-based delivery vehicle, covalently linked to or encapsulated in a dendrimer, or conjugated to an aptamer.
75. The compound according to any one of the preceding items, wherein the smartRNA is capable of assembling with endogenous RNA binding protein (RNP) complex, thereby forming a smartRNP.
76. The compound according to any one of the preceding items, wherein the smartRNA is capable of forming a smartRNP by assembling with at least one of the endogenous proteins, such as with all the endogenous proteins, selected from the group consisting of:
   xii. Fibrillarin, such as Fibrillarin as set forth in SEQ ID NO: 1;
   xiii. NOP56, such as NOP56 as set forth in SEQ ID NO: 2;
   xiv. NOP58, such as NOP58 as set forth in SEQ ID NO: 3; and
   xv. SNU13, such as SNU13 as set forth in SEQ ID NO: 4.
77. The compound according to any one of the preceding items, wherein binding of the first and second contiguous nucleotide sequence to the first and second target nucleic acid sequence of the target mRNA, respectively, increases the 2'-O-methylation level of one or more adenosines of the polyA-tail of the target mRNA.
78. The compound according to any one of the preceding items, wherein binding of the first and second contiguous nucleotide sequence to the first and second target nucleic acid sequence of the target mRNA, respectively, increases the stability of the target mRNA.
79. The compound according to any one of the preceding items, wherein binding of the first and second contiguous nucleotide sequence to the first and second target nucleic acid sequence of the target mRNA, respectively, increases the expression of the protein encoded by the target mRNA.
80. The compound according to any one of the preceding items, wherein binding of the first and second contiguous nucleotide sequence to the first and second target nucleic acid sequence of the target mRNA, respectively, reduces deadenylation of the target mRNA.
81. The compound according to any one of the preceding items, wherein binding of the first and second contiguous nucleotide sequence to the first and second target nucleic acid sequence of the target mRNA, respectively, reduces degradation of the target mRNA.
82. The compound according to item 75 or 76, wherein the binding of the first and second contiguous nucleotide sequence of the smartRNA contained in a smartRNP complex to the first and second target nucleic acid sequence of the target mRNA, respectively, increases the 2'-O-methylation level of one or more adenosines of the polyA-tail of the target mRNA.
83. The compound according to item 82, wherein said increase of the 2'-O-methylation of one or more adenosines of the polyA-tail of the target mRNA increases the stability of the target mRNA.
84. The compound according to item 82, wherein said increase of the 2'-O-methylation of one or more adenosines of the polyA-tail of the target mRNA increases the expression of the protein encoded by the target mRNA.
85. The compound according to item 82, wherein said increase of the 2'-O-methylation of one or more adenosines of the polyA-tail of the target mRNA reduces the deadenylation of the target mRNA.
86. The compound according to item 82, wherein said increase of the 2'-O-methylation of one or more adenosines of the polyA-tail of the target mRNA reduces the degradation of the target mRNA.
87. The compound according to any one of the preceding items, wherein the compound is capable of increasing or upregulating the expression of a target mRNA, such as capable of increasing the expression of target mRNA by at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 550, 600, 650, 700, 750, 800, 850, 900, 950, or at least 1000%, compared to a control.
88. The compound according to any one of the preceding items, wherein the compound is capable of increasing or upregulating the expression of a target protein, such as capable of increasing the expression of target protein by at least 10, 15, 20, 25, 30, 35, 40, 45, 50, 55, 60, 65, 70, 75, 80, 85, 90, 95, 100, 110, 120, 130, 140, 150, 160, 170, 180, 190, or at least 200%, compared to a control.
89. The compound according to item 87 or 88, wherein the control is a cell that has not been exposed to the compound.
90. The compound according to any one of the preceding items, wherein the compound is in the form of a pharmaceutically acceptable salt; preferably a sodium salt or a potassium salt.
91. A vector comprising a nucleotide sequence encoding the smartRNA according to any one of the preceding items.
92. A host cell expressing the smartRNA according to any one of items 1 to 90.
93. A composition comprising the smartRNA according to any one of items 1 to 90, the vector according to item 91, or the host cell according to item 92.
94. The composition according to item 93, wherein the composition is a pharmaceutical composition.
95. The composition according to any one of items 93 to 94, wherein the composition further comprises a pharmaceutically acceptable diluent, solvent, carrier, salt and/or adjuvant; preferably an aqueous diluent or solvent; more preferably phosphate buffered saline.
96. The composition according to any one of items 93 to 95, wherein the composition further comprises one or more additional smartRNAs according to any one of items 1 to 90.
97. The composition according to any one of items 93 to 96, wherein the composition further comprises one or more additional therapeutic agents, such as agonist therapeutic agents.
98. The compound according to any one of items 1 to 90, the vector according to item 91, the host cell according to item 92, or the composition according to any one of items 93 to 97, for use as a medicament.
99. The compound according to any one of items 1 to 90, the vector according to item 91, the host cell according to item 92, or the composition according to any one of items 93 to 97, for use in preventing, treating and/or ameliorating a medical condition affecting a subject.
100. Use of the compound according to any one of items 1 to 90, the vector according to item 91, the host cell according to item 92, or the composition according to any one of items 93 to 97, for the preparation of a medicament for preventing, treating and/or ameliorating a medical condition affecting a subject.
101. A method for preventing, treating and/or ameliorating a medical condition affecting a subject comprising administering a therapeutically or prophylactically effective amount of the compound according to any one of items 1 to 90, the vector according to item 91, the host cell according to item 92, or the composition according to any one of items 93 to 97 to the subject.
102. The compound, vector, host cell, or composition according to item 99, the use according to item 100, or the method according to item 101, wherein the medical condition is characterized by deficient protein expression of the protein encoded by the target mRNA.
103. The compound, vector, host cell, composition, use, or method according to any one of items 99 to 102, wherein the medical condition is selected from the group consisting of haploinsufficiency disorders; cancer; immunological disorders; cardiovascular diseases; respiratory diseases; metabolic diseases; infectious diseases; digestive diseases; and neurological diseases.
104. The compound, vector, host cell, composition, use, or method according to item 103, wherein the medical condition is a haploinsufficiency disorder.
105. The compound, vector, host cell, composition, use, or method according to item 104, wherein the haploinsufficiency disorder and/or neurological disease is selected from the group consisting of palmoplantar keratoderma, punctate type 1A; progressive familial intrahepatic cholestasis type 3;low phospholipid associated cholelithiasis; adrenoleukodystrophy;X-linked cerebral ad renoleu kodystrophy; isolated adrenal insufficiency;adrenomyeloneuropathy; telangiectasia, hereditary hemorrhagic, type 2;hereditary hemorrhagic telangiectasia; ADNP-related multiple congenital anomalies - intellectual disability - autism spectrum disorder; non-syndromic X-linked intellectual disability;FRAXE intellectual disability; AHDC1-related intellectual disability - obstructive sleep apnea - mild dysmorphism syndrome; parietal foramina 2; Brugada syndrome;long QT syndrome;complex neurodevelopmental disorder;catecholaminergic polymorphic ventricular tachycardia; KBG syndrome; syndromic intellectual disability; hypogonadotropic hypogonadism 1 with or without anosmia; X-linked syndromic intellectual disability;syndromic X-linked intellectual disability 5; classic or attenuated familial adenomatous polyposis;gastric adenocarcinoma and proximal polyposis of the stomach;APC-related attenuated familial adenomatous polyposis;classic familial adenomatous polyposis;attenuated familial adenomatous polyposis;familial adenomatous polyposis 1; hypercholesterolemia, autosomal dominant, type B;familial hypobetalipoproteinemia 1;homozygous familial hypercholesterolemia; Kennedy disease;androgen insensitivity syndrome; short stature, rhizomelic, with microcephaly, micrognathia, and developmental delay; X-linked complex neurodevelopmental disorder; Coffin-Siris syndrome;intellectual disability, autosomal dominant 14; Coffin-Siris syndrome;Coffin-Siris syndrome 1; Coffin-Siris syndrome;Coffin-Siris syndrome 6; X-linked chondrodysplasia punctata 1; developmental and epileptic encephalopathy;X-linked complex neurodevelopmental disorder;X-linked lissencephaly with abnormal genitalia; syndromic complex neurodevelopmental disorder;complex neurodevelopmental disorder; Bohring-Opitz syndrome; syndromic intellectual disability;severe feeding difficulties-failure to thrive-microcephaly due to ASXL3 deficiency syndrome; hereditary nonpolyposis colon cancer;hereditary breast carcinoma;familial ovarian cancer;ataxia telangiectasia; hemiplegic migraine-developmental and epileptic encephalopathy spectrum; Menkes disease;X-linked distal spinal muscular atrophy type 3; ATR-X-related syndrome;alpha thalassemia-X-linked intellectual disability syndrome; syndromic intellectual disability;autism spectrum disorder due to AUTS2 deficiency; diabetes insipidus, nephrogenic, X-linked; oligodontia-cancer predisposition syndrome; myofibrillar myopathy;dilated cardiomyopathy;myofibrillar myopathy 6;dilated cardiomyopathy 1HH; BAP1-related tumor predisposition syndrome; hereditary breast carcinoma; hereditary nonpolyposis colon cancer;familial ovarian cancer; severe motor and intellectual disabilities-sensorineural deafness-dystonia syndrome; Dias-Logan syndrome; microphthalmia, syndromic 2; pulmonary arterial hypertension;juvenile polyposis syndrome;generalized juvenile polyposis/juvenile polyposis coli; pulmonary arterial hypertension;congenital heart disease;primary pulmonary hypertension;pulmonary hypertension, primary, 1 ;obsolete idiopathic and/or familial pulmonary arterial hypertension; syndromic intellectual disability; Fanconi anemia, complementation group S;breast-ovarian cancer, familial, susceptibility to, 1hereditary breast ovarian cancer syndrome; Fanconi anemia complementation group D1;breast-ovarian cancer, familial, susceptibility to, 2;Fanconi anemia complementation group A;Fanconi anemia;hereditary breast ovarian cancer syndrome; Fanconi anemia complementation group J;familial ovarian cancer;hereditary breast carcinoma;Fanconi anemia;Fanconi anemia complementation group G; intellectual developmental disorder with dysmorphic facies and ptosis; X-linked syndromic intellectual disability;X-linked intellectual disability; isolated growth hormone deficiency type III;Bruton-type agammaglobulinemia; episodic ataxia type 2; cerebellar dysfunction with variable cognitive and behavioral abnormalities; X-linked syndromic intellectual disability;syndromic X-linked intellectual disability Najm type; hyper-IgM syndrome type 1; hyperparathyroidism 2 with jaw tumors;hyperparathyroidism 1;parathyroid gland carcinoma; CDH1-related diffuse gastric and lobular breast cancer syndrome;familial ovarian cancer;hereditary diffuse gastric adenocarcinoma; CDKL5 disorder;developmental and epileptic encephalopathy, 2; multiple endocrine neoplasia type 4;hereditary nonpolyposis colon cancer; Beckwith-Wiedemann syndrome; melanoma-pancreatic cancer syndrome;familial atypical multiple mole melanoma syndrome;melanoma, cutaneous malignant, susceptibility to, 2; complex neurodevelopmental disorder;developmental and epileptic encephalopathy 94; CHARGE syndrome; complex neurodevelopmental disorder; hereditary nonpolyposis colon cancer;hereditary breast carcinoma;familial ovarian cancer; choroideremia; isolated congenital megalocornea; complex neurodevelopmental disorder; Dent disease type 1; complex neurodevelopmental disorder; X-linked complex neurodevelopmental disorder;intellectual disability, X-linked, syndromic, Houge type; complex neurodevelopmental disorder;holoprosencephaly 12 with or without pancreatic agenesis; osteogenesis imperfecta type 1;osteogenesis imperfecta type 2;osteogenesis imperfecta type 3;combined osteogenesis imperfecta and Ehlers-Danlos syndrome 1;Ehlers-Danlos syndrome, arthrochalasia type;Caffey disease;Ehlers-Danlos syndrome, classic type, 1;osteogenesis imperfecta type 4; spondyloepiphyseal dysplasia, Stanescu type;achondrogenesis type II;platyspondylic dysplasia, Torrance type;spondylometaphyseal dysplasia;spondyloepiphyseal dysplasia congenita;Kniest dysplasia;spondyloperipheral dysplasia;Stickler syndrome type 1spondyloepiphyseal dysplasia with metatarsal shortening; Ehlers-Danlos syndrome, vascular type;autosomal dominant Ehlers-Danlos syndrome, vascular type; Alport syndrome;X-linked Alport syndrome; Ehlers-Danlos syndrome, classic type;Ehlers-Danlos syndrome, classic type, 1; Rubinstein-Taybi syndrome;Rubinstein-Taybi syndrome due to CREBBP mutations; syndromic intellectual disability; hereditary nonpolyposis colon cancer;CTNNA1-related diffuse gastric and lobular breast cancer syndrome;hereditary gastric cancer; severe intellectual disability-progressive spastic diplegia syndrome; complex neurodevelopmental disorder;pseudohypoaldosteronism type 2E;neurodevelopmental disorder with or without autism or seizures; X-linked intellectual disability, Cabezas type; granulomatous disease, chronic, X-linked; Brooke-Spiegler syndrome;frontotemporal dementia and/or amyotrophic lateral sclerosis 8; lissencephaly spectrum disorders;lissencephaly type 1 due to doublecortin gene mutation; X-linked syndromic intellectual disability;intellectual disability, X-linked 102; DDX41-related hematologic malignancy predisposition syndrome; DICER1-related tumor predisposition;pleuropulmonary blastoma;obsolete DICER1 syndrome;rhabdomyosarcoma;goiter, multinodular 1, with or without Sertoli-Leydig cell tumors; non-syndromic X-linked intellectual disability; complex neurodevelopmental disorder; neurodevelopmental disorder with nonspecific brain abnormalities and with or without seizures; progressive muscular dystrophy;dilated cardiomyopathy 3B;Duchenne muscular dystrophy; hypertrophic cardiomyopathy;arrhythmogenic cardiomyopathy with wooly hair and keratoderma;dilated cardiomyopathy;arrhythmogenic right ventricular dysplasia 8;arrhythmogenic right ventricular cardiomyopathy;familial isolated arrhythmogenic right ventricular dysplasia; complex neurodevelopmental disorder;DYRK1A-related intellectual disability syndrome; hypotonia, ataxia, and delayed development syndrome; MEND syndrome;X-linked chondrodysplasia punctata 2; X-linked hypohidrotic ectodermal dysplasia; craniofrontonasal syndrome; mandibulofacial dysostosis-microcephaly syndrome; Kleefstra syndrome;Kleefstra syndrome 1; supravalvular aortic stenosis; telangiectasia, hereditary hemorrhagic, type 1;juvenile polyposis syndrome;hereditary hemorrhagic telangiectasia; Rubinstein-Taybi syndrome due to EP300 haploinsufficiency; craniosynostosis 4; thrombocytopenia 5; exostoses, multiple, type 1; exostoses, multiple, type 2; branchio-oto-renal syndrome;branchiootorenal syndrome 1; nonsyndromic genetic hearing loss;dilated cardiomyopathy 1J;autosomal dominant nonsyndromic hearing loss 10; hemophilia A;hemophilia; hemophilia B;thrombophilia, X-linked, due to factor 9 defect;hemophilia; Fanconi anemia complementation group B; Marfan syndrome;familial thoracic aortic aneurysm and aortic dissection;Shprintzen-Goldberg syndrome; Aarskog-Scott syndrome, X-linked; LADD syndrome; Hartsfield-Bixler-Demyer syndrome;osteoglophonic dwarfism;Pfeiffer syndrome type 1 ;hypogonadotropic hypogonadism 2 with or without anosmia; hereditary leiomyomatosis and renal cell cancer; obsolete Birt-Hogg-Dube syndrome;Birt-Hogg-Dube syndrome 1;Birt-Hogg-Dube syndrome; inherited ichthyosis; familial thoracic aortic aneurysm and aortic dissection;periventricular nodular heterotopia;heterotopia, periventricular, X-linked dominant; myofibrillar myopathy 5;dilated cardiomyopathy;myofibrillar myopathy; fragile X syndrome; anterior segment dysgenesis 3;Axenfeld-Rieger syndrome type 3; lymphedema-distichiasis syndrome; alveolar capillary dysplasia with misalignment of pulmonary veins; FOXG1 disorder; blepharophimosis, ptosis, and epicanthus inversus syndrome; intellectual disability-severe speech delay-mild dysmorphism syndrome;congenital heart disease; nystagmus 1, congenital, X-linked; exudative vitreoretinopathy;exudative vitreoretinopathy 1; GATA2 deficiency with susceptibility to MDS/AML;deafness-lymphedema-leukemia syndrome; hypoparathyroidism-deafness-renal disease syndrome; structural congenital heart disease, multiple types - GATA4;atrial septal defect 2; GATA6-related congenital heart disease with or without pancreatic agenesis or neonatal diabetes;pancreatic hypoplasia-diabetes-congenital heart disease syndrome; severe intellectual disability-poor language-strabismus-grimacing face-long fingers syndrome; GTP cyclohydrolase I deficiency;dopa-responsive dystonia;dystonia 5; brachydactyly type C; inborn glycerol kinase deficiency; Fabry disease; holoprosencephaly 9; Greig cephalopolysyndactyly syndrome; glomuvenous malformation; pseudopseudohypoparathyroidism; Simpson-Golabi-Behmel syndrome;Simpson-Golabi-Behmel syndrome type 1; complex neurodevelopmental disorder; complex neurodevelopmental disorder;intellectual disability, autosomal dominant 6; frontotemporal dementia and/or amyotrophic lateral sclerosis; ; linear skin defects with multiple congenital anomalies 1; Cornelia de Lange syndrome;Cornelia de Lange syndrome 5; intellectual disability, autosomal dominant 43; acute intermittent porphyria; monogenic diabetes;maturity-onset diabetes of the young;maturity-onset diabetes of the young type 3; renal cysts and diabetes syndrome; neurodevelopmental disorder-craniofacial dysmorphism-cardiac defect-hip dysplasia syndrome; synpolydactyly type 1; Lesch-Nyhan syndrome; mucopolysaccharidosis type 2;mucopolysaccharidosis type 2, severe form;mucopolysaccharidosis type 2, attenuated form; growth delay due to insulin-like growth factor I resistance; incontinentia pigmenti;IKBKG-related immunodeficiency with or without ectodermal dysplasia; non-syndromic X-linked intellectual disability;intellectual disability, X-linked 21; X-linked complex neurodevelopmental disorder;intellectual disability, X-linked 1; van der Woude syndrome 1; Alagille syndrome due to a JAG1 point mutation; Koolen-de Vries syndrome; syndromic intellectual disability;autosomal dominant intellectual disability-craniofacial anomalies-cardiac defects syndrome; RASopathy;KAT6B-related multiple congenital anomalies syndrome;blepharophimosis - intellectual disability syndrome, SBBYS type; Brugada syndrome;long QT syndrome;short QT syndrome;familial long QT syndrome;long QT syndrome 2; Jervell and Lange-Nielsen syndrome; hypertrophic cardiomyopathy;long QT syndrome;short QT syndrome;familial long QT syndrome;long QT syndrome 1; neonatal-onset developmental and epileptic encephalopathy;complex neurodevelopmental disorder;neonatal encephalopathy with non-epileptic myoclonus;seizures, benign familial neonatal, 1; X-linked syndromic intellectual disability; Kabuki syndrome 2; microcephaly with or without chorioretinopathy, lymphedema, or intellectual disability; gastrointestinal stromal tumor;piebaldism; Wiedemann-Steiner syndrome; complex neurodevelopmental disorder with motor features;dystonia 28, childhood-onset; syndromic intellectual disability; Kabuki syndrome 1; complex neurodevelopmental disorder;O'Donnell-Luria-Rodan syndrome; complex neurodevelopmental disorder; L1 syndrome;X-linked hydrocephalus with stenosis of the aqueduct of Sylvius; Danon disease; hypercholesterolemia, familial, 1;homozygous familial hypercholesterolemia; Buschke-Ollendorff syndrome; arrhythmogenic right ventricular cardiomyopathy;dilated cardiomyopathy;dilated cardiomyopathy 1A;Emery-Dreifuss muscular dystrophy 2, autosomal dominant;autosomal dominant Emery-Dreifuss muscular dystrophy; Emery-Dreifuss muscular dystrophy; nail-patella syndrome; X-linked intellectual disability;X-linked immunodeficiency with magnesium defect, Epstein-Barr virus infection and neoplasia; hereditary pheochromocytoma-paraganglioma;pheochromocytoma; complex neurodevelopmental disorder;intellectual disability, autosomal dominant 1; Rett syndrome;syndromic X-linked intellectual disability Lubs type; syndromic intellectual disability;congenital heart disease; complex neurodevelopmental disorder;intellectual disability, autosomal dominant 20; syndromic intellectual disability; multiple endocrine neoplasia type 1; X-linked Opitz G/BBB syndrome; Waardenburg syndrome type 2;Waardenburg syndrome type 2A; mismatch repair cancer syndrome 1;Lynch syndrome;hereditary breast carcinoma;Lynch syndrome 2;mismatch repair cancer syndrome; Currarino triad; mismatch repair cancer syndrome 1;Lynch syndrome;hereditary breast carcinoma;Lynch syndrome 1;mismatch repair cancer syndrome 2;mismatch repair cancer syndrome; mismatch repair cancer syndrome 1;Lynch syndrome;hereditary breast carcinoma;Lynch syndrome 5;mismatch repair cancer syndrome 3;mismatch repair cancer syndrome; Basilicata-Akhtar syndrome; tooth agenesis, selective, 1; X-linked myotubular myopathy; dilated cardiomyopathy;arrhythmogenic right ventricular cardiomyopathy;hypertrophic cardiomyopathy;familial hypertrophic cardiomyopathy;hypertrophic cardiomyopathy 4; Feingold syndrome type 1; syndromic intellectual disability;intellectual disability, autosomal dominant 39; syndromic intellectual disability; complex neurodevelopmental disorder; complex neurodevelopmental disorder; Norrie disease; X-linked complex neurodevelopmental disorder;X-linked intellectual disability, Cantagrel type; neurofibromatosis type 1;familial ovarian cancer; neurofibromatosis type 2; brain malformations with or without urinary tract defects;chromosome 1p32-p31 deletion syndrome; Marshall-Smith syndrome;Malan overgrowth syndrome; Nance-Horan syndrome; Cornelia de Lange syndrome;Cornelia de Lange syndrome 1; dilated cardiomyopathy;NKX2.5-related congenital, conduction and myopathic heart disease; NOG-related symphalangism spectrum disorder;multiple synostoses syndrome 1; focal epilepsy; X-linked adrenal hypoplasia congenita; Bosch-Boonstra-Schaaf optic atrophy syndrome; autosomal dominant pseudohypoaldosteronism type 1;pseudohyperaldosteronism type 2; complex neurodevelopmental disorder; 46,XY sex reversal 3; complex neurodevelopmental disorder; Sotos syndrome; syndromic intellectual disability; CK syndrome;CHILD syndrome; complex neurodevelopmental disorder; NYX-related retinopathy;congenital stationary night blindness 1A; oculocerebrorenal syndrome; ciliopathy;orofaciodigital syndrome I; X-linked intellectual disability-cerebellar hypoplasia syndrome; ornithine carbamoyltransferase deficiency; syndromic microphthalmia type 5; classic lissencephaly; focal segmental glomerulosclerosis 7;renal coloboma syndrome; Waardenburg syndrome;Waardenburg syndrome type 1; PAX6-related ocular dysgenesis;aniridia 1; tooth agenesis, selective, 3; congenital anomalies of kidney and urinary tract syndrome with or without hearing loss, abnormal ears, or developmental delay; X-linked complex neurodevelopmental disorder; Leigh syndrome;pyruvate dehydrogenase deficiency; X-linked hypophosphatemic rickets;X-linked dominant hypophosphatemic rickets; complex neurodevelopmental disorder; Borjeson-Forssman-Lehmann syndrome; syndromic X-linked intellectual disability Siderius type; PHIP-related behavioral problems-intellectual disability-obesity-dysmorphic features syndrome; Axenfeld-Rieger syndrome; autosomal dominant polycystic kidney disease;autosomal recessive polycystic kidney disease;polycystic kidney disease 1; autosomal dominant polycystic kidney disease;polycystic kidney disease 2; Brugada syndrome 1;arrhythmogenic right ventricular cardiomyopathy;dilated cardiomyopathy;catecholaminergic polymorphic ventricular tachycardia;arrhythmogenic right ventricular dysplasia 9;familial isolated arrhythmogenic right ventricular dysplasia; Pelizeaus-Merzbacher spectrum disorder; Charcot-Marie-Tooth disease type 1A;hereditary neuropathy with liability to pressure palsies;Charcot-Marie-Tooth disease type 1;Charcot-Marie-Tooth disease type 1E; mismatch repair cancer syndrome 1;Lynch syndrome;hereditary breast carcinoma;Lynch syndrome 4;mismatch repair cancer syndrome;mismatch repair cancer syndrome 4; intellectual disability-microcephaly-strabismus-behavioral abnormalities syndrome; Treacher Collins syndrome 2; focal dermal hypoplasia; nonsyndromic genetic hearing loss;autosomal dominant nonsyndromic hearing loss 15; Renpenning syndrome; PRPS1 deficiency disorder;phosphoribosylpyrophosphate synthetase superactivity; neuroocular syndrome; infantile convulsions and choreoathetosis;self-limited familial infantile epilepsy; nevoid basal cell carcinoma syndrome; non-syndromic X-linked intellectual disability;autism, susceptibility to, X-linked 4; PTEN hamartoma tumor syndrome;multiple exostoses with spastic tetraparesis; brachydactyly type E2;mesomelic dysplasia; Noonan syndrome with multiple lentigines;cardiofaciocutaneous syndrome;Costello syndrome;Noonan syndrome;LEOPARD syndrome 2;LEOPARD syndrome 3;LEOPARD syndrome 1;Noonan syndrome 1;metachondromatosis; syndromic intellectual disability; complex neurodevelopmental disorder;PURA-related severe neonatal hypotonia-seizures-encephalopathy syndrome due to a point mutation; syndromic intellectual disability; early-onset parkinsonism-intellectual disability syndrome;intellectual disability, X-linked 72; Cornelia de Lange syndrome; hereditary breast carcinoma;Fanconi anemia complementation group O;familial ovarian cancer;breast-ovarian cancer, familial, susceptibility to, 3; hereditary breast carcinoma;breast-ovarian cancer, familial, susceptibility to, 4;familial ovarian cancer; Smith-Magenis syndrome; Noonan syndrome;capillary malformation-arteriovenous malformation 1; retinoblastoma;hereditary retinoblastoma; multiple endocrine neoplasia type 2A;multiple endocrine neoplasia type 2B;familial medullary thyroid carcinoma;Hirschsprung disease, susceptibility to, 1; RP2-related retinopathy;retinitis pigmentosa 2; Diamond-Blackfan anemia 4; Diamond-Blackfan anemia;Diamond-Blackfan anemia 1; Diamond-Blackfan anemia;Diamond-Blackfan anemia 3; Diamond-Blackfan anemia 10; Coffin-Lowry syndrome; X-linked retinoschisis; hereditary thrombocytopenia and hematologic cancer predisposition syndrome;hereditary thrombocytopenia and hematological cancer predisposition syndrome associated with RUNX1;microcephaly, corpus callosum dysgenesis, and cleft lip/palate; Duane-radial ray syndrome; SATB2 associated disorder; generalized epilepsy with febrile seizures plus;Dravet syndrome;developmental and epileptic encephalopathy;familial hemiplegic migraine;generalized epilepsy with febrile seizures plus, type 2;developmental and epileptic encephalopathy, 6; complex neurodevelopmental disorder; Brugada syndrome;arrhythmogenic right ventricular cardiomyopathy;dilated cardiomyopathy;familial long QT syndrome;catecholaminergic polymorphic ventricular tachycardia;short QT syndrome;Brugada syndrome 1;long QT syndrome 3;long QT syndrome; hereditary pheochromocytoma-paraganglioma;paraganglioma;paragangliomas 2; hereditary pheochromocytoma-paraganglioma;mitochondrial disease;paraganglioma;pheochromocytoma;paragangliomas 4; hereditary pheochromocytomaparaganglioma;mitochondrial disease;paragangliomas 3;paraganglioma; hereditary pheochromocytoma-paraganglioma;mitochondrial disease;pheochromocytoma;paraganglioma;paragangliomas 1; complex neurodevelopmental disorder;Schinzel-Giedion syndrome;intellectual disability, autosomal dominant 29; schizophrenia; SETD2-related neurodevelopmental disorder without or with macrocephaly/overgrowth;SETD2-related microcephaly-severe intellectual disability-multiple congenital anomalies syndrome;complex neurodevelopmental disorder; syndromic complex neurodevelopmental disorder;intellectual disability-facial dysmorphism syndrome due to SETD5 haploinsufficiency; SF3B4-related acrofacial dysostosis;Nager acrofacial dysostosis; myoclonic dystonia 11; X-linked lymphoproliferative disease due to SH2D1A deficiency; complex neurodevelopmental disorder; Phelan-McDermid syndrome; holoprosencephaly 3; Leri-Weill dyschondrosteosis; SIN3A-related intellectual disability syndrome;chromosome 15q24 deletion syndrome; holoprosencephaly 2; Allan-Herndon-Dudley syndrome; GLUT1 deficiency syndrome;childhood onset GLUT1 deficiency syndrome 2; X-linked complex neurodevelopmental disorder;SLC35A2-congenital disorder of glycosylation; complex neurodevelopmental disorder; creatine transporter deficiency;cerebral creatine deficiency syndrome; Christianson syndrome; aneurysm-osteoarthritis syndrome;familial thoracic aortic aneurysm and aortic dissection;Loeys-Dietz syndrome; juvenile polyposis/hereditary hemorrhagic telangiectasia syndrome;Myhre syndrome;pulmonary arterial hypertension juvenile polyposis syndrome;generalized juvenile polyposis/juvenile polyposis coli; rhabdoid tumor predisposition syndrome 1;Coffin-Siris syndrome;familial rhabdoid tumor; X-linked complex neurodevelopmental disorder;Cornelia de Lange syndrome 2; syndromic X-linked intellectual disability Snyder type; ZTTK syndrome; Waardenburg syndrome type 4C; intellectual disability, autosomal dominant 27; anophthalmia/microphthalmia-esophageal atresia syndrome; Lamb-Shaffer syndrome; campomelic dysplasia;isolated Pierre-Robin syndrome;Cooks syndrome; hereditary spastic paraplegia 4; hereditary chronic pancreatitis; Legius syndrome; complex neurodevelopmental disorder; 46,XY sex reversal 1; X-linked syndromic intellectual disability; familial ovarian cancer;Peutz-Jeghers syndrome; recessive X-linked ichthyosis; developmental and epileptic encephalopathy;developmental and epileptic encephalopathy, 4; X-linked complex neurodevelopmental disorder; SYNCRIP-related neurodevelopmental disorder; complex neurodevelopmental disorder; syndromic intellectual disability; complex neurodevelopmental disorder;intellectual disability, autosomal dominant 41; complex neurodevelopmental disorder; cleft palate with or without ankyloglossia, X-linked; ulnar-mammary syndrome; pulmonary arterial hypertension;coxopodopatellar syndrome; Holt-Oram syndrome; TCF12-related craniosynostosis; developmental delay with variable intellectual impairment and behavioral abnormalities;complex neurodevelopmental disorder; Pitt-Hopkins syndrome; Treacher-Collins syndrome;Treacher Collins syndrome 1; Char syndrome; Loeys-Dietz syndrome;multiple self-healing squamous epithelioma;familial thoracic aortic aneurysm and aortic dissection;Loeys-Dietz syndrome 1; holoprosencephaly 4; deafness dystonia syndrome; hereditary pheochromocytoma-paraganglioma;pheochromocytoma; complex neurodevelopmental disorder; Li-Fraumeni syndrome; spondyloepiphyseal dysplasia tarda, X-linked; syndromic intellectual disability;micrognathia-recurrent infections-behavioral abnormalities-mild intellectual disability syndrome; complex neurodevelopmental disorder;Clark-Baraitser syndrome; trichorhinophalangeal syndrome type I; tuberous sclerosis;tuberous sclerosis 1; tuberous sclerosis;tuberous sclerosis 2; dilated cardiomyopathy;hypertrophic cardiomyopathy;arrhythmogenic right ventricular cardiomyopathy;tibial muscular dystrophy;myopathy, myofibrillar, 9, with early respiratory failure;TTN-related myopathy;dilated cardiomyopathy 1G; Saethre-Chotzen syndrome;Sweeney-Cox syndrome;TWIST1-related craniosynostosis; syndromic X-linked intellectual disability Nascimento type; Angelman syndrome; X-linked complex neurodevelopmental disorder;syndromic X-linked intellectual disability 14; Hao-Fountain syndrome; X-linked syndromic intellectual disability;intellectual disability, X-linked 99; von Hippel-Lindau disease; DeSanto-Shinawi syndrome;DeSanto-Shinawi syndrome due to WAC point mutation; syndromic intellectual disability;complex neurodevelopmental disorder; Skraban-Deardorff syndrome; X-linked complex neurodevelopmental disorder;neurodegeneration with brain iron accumulation 5; Wilms tumor 1;Denys-Drash syndrome; X-linked lymphoproliferative disease due to XIAP deficiency; X inactivation, familial skewed, 1; complex neurodevelopmental disorder;intellectual disability, autosomal dominant 22; X-linked syndromic intellectual disability;Wieacker-Wolff syndrome; syndromic X-linked intellectual disability Raymond type; Mowat-Wilson syndrome; holoprosencephaly; heterotaxy, visceral, 1, X-linked; syndromic complex neurodevelopmental disorder; and weiss-kruszka syndrome.
106. The compound, vector, host cell, composition, use, or method according to item 104 or 105, wherein the haploinsufficiency disorder and/or neurological disease is caused by haploinsufficiency of one or more genes selected from the group consisting of: *AAGAB; ABCB4; ABCD1; ACVRL1; ADNP; AFF2; AHDC1; ALX4; ANK2; ANKRD11; ANKRD17; ANOS1; AP1S2; APC; APOB; AR; ARCN1; ARHGEF9; ARID1A; ARID1B; ARID2; ARSL; ARX; ASH1L; ASXL1; ASXL3; ATM; ATP1A2; ATP7A; ATRX; AUTS2; AVPR2; AXIN2; BAG3; BAP1; BARD1; BCAP31; BCL11A; BCOR; BMPR1A; BMPR2; BPTF; BRCA1; BRCA2; BRIP1; BRPF1; BRWD3; BTK; CACNA1A; CAMTA1; CASK; CD40LG;* CDC73; *CDH1; CDKL5; CDKN1B; CDKN1C; CDKN2A; CHD2; CHD7; CHD8; CHEK2; CHM; CHRDL1; CIC; CLCN5; CLTC; CNKSR2; CNOT1; COL1A1; COL2A1; COL3A1; COL4A5; COL5A1; CREBBP; CTCF; CTNNA1; CTNNB1; CUL3; CUL4B; CYBB; CYLD; DCX; DDX3X; DDX41; DICER1; DLG3; DLG4; DLL1; DMD; DSP; DYRK1A; EBF3; EBP; EDA; EFNB1; EFTUD2; EHMT1; ELN; ENG; EP300; ERF; ETV6; EXT1; EXT2; EYA1; EYA4; F8; F9; FANCB; FBN1; FGD1; FGF10; FGFR1; FH; FLCN; FLG; FLNA; FLNC; FMR1; FOXC1; FOXC2; FOXF1; FOXG1; FOXL2; FOXP1; FRMD7; FZD4; GATA2; GATA3; GATA4; GATA6; GATAD2B; GCH1; GDF5; GK; GLA; GLI2; GLI3; GLMN; GNAS; GPC3; GRIN2A; GRIN2B; GRN; HCCS; HDAC8; HIVEP2; HMBS; HNF1A; HNF1B; HNRNPK; HOXD13; HPRT1; IDS; IGF1R; IKBKG; IL1RAPL1; IQSEC2; IRF6; JAG1; KANSL1; KAT6A; KAT6B; KCNH2; KCNQ1; KCNQ2; KDM5C; KDM6A; KIF11; KIT; KMT2A; KMT2B; KMT2C; KMT2D; KMT2E; KMT5B; L1CAM; LAMP2; LDLR; LEMD3; LMNA; LMX1B; MAGT1; MAX; MBD5; MECP2; MED13L; MEF2C; MEIS2; MEN1; MID1; MITF; MLH1; MNX1; MSH2; MSH6; MSL3; MSX1; MTM1; MYBPC3; MYCN; MYT1L; NAA15; NBEA; NCKAP1; NDP; NEXMIF; NF1; NF2; NFIA; NFIX; NHS; NIPBL; NKX2-5; NOG; NPRL3; NR081; NR2F1; NR3C2; NR4A2; NR5A1; NRXN1; NSD1; NSD2; NSDHL; NUS1; NYX; OCRL; OFD1; OPHN1; OTC; OTX2; PAFAH1B1; PAX2; PAX3; PAX6; PAX9; PBX1; PCDH19; PDHA1; PHEX; PHF21A; PHF6; PHF8; PHIP; PITX2; PKD1; PKD2; PKP2; PLP1; PMP22; PMS2; POGZ; POLR1D; PORCN; POU4F3; PQBP1; PRPS1; PRR12; PRRT2; PTCH1; PTCHD1; PTEN; PTHLH; PTPN11; PUF60; PURA; QRICH1; RAB39B; RAD21; RAD51C; RAD51D; RAI1; RASA1; RB1; RET; RP2; RPS17; RPS19; RPS24; RPS26; RPS6KA3; RS1; RUNX1; SALL4; SATB2; SCN1A; SCN2A; SCN5A; SDHAF2; SDHB; SDHC; SDHD; SETBP1; SETD1A; SETD2; SETD5; SF3B4; SGCE; SH2D1A; SHANK2; SHANK3; SHH; SHOX; SIN3A; SIX3; SLC16A2; SLC2A1; SLC35A2; SLC6A1; SLC6A8; SLC9A6; SMAD3; SMAD4; SMARCB1; SMC1A; SMS; SON; SOX10; SOX11; SOX2; SOX5; SOX9; SPAST; SPINK1; SPRED1; SRRM2; SRY; STAG2; STK11; STS; STXBP1; SYN1; SYNCRIP; SYNGAP1; TAOK1; TBL1XR1; TBR1; TBX22; TBX3; TBX4; TBX5; TCF12; TCF20; TCF4; TCOF1; TFAP2B; TGFBR1; TGIF1; TIMM8A; TMEM127; TNRC6B; TP53; TRAPPC2; TRIO; TRIP12; TRPS1; TSC1; TSC2; TTN; TWIST1; UBE2A; UBE3A; UPF3B; USP7; USP9X; VHL; WAC; WDFY3; WDR26; WDR45; WT1; XIAP; XIST; ZBTB18; ZC4H2; ZDHHC9; ZEB2; ZIC2; ZIC3; ZMYND11; and ZNF462.*
107. The compound, vector, host cell, composition, use, or method according to any one of items 99 to 106, wherein preventing, treating and/or ameliorating a medical condition affecting a subject further comprises the administration of one or more additional therapeutic agents, such as agonist therapeutic agents.
108. The compound, vector, host cell, composition, use, or method according to any one of items 99 to 107, wherein the preventing, treating and/or ameliorating a medical condition affecting a subject comprises administering a therapeutically or prophylactically effective amount of the compound according to any one of items 1 to 90, the vector according to item 91, the host cell according to item 92, or the composition according to any one of items 93 to 97 to the subject affected by the medical condition.
109. A method of modulating the stability of a target mRNA, such as increasing the stability of a target mRNA, the method comprising the step of contacting the mRNA with a compound according to any one of items 1 to 90, a vector according to item 91, a host cell according to item 92, or a composition according to any one of items 93 to 97.
110. A method of increasing or upregulating the expression of a target mRNA, the method comprising the step of contacting the mRNA with a compound according to any one of items 1 to 90, a vector according to item 91, a host cell according to item 92, or a composition according to any one of items 93 to 97.
111. The method according to item 110, wherein the expression of the target mRNA is increased or upregulated by at least 10, 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 350, 400, 450, 500, 550, 600, 650, 700, 750, 800, 850, 900, 950, or at least 1000%, compared to a control.
112. A method of increasing or upregulating the expression of a target protein expressed by a target mRNA, the method comprising the step of contacting the mRNA with a compound according to any one of items 1 to 90, a vector according to item 91, a host cell according to item 92, or a composition according to any one of items 93 to 97.
113. The method according to item 112, wherein the expression of the target protein is increased or upregulated by by at least 10, 15, 20, 25, 30, 35, 40, 45, 50, 55, 60, 65, 70, 75, 80, 85, 90, 95, 100, 110, 120, 130, 140, 150, 160, 170, 180, 190, or 200%, compared to a control.
114. The compound according to item 111 or 113, wherein the control is a cell that has not been exposed to the compound.
115. The method according to any one of items 109 to 114, wherein the target mRNA originates from the transcription of any one of the genes listed in item 106.
116. The method according to any one of items 109 to 115, wherein the target protein originates from the transcription of any one of the genes listed in item 106.
117. The method according to any one of items 109 to 116, wherein the method is *in vitro* method.
118. The method according to any one of items 109 to 116, wherein the method is *in vivo* method.
119. A kit comprising the compound according to any one of items 1 to 90, the vector according to item 91, the host cell according to item 92, or the composition according to any one of items 93 to 97
120. A kit comprising the compound according to any one of items 1 to 90, the vector according to item 91, the host cell according to item 92, or the composition according to any one of items 93 to 97 and instructions for use.
121. The kit according to item 119 or 120, further comprising one or more additional therapeutic agents, such as agonist therapeutic agents.

### SEQUENCES

**SEQ ID NO: 1**
   FBL (rRNA 2'-O-methyltransferase fibrillarin [Homo sapiens]; NP_001427.2) 
MKPGFSPRGGGFGGRGGFGDRGGRGGRGGFGGGRGRGGGFRGRGRGGGGGGGGGGGGGR
GGGGFHSGGNRGRGRGGKRGNQSGKNVMVEPHRHEGVFICRGKEDALVTKNLVPGESVYGEKR
VSISEGDDKIEYRAWNPFRSKLAAAILGGVDQIHIKPGAKVLYLGAASGTTVSHVSDIVGPDGLVYAVE
FSHRSGRDLINLAKKRTNIIPVIEDARHPHKYRMLIAMVDVIFADVAQPDQTRIVALNAHTFLRNGGHF
VISIKANCIDSTASAEAVFASEVKKMQQENMKPQEQLTLEPYERDHAVVVGVYRPPPKVKN
**SEQ ID NO: 2**
   NOP56 (nucleolar protein 56 [Homo sapiens]; NP_006383.2) 
MVLLHVLFEHAVGYALLALKEVEEISLLQPQVEESVLNLGKFHSIVRLVAFCPFASSQVALENANAVSE
GVVHEDLRLLLETHLPSKKKKVLLGVGDPKIGAAIQEELGYNCQTGGVIAEILRGVRLHFHNLVKGLT
DLSACKAQLGLGHSYSRAKVKFNVNRVDNMIIQSISLLDQLDKDINTFSMRVREWYGYHFPELVKIIN
DNATYCRLAQFIGNRRELNEDKLEKLEELTMDGAKAKAILDASRSSMGMDISAIDLINIESFSSRVVSL
SEYRQSLHTYLRSKMSQVAPSLSALIGEAVGARLIAHAGSLTNLAKYPASTVQILGAEKALFRALKTR
GNTPKYGLIFHSTFIGRAAAKNKGRISRYLANKCSIASRIDCFSEVPTSVFGEKLREQVEERLSFYET
GEIPRKNLDVMKEAMVQAEEAAAEITRKLEKQEKKRLKKEKKRLAALALASSENSSSTPEECEEMSE
KPKKKKKQKPQEVPQENGMEDPSISFSKPKKKKSFSKEELMSSDLEETAGSTSIPKRKKSTPKEETV
NDPEEAGHRSGSKKKRKFSKEEPVSSGPEEAVGKSSSKKKKKFHKASQED
**SEQ ID NO: 3**
   NOP58 (nucleolar protein 58 [Homo sapiens]; NP_057018.1) 
MLVLFETSVGYAIFKVLNEKKLQEVDSLWKEFETPEKANKIVKLKHFEKFQDTAEALAAFTALMEGKIN
KQLKKVLKKIVKEAHEPLAVADAKLGGVIKEKLNLSCIHSPVVNELMRGIRSQMDGLIPGVEPREMAA
MCLGLAHSLSRYRLKFSADKVDTMIVQAISLLDDLDKELNNYIMRCREWYGWHFPELGKIISDNLTYC
KCLQKVGDRKNYASAKLSELLPEEVEAEVKAAAEISMGTEVSEEDICNILHLCTQVIEISEYRTQLYEY
LQNRMMAIAPNVTVMVGELVGARLIAHAGSLLNLAKHAASTVQILGAEKALFRALKSRRDTPKYGLIY
HASLVGQTSPKHKGKISRMLAAKTVLAIRYDAFGEDSSSAMGVENRAKLEARLRTLEDRGIRKISGT
GKALAKTEKYEHKSEVKTYDPSGDSTLPTCSKKRKIEQVDKEDEITEKKAKKAKIKVKVEEEEEEKVA
EEEETSVKKKKKRGKKKHIKEEPLSEEEPCTSTAIASPEKKKKKKKRENED
**SEQ ID NO: 4**
   SNU13/NHP2L1 (NHP2-like protein 1 [Homo sapiens]; NP_001003796.1)

MTEADVNPKAYPLADAHLTKKLLDLVQQSCNYKQLRKGANEATKTLNRGISEFIVMAADAEPLEIILHL
PLLCEDKNVPYVFVRSKQALGRACGVSRPVIACSVTIKEGSQLKQQIQSIQQSIERLLV

### Control

**SEQ ID NO: 5** snoRD22 (endogenous box C/D snoRNA; positive control; NR 000008.2)

5'-UCCCAAUGAAGAAACUUUCACAUGUCUUACUCUCUGUCCUAGUCCCAGAGCCUGUAAAGG
UGAACCCACUGGGACUGGCUGGGGGAGAAGAGGAAGAUUUGUUCCAGAAGGAACUGUCUGA
GGGAU-3'

### Scaffolds S1

**SEQ ID NO: 6**
   S1 (Smart_S1) 
5'-G GAUCACG GUGAUGA GAGCUCGGUACCU CUGA CCUCAG GCCU CUGAGG GGUGAUGA
ACUUGUUGAUGGU CUGA GUGAUC A-3'
**SEQ ID NO: 7**
   S1_D'_pU (smart_S1 with polyU-stretch as the D'-guide antisense element) 
5'-GAUCACG GUGAUGA GAUUUUUUUUUUU CUGA CCUCAG GCCU CUGAGG GGUGAUGA
ACUUGUUGAUGGU CUGA GUGAUC-3'
**SEQ ID NO: 8**
   S1_D_pU (smart_S1 with polyU-stretch as the D-guide antisense element) 
5'-GAUCACG GUGAUGA GAGCUCGGUACCU CUGA CCUCAG GCCU CUGAGG GGUGAUGA
ACUUUUUUUUUUU CUGA GUGAUC-3'
**SEQ ID NO: 9**
   S1_D'_pU_longD (smart_S1 with polyU-stretch as the D'-guide antisense element and an extended D-guide antisense element) 
5'-GAUCACG GUGAUGA GAUUUUUUUUUUU CUGA CCUCAG GCCU CUGAGG GGUGAUGA
ACUCGAGGCGGAUUCACCCUUGU CUGA GUGAUC-3'
**SEQ ID NO: 10**
   S1_D_pU_longD' (smart_S1 with polyU-stretch as the D-guide antisense element and an extended D'-guide antisense element)

5'-GAUCACG GUGAUGA GAUCGAGGCGGAUUCACCCUUGU CUGA CCUCAG GCCU CUGAGG
GGUGAUGA ACUUUUUUUUUUU CUGA GUGAUC-3'

### Scaffolds S1 - Controls

**SEQ ID NO: 11**
   S1_D'_pC (smart_S1 with polyC-stretch as the D'-guide antisense element) 
5'-GAUCACG GUGAUGA GACCCCCCCCCCU CUGA CCUCAG GCCU CUGAGG GGUGAUGA
ACUUGUUGAUGGU CUGA GUGAUC-3'
**SEQ ID NO: 12**
   S1_D_pC (smart_S1 with polyC-stretch as the D-guide antisense element) 
5'-GAUCACG GUGAUGA GAGCUCGGUACCU CUGA CCUCAG GCCU CUGAGG GGUGAUGA
ACCCCCCCCCCCU CUGA GUGAUC-3'
**SEQ ID NO: 13**
   S1_D'_pR (smart_S1 with a randomized nucleotide stretch as the D'-guide antisense element) 
5'-GAUCACG GUGAUGA GAACUGAUCGAGU CUGA CCUCAG GCCU CUGAGG GGUGAUGA
ACUUGUUGAUGGU CUGA GUGAUC-3'
**SEQ ID NO: 14**
   S1_D_pR (smart_S1 with a randomized nucleotide stretch as the D-guide antisense element) 
5'-GAUCACG GUGAUGA GAGCUCGGUACCU CUGA CCUCAG GCCU CUGAGG GGUGAUGA
ACACUGAUCGAGU CUGA GUGAUC-3'
**SEQ ID NO: 15**
   S1_CC'mut (smart_S1 with C- and C'-box mutated; mutations indicated in lower case; negative control) 
5'-G GAUCACG GUccUGA GAGCUCGGUACCU CUGA CCUCAG GCCU CUGAGG GGUccUGA
ACUUGUUGAUGGU CUGA GUGAUC A-3'
**SEQ ID NO: 16**
   S1_Dmut (smart_S1 with D-box mutated; mutations indicated in lower case; negative control) 
5'-G GAUCACG GUGAUGA GAGCUCGGUACCU CUGA CCUCAG GCCU CUGAGG GGUGAUGA
ACUUGUUGAUGGU CUcc GUGAUC A-3'
**SEQ ID NO: 17**
   S1_DD'mut (mutated nucleotides indicated by lowercase letters; negative control)

GAUCACG GUGAUGA GAGCUCGGUACCU gUcg CCUCAG GCCU CUGAGG GGUGAUGA
ACUUGUUGAUGGU gUcg GUGAUC

### Other sequences

**SEQ ID NO: 18** 5' of smart_D'pU_scaffold
   GAUCACG GUGAUGA GAUUUUUUUUUUU CUGA CCUCAG GCCU CUGAGG GGUGAUGA
**SEQ ID NO: 19**
   3' of smart_D'pU_scaffold
   U CUGA GUGAUC
**SEQ ID NO: 20**
   5' of smart_DpU_scaffold
   GAUCACG GUGAUGA
**SEQ ID NO: 21**
   3' of smart_DpU_scaffold
   U CUGA CCUCAG GCCU CUGAGG GGUGAUGA GAUUUUUUUUUUU CUGA GUGAUC
**SEQ ID NO: 22**
   Cy3-conjugated substrate-pA₆₀ no methyl (control) 
GUCCUUUCCUAAUAAAAUGAGGAAAUUGCAUCGCAUUGGGAAAAAAAAAAAAAAAAAAAAA
AAAAAAAAAAAAAAAAAAAAAAAAAAAAAAAAAAAAAAAAA
**SEQ ID NO: 23**
   Cy5-conjugated substrate-pA₆₀ no methyl 
GUCCUUUCCUAAUAAAAUGAGGAAAUUGCAUCGCAUUGGGAAAAAAAAAAAAAAAAAAAAA
AAAAAAAAAAAAAAAAAAAAAAAAAAAAAAAAAAA
**SEQ ID NO: 24**
   Cy5-conjugated substrate-pA₆₀ methyl @ 60 
GUCCUUUCCUAAUAAAAUGAGGAAAUUGCAUCGCAUUGGAAAAAAAAAAAAAAAAAAAA
AAAAAAAAAAAAAAAAAAAAAAAAAAAAAAAAA(m)A
**SEQ ID NO: 25**
   Cy5-conjugated substrate-pA₆₀ methyl @ 30 
GUCCUUUCCUAAUAAAAUGAGGAAAUUGCAUCGCAUUGGGAAAAAAAAAAAAAAAAAAAAAAA
AAAAAA(m)AAAAAAAAAAAAAAAAAAAAAAAAAAAAAAA
**SEQ ID NO: 26**
   Cy5-conjugated substrate-pA₆₀ methylated @6 at random 
GUCCUUUCCUAAUAAAUGAGGAAAUUGCAUCGCAUUGGAAAAA(m)AAAAAAAAAAAAAAAAAAA
AAAAAAAAAAAAAAAAAAAAAAAAAAAAAAAAAAAAAAAA
**SEQ ID NO: 27**
   Cy5-conjugated substrate-pA₆₀ methylated @16 at random 
GUCCUUUCCUAAUAAAAUGAGGAAAUUGCAUCGCAUUGGGAAAAAAAAAAAAAAAAAAA(m)AAAAAA
AAAAAAAAAAAAAAAAAAAAAAAAAAAAAAAAAAAAAAAAA
**SEQ ID NO: 28**
   Cy5-conjugated substrate-pA₆₀ methylated @26 at random 
GUCCUUUCCUAAUAAAAUGAGGAAAUUGCAUCGCAUUGGGAAAAAAAAAAAAAAAAAAAAAAAAAA

AA(m)AAAAAAAAAAAAAAAAAAAAAAAAAAAAAAAAAAAA
**SEQ ID NO: 29**
   Cy5-conjugated substrate-pA₆₀ methylated @36 at random 
GUCCUUUCCUAAUAAAAUGAGGAAAUUGCAUCGCAUUGGAAAAAAAAAAAAAAAAAAAAA
AAAAAAAAAAAA(m)AAAAAAAAAAAAAAAAAAAAAAAAA
**SEQ ID NO: 30**
   Cy5-conjugated substrate-pA₆₀ methylated @46 at random 
GUCCUUUCCUAAUAAAAUGAGGAAAUUGCAUCGCAUUGGGAAAAAAAAAAAAAAAAAAAAAAAA
AAAAAAAAAAAAAAAAAAAAAA(m)AAAAAAAAAAAAAAA
**SEQ ID NO: 31**
   Cy5-conjugated substrate-pA₆₀ methylated @56 at random 
GUCCUUUCCUAAUAAAAUGAGGAAAUUGCAUCGCAUUGGGAAAAAAAAAAAAAAAAAAAAAAAAAAAAAAAAA
AAAAAAAAAAAAAAAAAAAAAAAAAAAAAAAAAAAA(m)AAAAA
**SEQ ID NO: 32**
   20mer body RNA
   GGAAAUUGCAUCGCAUUGGG
**SEQ ID NO: 33**
   20mer RNA oligo conjugated to a Cy5-group at the 5'-end
   GUCCUUUCCUAAUAAAAUGA

## Claims

1. A compound comprising a small artificial RNA (smartRNA) oligonucleotide comprising or consisting of, from 5' to 3':
- a "5' stem I" nucleotide sequence comprising a "5' stem I" region of complementarity;
- a "C box" nucleotide sequence comprising a "C box" region of complementarity;
- a "D' guide" nucleotide sequence comprising a "D' guide" region of complementarity;
- a "D' box" nucleotide sequence comprising a "D' box" region of complementarity
- a "5' stem II" nucleotide sequence comprising a "5' stem II" region of complementarity;
- optionally a "loop" nucleotide sequence;
- a "3' stem II" nucleotide sequence comprising a "3' stem II" region of complementarity;
- a "C' box" nucleotide sequence comprising a "C' box" region of complementarity
- a "D guide" nucleotide sequence comprising a "D guide" region of complementarity;
- a "D box" nucleotide sequence comprising a "D box" region of complementarity;
- a "3' stem I" nucleotide sequence comprising a "3' stem I" region of complementarity;
wherein the "5' stem I" and the "3' stem I" regions of complementarity are at least partially complementary to each other and thereby can anneal at least partially to each other to form an at least partially double-stranded "stem I" secondary structure;
wherein the "C box" and the "D box" regions of complementarity are at least partially complementary to each other and thereby can anneal at least partially to each other to form an at least partially double-stranded "C/D box" secondary structure;
wherein the "D' box" and the "C' box" regions of complementarity are at least partially complementary to each other and thereby can anneal at least partially to each other to form an at least partially double-stranded "C'/D' box" secondary structure;
wherein the "5' stem II" and the 3' stem II" regions of complementarity are at least partially complementary to each other and thereby can anneal at least partially to each other to form an at least partially double-stranded "stem II" secondary structure;
wherein the "D guide" region of complementarity comprises or consist of a first contiguous nucleotide sequence which is complementary to a same-length portion of a first target nucleic acid sequence of a target mRNA;
wherein the "D' guide" region of complementarity comprises or consist of a second contiguous nucleotide sequence which is complementary to a same-length portion of a second target nucleic acid sequence of the same target mRNA; and
wherein the first or second target nucleic acid sequence comprises the polyA-tail of said target mRNA.

2. The compound according to claim 1, wherein:
- the "stem I" nucleotide sequences and/or regions of complementarity are independently between 0 and 30 nucleotides in length;
- the "C box" and "D box" nucleotide sequences and/or regions of complementarity are independently between 0 and 30 nucleotides in length;
- the "D' box" and "C' box" nucleotide sequences and/or regions of complementarity are independently between 1 and 30 nucleotides in length;
- the "stem II" nucleotide sequences and/or regions of complementarity are independently between 0 and 30 nucleotides in length; and/or
- the "loop" nucleotide sequence is between 1 and 30 nucleotides in length;
and wherein:
- the "stem I" regions of complementarity are at least 70% complementary to each other;
- the "C box" and the "D box" regions of complementarity are at least 70% complementary to each other;
- the "D' box" and the "C' box" regions of complementarity are at least 70% complementary to each other; and/or
- the "stem II" regions of complementarity are at least 70% complementary to each other.

3. The compound according to any of the preceding claims, wherein the first contiguous nucleotide sequence of the "D guide" region of complementarity and/or the second contiguous nucleotide sequence of the "D' guide" region of complementarity are independently between 7 and 30 nucleotides in length and comprises a homo-polymeric sequence.

4. The compound according to claim 3, wherein the homo-polymeric sequence is a poly-uridine sequence of at least 7 contiguous uridines, preferably between 7 and 12 uridines.

5. The compound according to any one of the preceding claims, wherein the smartRNA comprises at least one modified nucleotide.

6. The compound according to any one of the preceding claims, wherein the smartRNA comprises at least one modified internucleoside linkage.

7. The compound according to any one of the preceding claims, wherein the smartRNA comprises one or more nick.

8. The compound according to any one of the preceding claims, wherein the compound is covalently attached to at least one conjugate moiety.

9. The compound according to any one of the preceding claims, wherein the smartRNA is capable of assembling with endogenous RNA binding protein (RNP) complex, thereby forming a smartRNP.

10. The compound according to any one of the preceding claims, wherein the binding of the first contiguous nucleotide sequence of the "D guide" region of complementarity and of the second contiguous nucleotide sequence of the "D' guide" region of complementarity to the first and second target nucleic acid sequence of the target mRNA, respectively, increases the expression of the protein encoded by the target mRNA.

11. A pharmaceutical composition comprising the smartRNA according to any one of the preceding claims.

12. A vector comprising a nucleotide sequence encoding the smartRNA according to any one of the preceding claims.

13. A host cell expressing the smartRNA according to any one of the preceding claims.

14. The compound according to any one of claim 1 to 9, the pharmaceutical composition according to claim 11, the vector according to claim 12, or the host cell according to claim 13, for use as a medicament.

15. The compound, pharmaceutical composition, vector, or host cell for use as a medicament according to claim 14, in preventing, treating and/or ameliorating a medical condition affecting a subject, wherein the medical condition is **characterized by** deficient protein expression of the protein encoded by the target mRNA.
